# EUROPEAN PATENT APPLICATION

(11) **EP 4 317 416 A1**
(43) Date of publication of application: **07.02.2024**
(21) Application number: 22780919.1
(22) Date of filing: 29.03.2022
(51) Int. Cl.: C12N 1/21, C12N 1/14, C12N 1/15, C12N 1/19, C12N 9/04, C12N 9/10, C12N 9/88, C12N 15/53, C12N 15/54, C12N 15/60, C12P 7/02, C12P 7/42, C12P 7/44, C12P 13/00

(54) **RECOMBINANT MICROORGANISM AND METHOD FOR PRODUCING C6 COMPOUND**

(30) Priority: 30.03.2021 JP 2021057325; 16.04.2021 JP 2021069632
(71) Applicant: Asahi Kasei Kabushiki Kaisha, Tokyo 1000006 (JP)
(72) Inventor: YAMADA Yutaro, Tokyo 100-0006 (JP); USHIKI Norisuke, Tokyo 100-0006 (JP); MIYATAKE Ryo, Tokyo 100-0006 (JP); ISAKA Koji, Tokyo 100-0006 (JP)
(74) Representative: D Young & Co LLP
(86) International application number: PCT/JP2022/015439
(87) International publication number: WO 2022/210708

(57) **Abstract**

Provided are a recombinant microorganism having a 2,3-dehydroadipyl-CoA reductase activity and a production method of a C6 compound, and are a recombinant microorganism capable of producing adipic acid or an adipic acid derivative, and a production method of adipic acid or an adipic acid derivative.

A recombinant microorganism contains at least one of an exogenous gene encoding a protein having an enzymatic activity to reduce 2,3-dehydroadipyl-CoA for conversion into adipyl-CoA and an exogenous gene encoding a 3-hydroxyadipyl-CoA dehydratase.

## Description

### Technical Field

The present invention relates to a recombinant microorganism and a method for producing a C6 compound. The present invention also relates to a recombinant microorganism and a method for producing adipic acid or a derivative thereof.

### Background Art

A C6 compound such as adipic acid, hexamethylenediamine, 1,6-hexanediol, 6-aminohexanoic acid, 6-amino-1-hexanol, or 6-hydroxyhexanoic acid is used as a raw material for a polyamide and a polyurethane, and is regarded as an important compound in the chemical industry. For example, adipic acid (CAS No. 124-04-9) is a monomer compound used as a polyamide raw material typified by nylon-66 or the like, urethane, and a plasticizer raw material. Common production methods of adipic acid, at present, are chemical synthesis methods, for example, a method for oxidizing cyclohexanol alone or a mixture (K/A oil) of cyclohexanol and cyclohexanone with nitric acid.

Recently, since fossil fuels are in danger of depletion and are considered to be a cause of global warming. Therefore, in a chemical production process, it is desired to shift from raw materials derived from fossil fuels to renewable raw materials, for example, biomass-derived raw materials, and a method of producing a compound by fermentative production of microorganisms whose metabolism is modified by gene recombination has been proposed.

For example, microorganisms for biosynthesis of adipic acid, 6-aminocaproic acid, or caprolactam have been reported (Patent Literature 1). As an example, there are disclosed methods for culturing microorganisms containing a "succinyl-CoA:acetyl-CoA acyltransferase" that converts succinyl-CoA and acetyl-CoA into 3-oxoadipyl-CoA; a "3-hydroxyacyl-CoA dehydrogenase" that converts 3-oxoadipyl-CoA into 3-hydroxyadipyl-CoA; a "3-hydroxyadipyl-CoA dehydratase" that converts 3-hydroxyadipyl-CoA into 5-carboxy-2-pentenoyl-CoA; a "5-carboxy-2-pentenoyl-CoA reductase" that converts 5-carboxy-2-pentenoyl-CoA into adipyl-CoA; and a "phosphotransadipylase" and an "adipate kinase" that convert adipyl-CoA into adipate, but only genes that may promote a reaction are shown as examples of the respective enzymes, and it has not been demonstrated that the genes of these enzymes can efficiently produce a C6 compound from biomass-derived carbon sources.

In addition, although a method for preparing an adipate thioester including a step of enzymatically converting a 2,3-dehydroadipate thioester into an adipate thioester has also been proposed (Patent Literature 2), only some enoyl-CoA reductases derived from biological species such as Candida tropicalis, Euglena gracilis, Clostridium beijerinckii, and Yarrowia lipolytica exhibit an enzymatic activity, and no examples demonstrating fermentative production of a C6 compound have been shown.

Furthermore, it has been reported that 36 mg/L of adipic acid can be produced using glucose as a carbon source utilizing a culture of a genetically modified Escherichia coli in which enzymes PaaJ, PaaH, and PaaF derived from Escherichia coli and enzymes DcaA and TesB derived from Acinetobacter baylyi are co-expressed (Non Patent Literature 1).

An example of producing adipic acid and 6-aminocaproic acid by culturing Escherichia coli exhibiting an enzymatic activity of trans-enoyl-CoA reductases derived from a plurality of species and further expressing a trans-enoyl-CoA reductase derived from Candida tropicalis or Drosophila melanogaster or a variant of these enzymes has also been proposed (Patent Literature 3).

However, the examples of fermentative production according to the above prior arts do not necessarily satisfy sufficient productivity for industrial use, and there is a need to improve productivity of microorganisms.

In addition, regarding adipic acid, as a production method using a biomass-derived raw material, a method in which a biomass-derived carbon source is converted into cis,cis-muconic acid using a genetically modified microorganism, and then the resulting cis,cis-muconic acid is hydrogenated to produce adipic acid has also been proposed (Patent Literature 4). In addition, a method for producing adipic acid from α-ketoglutaric acid using a genetic recombinant has also been proposed (Patent Literature 5).

Furthermore, a method for fermentatively producing adipic acid directly from a biomass-derived raw material has also been proposed (Patent Literature 6). In this method, examples of non-naturally occurring microorganisms capable of producing adipic acid include non-naturally occurring microorganisms containing a "succinyl-CoA:acetyl-CoA acyltransferase" that converts succinyl-CoA and acetyl-CoA into 3-oxoadipyl-CoA; a "3-hydroxyacyl-CoA dehydrogenase" that converts 3-oxoadipyl-CoA into 3-hydroxyadipyl-CoA; a "3-hydroxyadipyl-CoA dehydratase" that converts 3-hydroxyadipyl-CoA into 5-carboxy-2-pentenoyl-CoA; a "5-carboxy-2-pentenoyl-CoA reductase" that converts 5-carboxy-2-pentenoyl-CoA into adipyl-CoA; and a "phosphotransadipylase"/"adipate kinase" that convert adipyl-CoA into adipate, but for each enzyme, only genes encoding an enzyme that may promote a reaction are shown, and it has not been demonstrated that adipic acid can be produced from a biomass-derived carbon material with an excellent yield by using these genes.

### Citation List

### Patent Literature

Patent Literature 1: Japanese Patent No. 5951990
Patent Literature 2: JP 2011-512868 A
Patent Literature 3: WO 2020/219859 A
Patent Literature 4: WO 1995/007996 A
Patent Literature 5: WO 2010/104391 A
Patent Literature 6: JP 2011-515111 A

### Non Patent Literature

Non Patent Literature 1: Kallscheuer, Nicolai, et al. "Improved production of adipate with Escherichia coli by reversal of β-oxidation." Applied microbiology and biotechnology 101.6 (2017): 2371-2382.

### Summary of Invention

### Technical Problem

An object of the present invention is to provide a recombinant microorganism having a 2,3-dehydroadipyl-CoA reductase activity, and an efficient production method of a C6 compound.

Another object of the present invention is to provide a recombinant microorganism capable of producing adipic acid or an adipic acid derivative, and a production method of adipic acid or an adipic acid derivative. Solution to Problem

As a result of intensive studies to solve the problems described above of the prior arts, the present inventors have found that a recombinant microorganism having an excellent 2,3-dehydroadipyl-CoA reductase activity can be obtained by expressing an exogenous specific enzyme in a host microorganism, and a C6 compound can be further efficiently produced by culturing the recombinant microorganism, thereby arriving at the present invention.

That is, the present invention provides the followings:
[1] a recombinant microorganism containing an exogenous gene encoding a protein having an enzymatic activity to reduce 2,3-dehydroadipyl-CoA for conversion into adipyl-CoA,
   in which the exogenous gene is
   (i) a DNA encoding a protein consisting of an amino acid sequence having a sequence identity of 80% or higher with an amino acid sequence set forth in SEQ ID NO: 8, 9, 10, 11, 12, 13, 14, 15, or 16,
   (ii) a DNA encoding a protein consisting of an amino acid sequence in which 1 to 10 amino acids are deleted, substituted, inserted, and/or added in an amino acid sequence set forth in SEQ ID NO: 8, 9, 10, 11, 12, 13, 14, 15, or 16,
   (iii) a DNA consisting of a polynucleotide sequence having a sequence identity of 80% or higher with a polynucleotide sequence set forth in SEQ ID NO: 57, 58, 59, 60, 61, 62, 63, 64, or 65,
   (iv) a DNA encoding a protein consisting of an amino acid sequence in which 1 to 10 amino acids are deleted, substituted, inserted, and/or added in an amino acid sequence of a protein encoded by a polynucleotide sequence set forth in SEQ ID NO: 57, 58, 59, 60, 61, 62, 63, 64, or 65,
   (v) a DNA hybridizing with a DNA consisting of a polynucleotide sequence complementary to a polynucleotide sequence set forth in SEQ ID NO: 57, 58, 59, 60, 61, 62, 63, 64, or 65 under a stringent condition, or
   (vi) a DNA consisting of a degenerate isomer of a polynucleotide sequence set forth in SEQ ID NO: 57, 58, 59, 60, 61, 62, 63, 64, or 65;
[2] the recombinant microorganism according to [1], in which the exogenous gene is:
   (xi) a DNA encoding a protein consisting of an amino acid sequence set forth in SEQ ID NO: 8, 9, 10, 11, 12, 13, 14, 15, or 16, or
   (xiii) a DNA consisting of a polynucleotide sequence set forth in SEQ ID NO: 57, 58, 59, 60, 61, 62, 63, 64, or 65;
[3] the recombinant microorganism according to [1] or [2], in which the exogenous gene is derived from at least one selected from Candida auris, Kluyveromyces marxianus, Pichia kudriavzevii, Thermothelomyces thermophilus, Thermothielavioides terrestris, Chaetomium thermophilum, Podospora anserina, Purpureocillium lilacinum, and Pyrenophora teres;
[4] the recombinant microorganism according to any one of [1] to [3], in which the recombinant microorganism has a production pathway of a C6 compound, and
   in which the C6 compound is at least one selected from the group consisting of adipic acid, hexamethylenediamine, 1,6-hexanediol, 6-aminohexanoic acid, 6-amino-1-hexanol, and 6-hydroxyhexanoic acid;
[5] the recombinant microorganism according to any one of [1] to [4], in which the recombinant microorganism belongs to the genus Escherichia, the genus Bacillus, the genus Corynebacterium, the genus Arthrobacter, the genus Brevibacterium, the genus Clostridium, the genus Zymomonas, the genus Pseudomonas, the genus Burkholderia, the genus Streptomyces, the genus Rhodococcus, the genus Synechocystis, the genus Alkalihalobacillus, the genus Saccharomyces, the genus Schizosaccharomyces, the genus Yarrowia, the genus Candida, the genus Pichia, or the genus Aspergillus;
[6] the recombinant microorganism according to any one of [1] to [5], in which the recombinant microorganism is Escherichia coli;
[7] the recombinant microorganism according to any one of [1] to [6], further containing at least one selected from the group consisting of:
   • a gene encoding a 3-oxoadipyl-CoA thiolase;
   • a gene encoding a 3-hydroxyadipyl-CoA dehydrogenase;
   • a gene encoding a 3-hydroxyadipyl-CoA dehydratase;
   • a gene encoding a carboxylic acid reductase;
   • a gene encoding an alcohol dehydrogenase; and
   • a gene encoding a transaminase (aminotransferase);
[8] a method for producing a C6 compound, the production method including a culture step of culturing the recombinant microorganism according to any one of [1] to [7],
   in which the C6 compound is at least one selected from the group consisting of adipic acid, hexamethylenediamine, 1,6-hexanediol, 6-aminohexanoic acid, 6-amino-1-hexanol, and 6-hydroxyhexanoic acid;
[9] a recombinant protein (a) having an enzymatic activity to reduce 2,3-dehydroadipyl-CoA for conversion into adipyl-CoA, and
   (b) satisfying any one of the followings:
   (i) consisting of an amino acid sequence having a sequence identity of 80% or higher with an amino acid sequence set forth in SEQ ID NO: 8, 9, 10, 11, 12, 13, 14, 15, or 16;
   (ii) consisting of an amino acid sequence in which 1 to 10 amino acids are deleted, substituted, inserted, and/or added in an amino acid sequence set forth in SEQ ID NO: 8, 9, 10, 11, 12, 13, 14, 15, or 16;
   (iii) encoded by a DNA consisting of a polynucleotide sequence having a sequence identity of 80% or higher with a polynucleotide sequence set forth in SEQ ID NO: 57, 58, 59, 60, 61, 62, 63, 64, or 65;
   (iv) consisting of an amino acid sequence in which 1 to 10 amino acids are deleted, substituted, inserted, and/or added in an amino acid sequence of a protein encoded by a polynucleotide sequence set forth in SEQ ID NO: 57, 58, 59, 60, 61, 62, 63, 64, or 65;
   (v) encoded by a DNA hybridizing with a DNA consisting of a polynucleotide sequence complementary to a polynucleotide sequence set forth in SEQ ID NO: 57, 58, 59, 60, 61, 62, 63, 64, or 65 under a stringent condition; and
   (vi) encoded by a DNA consisting of a degenerate isomer of a polynucleotide sequence set forth in SEQ ID NO: 57, 58, 59, 60, 61, 62, 63, 64, or 65;
[10] a recombinant protein:
   (xi) consisting of an amino acid sequence set forth in SEQ ID NO: 8, 9, 10, 11, 12, 13, 14, 15, or 16; or
   (xiii) encoded by a DNA consisting of a polynucleotide sequence set forth in SEQ ID NO: 57, 58, 59, 60, 61, 62, 63, 64, or 65; and
[11] a method for producing a C6 compound, the method including using the recombinant protein according to [9] or [10].

Furthermore, as a result of intensive studies to solve the above problems, the present inventors have found that adipic acids can be efficiently produced by culturing a genetically modified microorganism expressing a predetermined enzyme of the genus Burkholderia (Burkholderia sp.) LEBP-3 strain (Accession Number: NITE BP-03334), which is a microorganism newly isolated from nature, thereby arriving at the present invention.

That is, the present invention further provides the followings:
[12] a recombinant microorganism containing at least one of an exogenous gene encoding a 3-hydroxyadipyl-CoA dehydratase and an exogenous gene encoding a 2,3-dehydroadipyl-CoA reductase;
[13] the recombinant microorganism according to [12], in which at least one of the 3-hydroxyadipyl-CoA dehydratase and the 2,3-dehydroadipyl-CoA reductase is derived from the genus Burkholderia (Burkholderia sp.) LEBP-3 strain (Accession Number: NITE BP-03334);
[14] the recombinant microorganism according to [12] or [13], in which the 3-hydroxyadipyl-CoA dehydratase is encoded by a DNA having a sequence identity of 80% or higher, 85% or higher, 88% or higher, 90% or higher, 93% or higher, 95% or higher, 97% or higher, 98% or higher, or 99% or higher with a PCR amplification product obtained using a chromosomal DNA of the genus Burkholderia (Burkholderia sp.) LEBP-3 strain as a template and using, as a primer,
   (1a) a nucleotide consisting of a base sequence set forth in SEQ ID NO: 168 and a nucleotide consisting of a base sequence set forth in SEQ ID NO: 169, or
   (1b) a nucleotide having a base sequence set forth in SEQ ID NO: 168 and a nucleotide having a base sequence set forth in SEQ ID NO: 169, and
   the DNA has 626 to 940 bp;
[15] the recombinant microorganism according to any one of [12] to [14], in which the 2,3-dehydroadipyl-CoA reductase is encoded by a DNA having a sequence identity of 80% or higher, 85% or higher, 88% or higher, 90% or higher, 93% or higher, 95% or higher, 97% or higher, 98% or higher, or 99% or higher with a PCR amplification product obtained using a chromosomal DNA of the genus Burkholderia (Burkholderia sp.) LEBP-3 strain as a template and using, as a primer,
   (2a) a nucleotide consisting of a base sequence set forth in SEQ ID NO: 170 and a nucleotide consisting of a base sequence set forth in SEQ ID NO: 171, or
   (2b) a nucleotide having a base sequence set forth in SEQ ID NO: 170 and a nucleotide having a base sequence set forth in SEQ ID NO: 171, and
   the DNA has 924 to 1,386 bp;
[16] the recombinant microorganism according to [12] or [13], in which the 3-hydroxyadipyl-CoA dehydratase is encoded by a DNA that is a PCR amplification product obtained using a chromosomal DNA of the genus Burkholderia (Burkholderia sp.) LEBP-3 strain as a template and using, as a primer,
   (1a) a nucleotide consisting of a base sequence set forth in SEQ ID NO: 168 and a nucleotide consisting of a base sequence set forth in SEQ ID NO: 169, or
   (1b) a nucleotide having a base sequence set forth in SEQ ID NO: 168 and a nucleotide having a base sequence set forth in SEQ ID NO: 169, and
   the DNA has 783 or 784 bp;
[17] the recombinant microorganism according to any one of [12] to [14], in which the 2,3-dehydroadipyl-CoA reductase is encoded by a DNA that is a PCR amplification product obtained using a chromosomal DNA of the genus Burkholderia (Burkholderia sp.) LEBP-3 strain as a template and using, as a primer,
   (2a) a nucleotide consisting of a base sequence set forth in SEQ ID NO: 170 and a nucleotide consisting of a base sequence set forth in SEQ ID NO: 171, or
   (2b) a nucleotide having a base sequence set forth in SEQ ID NO: 170 and a nucleotide having a base sequence set forth in SEQ ID NO: 171, and
   the DNA has 1,155 or 1,156 bp;
[18] the recombinant microorganism according to any one of [12] to [17], in which the recombinant microorganism belongs to a genus selected from the group consisting of the genus Escherichia, the genus Corynebacterium, the genus Bacillus, the genus Acinetobacter, the genus Burkholderia, the genus Pseudomonas, the genus Clostridium, the genus Saccharomyces, the genus Schizosaccharomyces, the genus Yarrowia, the genus Candida, the genus Pichia, and the genus Aspergillus;
[19] the recombinant microorganism according to any one of [12] to [18], in which the recombinant microorganism has an adipic acid production pathway;
[20] the recombinant microorganism according to any one of [12] to [19], in which the recombinant microorganism has a hexamethylenediamine production pathway;
[21] the recombinant microorganism according to any one of [12] to [19], in which the recombinant microorganism has a 1,6-hexanediol production pathway;
[22] the recombinant microorganism according to any one of [12] to [19], in which the recombinant microorganism has a 6-amino-1-hexanol production pathway;
[23] a method for producing a target compound, the method including a culture step of culturing the recombinant microorganism according to any one of [12] to [18],
   in which the target compound is selected from the group consisting of adipic acid, an adipic acid derivative, hexamethylenediamine, 1,6-hexanediol, and 6-amino-1-hexanol, and
   the adipic acid derivative is selected from the group consisting of oxoadipic acid, levulinic acid, 3-hydroxyadipic acid, and 2,3-dehydroadipic acid;
[24] a method for producing adipic acid, the method including a culture step of culturing the recombinant microorganism according to [19];
[25] a method for producing hexamethylenediamine, the method including a culture step of culturing the recombinant microorganism according to [20];
[26] a method for producing 1,6-hexanediol, the method including a culture step of culturing the recombinant microorganism according to [21];
[27] a method for producing 6-amino-1-hexanol, the method including a culture step of culturing the recombinant microorganism according to [22];
[28] (A-1) a recombinant polypeptide encoded by a DNA having a sequence identity of 80% or higher, 85% or higher, 88% or higher, 90% or higher, 93% or higher, 95% or higher, 97% or higher, 98% or higher, or 99% or higher with a PCR amplification product obtained using a chromosomal DNA of the genus Burkholderia (Burkholderia sp.) LEBP-3 strain as a template, and using, as a primer,
   (a1) a nucleotide consisting of a base sequence set forth in SEQ ID NO: 168 and a nucleotide consisting of a base sequence set forth in SEQ ID NO: 169, or
   (a2) a nucleotide having a base sequence set forth in SEQ ID NO: 168 and a nucleotide having a base sequence set forth in SEQ ID NO: 169,
   in which the recombinant polypeptide has a 3-hydroxyadipyl-CoA dehydratase activity, and
   the DNA has 626 to 940 bp;
   (A-2) a recombinant polypeptide consisting of an amino acid sequence in which 1 to 10 amino acids are deleted, substituted, inserted, and/or added in an amino acid sequence of a polypeptide encoded by a DNA that is a PCR amplification product obtained using a chromosomal DNA of the genus Burkholderia (Burkholderia sp.) LEBP-3 strain as a template, and using, as a primer,
   (a1) a nucleotide consisting of a base sequence set forth in SEQ ID NO: 168 and a nucleotide consisting of a base sequence set forth in SEQ ID NO: 169, or
   (a2) a nucleotide having a base sequence set forth in SEQ ID NO: 168 and a nucleotide having a base sequence set forth in SEQ ID NO: 169,
   in which the recombinant polypeptide has a 3-hydroxyadipyl-CoA dehydratase activity, and
   the DNA has 626 to 940 bp;
      or
   (A-3) a recombinant polypeptide encoded by a DNA consisting of a degenerate isomer of a PCR amplification product obtained using a chromosomal DNA of the genus Burkholderia (Burkholderia sp.) LEBP-3 strain as a template, and using, as a primer,
   (a1) a nucleotide consisting of a base sequence set forth in SEQ ID NO: 168 and a nucleotide consisting of a base sequence set forth in SEQ ID NO: 169, or
   (a2) a nucleotide having a base sequence set forth in SEQ ID NO: 168 and a nucleotide having a base sequence set forth in SEQ ID NO: 169,
   in which the recombinant polypeptide has a 3-hydroxyadipyl-CoA dehydratase activity, and
   the DNA has 626 to 940 bp; and
[29] (B-1) a recombinant polypeptide encoded by a DNA having a sequence identity of 80% or higher, 85% or higher, 88% or higher, 90% or higher, 93% or higher, 95% or higher, 97% or higher, 98% or higher, or 99% or higher with a PCR amplification product obtained using a chromosomal DNA of the genus Burkholderia (Burkholderia sp.) LEBP-3 strain as a template, and using, as a primer,
   (b1) a nucleotide consisting of a base sequence set forth in SEQ ID NO: 170 and a nucleotide consisting of a base sequence set forth in SEQ ID NO: 171, or
   (b2) a nucleotide having a base sequence set forth in SEQ ID NO: 170 and a nucleotide having a base sequence set forth in SEQ ID NO: 171, and
   in which the recombinant polypeptide has a 2,3-dehydroadipyl-CoA reductase activity, and
   the DNA has 924 to 1,386 bp;
   (B-2) a recombinant polypeptide consisting of an amino acid sequence in which 1 to 10 amino acids are deleted, substituted, inserted, and/or added in an amino acid sequence of a polypeptide encoded by a DNA that is a PCR amplification product obtained using a chromosomal DNA of the genus Burkholderia (Burkholderia sp.) LEBP-3 strain as a template, and using, as a primer,
   (b1) a nucleotide consisting of a base sequence set forth in SEQ ID NO: 170 and a nucleotide consisting of a base sequence set forth in SEQ ID NO: 171, or
   (b2) a nucleotide having a base sequence set forth in SEQ ID NO: 170 and a nucleotide having a base sequence set forth in SEQ ID NO: 171, and
   in which the recombinant polypeptide has a 2,3-dehydroadipyl-CoA reductase activity, and
   the DNA has 924 to 1,386 bp;
      or
   (B-3) a recombinant polypeptide encoded by a DNA consisting of a degenerate isomer of a PCR amplification product obtained using a chromosomal DNA of the genus Burkholderia (Burkholderia sp.) LEBP-3 strain as a template, and using, as a primer,
   (b1) a nucleotide consisting of a base sequence set forth in SEQ ID NO: 170 and a nucleotide consisting of a base sequence set forth in SEQ ID NO: 171, or
   (b2) a nucleotide having a base sequence set forth in SEQ ID NO: 170 and a nucleotide having a base sequence set forth in SEQ ID NO: 171, and
   in which the recombinant polypeptide has a 2,3-dehydroadipyl-CoA reductase activity, and
   the DNA has 924 to 1,386 bp.

### Advantageous Effects of Invention

According to the present invention, there are provided a recombinant microorganism having a 2,3-dehydroadipyl-CoA reductase activity, and a production method of a C6 compound.

In addition, according to the present invention, there are provided a recombinant microorganism capable of producing adipic acid or an adipic acid derivative, and a production method of adipic acid or an adipic acid derivative.

### Brief Description of Drawings

Fig. 1 is a diagram illustrating an example of a biosynthetic pathway from acetyl-CoA and succinyl-CoA to a C6 compound.
Fig. 2 is a diagram illustrating an amino acid sequence of each enzyme and a base sequence of a primer.
Fig. 3 is a diagram illustrating an amino acid sequence of each enzyme.
Fig. 4 is a diagram illustrating an amino acid sequence of each enzyme.
Fig. 5 is a diagram illustrating an amino acid sequence of each enzyme.
Fig. 6 is a diagram illustrating a base sequence encoding each enzyme and a base sequence of a primer.
Fig. 7 is a diagram illustrating a base sequence encoding each enzyme and a base sequence of a primer.
Fig. 8 is a diagram illustrating a base sequence encoding each enzyme and a base sequence of a primer.
Fig. 9 is a diagram illustrating a base sequence encoding each enzyme.
Fig. 10 is a diagram illustrating a base sequence encoding each enzyme.
Fig. 11 is a diagram illustrating a base sequence of each primer.
Fig. 12 is a diagram illustrating a base sequence encoding each enzyme and a base sequence of each primer.
Fig. 13 is a diagram illustrating a base sequence of each primer.
Fig. 14 is a diagram illustrating a base sequence encoding each enzyme and a base sequence of each primer.
Fig. 15 is a diagram illustrating an amino acid sequence of each enzyme, a base sequence encoding each enzyme, and a base sequence of each primer.
Fig. 16 illustrates an example of adipic acid, adipic acid derivative, hexamethylenediamine, 1,6-hexanediol, and 6-amino-1-hexanol production pathways of a recombinant microorganism according to the present invention.
Fig. 17 illustrates genes to be amplified in PCR and primers used.
Fig. 18 illustrates an amino acid sequence of each enzyme.
Fig. 19A illustrates a base sequence encoding each enzyme.
Fig. 19B illustrates a base sequence encoding each enzyme.
Fig. 20A illustrates genes to be amplified in PCR and primers used.
Fig. 20B illustrates genes to be amplified in PCR and primers used.
Fig. 21 illustrates an amino acid sequence of an enzyme.
Fig. 22A illustrates an amino acid sequence of an enzyme.
Fig. 22B illustrates an amino acid sequence of an enzyme.
Fig. 22C illustrates an amino acid sequence of an enzyme.
Fig. 22D illustrates an amino acid sequence of an enzyme.
Fig. 22E illustrates an amino acid sequence of an enzyme.
Fig. 23A illustrates an amino acid sequence of an enzyme.
Fig. 23B illustrates an amino acid sequence of an enzyme.
Fig. 23C illustrates an amino acid sequence of an enzyme.
Fig. 23D illustrates an amino acid sequence of an enzyme.
Fig. 24A illustrates an amino acid sequence of an enzyme.
Fig. 24B illustrates an amino acid sequence of an enzyme.
Fig. 24C illustrates an amino acid sequence of an enzyme.
Fig. 24D illustrates an amino acid sequence of an enzyme.
Fig. 24E illustrates an amino acid sequence of an enzyme.
Fig. 25 illustrates an amino acid sequence of an enzyme.

### Description of Embodiments

Hereinafter, embodiments of the present invention will be described in detail. Note that the present invention is not limited to the following embodiments, and various modifications can be made within the scope of the gist of the present invention. In addition, unless otherwise specified, genetic manipulations such as acquisition of DNA and preparation of a vector and transformation described in the present specification can be performed by the methods described in known documents such as Molecular Cloning 4th Edition (Cold Spring Harbor Laboratory Press, 2012), Current Protocols in Molecular Biology (Greene Publishing Associates and Wiley-Interscience), and genetic engineering experimental note (Takaaki Tamura, YODOSHA CO., LTD.). In the present specification, unless otherwise specified, nucleotide sequences are described from the 5' direction to the 3' direction. In the present specification, the terms "polypeptide" and "protein" are used interchangeably.

In the present specification, a range of numerical values indicated by "to" represents a range including numerical values described before and after "to" as the minimum value and the maximum value, respectively. In the range of the numerical values described in stages in the present specification, an upper limit value or a lower limit value of a range of numerical values of a certain stage can be arbitrarily combined with an upper limit value or a lower limit value of a range of numerical values of another stage.

In the present specification, the term "endogenous" or "endogenous property" is used to mean that the host microorganism that is not modified by gene recombination referred to includes a host microorganism, regardless of whether the gene referred to or the protein encoded by the same (typically, an enzyme) is functionally expressed to the extent that a predominant biochemical reaction can proceed in the host cell.

In the present specification, the term "exogenous" or "exogenous property" is used to mean that a gene or a nucleic acid sequence is introduced into a host based on the present invention in a case where a pre-genetically modified host microorganism does not have a gene to be introduced by the present invention, in a case where an enzyme is not substantially expressed by the gene, or in a case where an amino acid sequence of the enzyme is encoded by a gene different from the gene but does not express an endogenous enzyme activity corresponding to after the genetic modification.

### <1> Invention A: Recombinant Microorganism and Production Method of C6 Compound

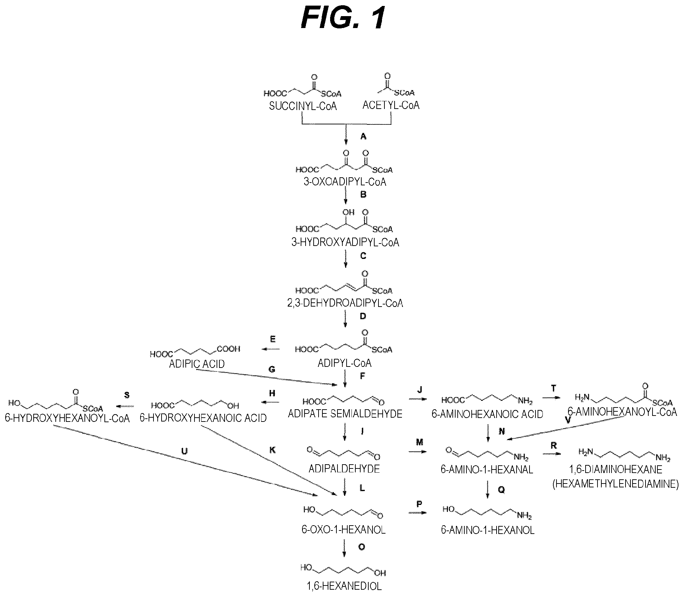

A recombinant microorganism according to the present invention contains an exogenous gene encoding a protein having an enzymatic activity to reduce 2,3-dehydroadipyl-CoA for conversion into adipyl-CoA. The reaction of reducing 2,3-dehydroadipyl-CoA for conversion into adipyl-CoA is illustrated in Step D in Fig. 1. The "protein having an enzymatic activity to reduce 2,3-dehydroadipyl-CoA for conversion into adipyl-CoA" is also referred to as "2,3-dehydroadipyl-CoA reductase". In addition, the enzymatic activity to reduce 2,3-dehydroadipyl-CoA for conversion into adipyl-CoA is also referred to as "2,3-dehydroadipyl-CoA reductase activity" or "2,3-dehydroadipyl-CoA reductase enzymatic activity", and these terms are used interchangeably.

The exogenous gene contained in the recombinant microorganism according to the present invention is selected from the followings:
(i) a DNA encoding a protein consisting of an amino acid sequence having a sequence identity of 80% or higher, 85% or higher, 88% or higher, 90% or higher, 93% or higher, 95% or higher, 97% or higher, 98% or higher, or 99% or higher with an amino acid sequence set forth in SEQ ID NO: 8, 9, 10, 11, 12, 13, 14, 15, or 16;
(ii) a DNA encoding a protein consisting of an amino acid sequence in which 1 to 10, 1 to 7, 1 to 5, or 1 to 3 amino acids are deleted, substituted, inserted, and/or added in an amino acid sequence set forth in SEQ ID NO: 8, 9, 10, 11, 12, 13, 14, 15, or 16;
(iii) a DNA consisting of a polynucleotide sequence having a sequence identity of 80% or higher, 85% or higher, 88% or higher, 90% or higher, 93% or higher, 95% or higher, 97% or higher, 98% or higher, or 99% or higher with a polynucleotide sequence set forth in SEQ ID NO: 57, 58, 59, 60, 61, 62, 63, 64, or 65;
(iv) a DNA encoding a protein consisting of an amino acid sequence in which 1 to 10, 1 to 7, 1 to 5, or 1 to 3 amino acids are deleted, substituted, inserted, and/or added in an amino acid sequence of a protein encoded by a polynucleotide sequence set forth in SEQ ID NO: 57, 58, 59, 60, 61, 62, 63, 64, or 65;
(v) a DNA hybridizing with a DNA consisting of a polynucleotide sequence complementary to a polynucleotide sequence set forth in SEQ ID NO: 57, 58, 59, 60, 61, 62, 63, 64, or 65 under a stringent condition; and
(vi) a DNA consisting of a degenerate isomer of a polynucleotide sequence set forth in SEQ ID NO: 57, 58, 59, 60, 61, 62, 63, 64, or 65.

The exogenous gene contained in the recombinant microorganism according to the present invention is preferably selected from the followings:
(i) a DNA encoding a protein consisting of an amino acid sequence having a sequence identity of 80% or higher with an amino acid sequence set forth in SEQ ID NO: 8, 9, 10, 11, 12, 13, 14, 15, or 16;
(ii) a DNA encoding a protein consisting of an amino acid sequence in which 1 to 10 amino acids are deleted, substituted, inserted, and/or added in an amino acid sequence set forth in SEQ ID NO: 8, 9, 10, 11, 12, 13, 14, 15, or 16;
(iii) a DNA consisting of a polynucleotide sequence having a sequence identity of 80% or higher with a polynucleotide sequence set forth in SEQ ID NO: 57, 58, 59, 60, 61, 62, 63, 64, or 65;
(iv) a DNA encoding a protein consisting of an amino acid sequence in which 1 to 10 amino acids are deleted, substituted, inserted, and/or added in an amino acid sequence of a protein encoded by a polynucleotide sequence set forth in SEQ ID NO: 57, 58, 59, 60, 61, 62, 63, 64, or 65;
(v) a DNA hybridizing with a DNA consisting of a polynucleotide sequence complementary to a polynucleotide sequence set forth in SEQ ID NO: 57, 58, 59, 60, 61, 62, 63, 64, or 65 under a stringent condition; and
(vi) a DNA consisting of a degenerate isomer of a polynucleotide sequence set forth in SEQ ID NO: 57, 58, 59, 60, 61, 62, 63, 64, or 65.

The exogenous gene contained in the recombinant microorganism according to the present invention is more preferably:
(xi) a DNA encoding a protein consisting of an amino acid sequence set forth in SEQ ID NO: 8, 9, 10, 11, 12, 13, 14, 15, or 16, or
(xiii) a DNA consisting of a polynucleotide sequence set forth in SEQ ID NO: 57, 58, 59, 60, 61, 62, 63, 64, or 65.

The exogenous gene is derived from, for example, at least one selected from Candida auris, Kluyveromyces marxianus, Pichia kudriavzevii, Thermothelomyces thermophilus, Thermothielavioides terrestris, Chaetomium thermophilum, Podospora anserina, Purpureocillium lilacinum, and Pyrenophora teres.

By containing the exogenous gene, the recombinant microorganism has an excellent 2,3-dehydroadipyl-CoA reductase activity. A target compound can be produced by using a recombinant microorganism having such an enzymatic activity. Examples of the target compound include a C6 compound.

In the present specification, the "C6 compound" includes:
· adipic acid (CAS No.124-04-9),
· hexamethylenediamine (CAS No.124-09-4),
· 1,6-hexanediol (CAS No.629-11-8),
· 6-aminohexanoic acid (CAS No.60-32-2),
· 6-amino-1-hexanol (CAS No.4048-33-3), and
· 6-hydroxyhexanoic acid (CAS No.1191-25-9), and
the "C6 compound" refers to one or a plurality of compounds selected from the group consisting of these compounds.

It is understood by those skilled in the art that the "C6 compound" in the present specification can take a neutral or ionized form including any salt form and this form is pH-dependent.

As described above, the microorganism of the present invention is a genetically modified microorganism into which an exogenous enzyme gene is introduced. The "genetically modified microorganism" is simply referred to as "recombinant microorganism".

In relation to the present invention, a host microorganism into which a target exogenous enzyme gene is introduced is not particularly limited, and may be either a prokaryote or a eukaryote. Any one of a microorganism isolated and preserved in advance, a microorganism newly isolated from nature, and a microorganism subjected to a genetic modification can be arbitrarily selected. The host microorganism belongs to, for example, the genus Escherichia, the genus Bacillus, the genus Corynebacterium, the genus Arthrobacter, the genus Brevibacterium, the genus Clostridium, the genus Zymomonas, the genus Pseudomonas, the genus Burkholderia, the genus Streptomyces, the genus Rhodococcus, the genus Synechocystis, the genus Alkalihalobacillus, the genus Saccharomyces, the genus Schizosaccharomyces, the genus Yarrowia, the genus Candida, the genus Pichia, or the genus Aspergillus. The host microorganism preferably belongs to the genus Escherichia, and more preferably belongs to Escherichia coli.

Therefore, the recombinant microorganism according to the present invention in which an exogenous gene is introduced into the host microorganism belongs to, for example, the genus Escherichia, the genus Bacillus, the genus Corynebacterium, the genus Arthrobacter, the genus Brevibacterium, the genus Clostridium, the genus Zymomonas, the genus Pseudomonas, the genus Burkholderia, the genus Streptomyces, the genus Rhodococcus, the genus Synechocystis, the genus Alkalihalobacillus, the genus Saccharomyces, the genus Schizosaccharomyces, the genus Yarrowia, the genus Candida, the genus Pichia, or the genus Aspergillus. The recombinant microorganism according to the present invention preferably belongs to the genus Escherichia, and more preferably belongs to Escherichia coli.

In the present specification, as for a certain compound, the phrase "having a production pathway" means that the genetically modified microorganism according to the present invention can express a sufficient amount of enzyme to allow each reaction stage of the production pathway of the compound to proceed, and can biosynthesize the compound. The recombinant microorganism of the present invention may use a host microorganism intrinsically having an ability to produce the compound, or may be a microorganism obtained by being modified so that a host microorganism intrinsically not having an ability to produce the compound has an ability to produce the compound.

In the present specification, the phrase "derived from" means that a gene or a protein encoded by the gene (typically an enzyme) is endogenously carried by a specific organism species referred to.

The genetically modified microorganism of the present invention contains an exogenous gene encoding a protein having an enzymatic activity (that is, a 2,3-dehydroadipyl-CoA reductase activity) that reduces 2,3-dehydroadipyl-CoA for conversion into adipyl-CoA, such that genetically modified microorganism expresses an enzyme (that is, a 2,3-dehydroadipyl-CoA reductase) in an amount sufficient for the reaction to proceed. The present enzyme is involved in the reaction of Step D in Fig. 1.

In addition, an enzyme that catalyzes the reaction stage of the C6 compound production pathway is further expressed, such that a genetically modified microorganism having a C6 compound production pathway can be obtained.

In one embodiment, the recombinant microorganism according to the present invention contains at least one selected from the group consisting of:
· a gene encoding a 3-oxoadipyl-CoA thiolase;
· a gene encoding a 3-hydroxyadipyl-CoA dehydrogenase;
· a gene encoding a 3-hydroxyadipyl-CoA dehydratase;
· a combination of a gene encoding a phosphate adipyltransferase and a gene encoding an adipate kinase;
· a gene encoding an adipyl-CoA hydrolase;
· a gene encoding an adipate-CoA transferase;
· a gene encoding a dehydrogenase;
· a gene encoding a carboxylic acid reductase;
· a gene encoding a transaminase (aminotransferase);
· a gene encoding an alcohol dehydrogenase;
· a gene encoding a CoA transferase; and
· a gene encoding an acid-thiol ligase.

· The reaction of Step A in Fig. 1 is catalyzed by a 3-oxoadipyl-CoA thiolase,
· the reaction of Step B in Fig. 1 is catalyzed by a 3-hydroxyadipyl-CoA dehydrogenase,
· the reaction of Step C in Fig. 1 is catalyzed by a 3-hydroxyadipyl-CoA dehydratase,
· the reaction of Step E in Fig. 1 is catalyzed by a combination of a gene encoding a phosphate adipyltransferase and a gene encoding an adipate kinase; an adipyl-CoA hydrolase; or an adipate-CoA transferase,
· at least one reaction of Steps F, U, and V in Fig. 1 is catalyzed by a dehydrogenase,
· at least one reaction of Steps G, I, K, and N in Fig. 1 is catalyzed by a carboxylic acid reductase,
· at least one reaction of Steps J, M, P, and R in Fig. 1 is catalyzed by a transaminase (aminotransferase),
· at least one reaction of Steps H, L, O, and Q in Fig. 1 is catalyzed by an alcohol dehydrogenase, and
· at least one reaction of Steps S and T is catalyzed by a CoA transferase or an acid-thiol ligase.

In a preferred embodiment, the recombinant microorganism according to the present invention further contains at least one selected from the group consisting of:
· a gene encoding a 3-oxoadipyl-CoA thiolase;
· a gene encoding a 3-hydroxyadipyl-CoA dehydrogenase;
· a gene encoding a 3-hydroxyadipyl-CoA dehydratase;
· a gene encoding a carboxylic acid reductase;
· a gene encoding an alcohol dehydrogenase; and
· a gene encoding a transaminase (aminotransferase).

It should be noted that in a case where the genetically modified microorganism of the present invention expresses a sufficient amount of enzyme that catalyzes a reaction stage of a C6 compound production pathway without introduction of an exogenous gene, the reaction may proceed by the enzyme encoded by the endogenous gene.

An example of the C6 compound production pathway that can be obtained by the genetically modified microorganism of the present invention is illustrated in Fig. 1. Hereinafter, the enzyme that catalyzes each reaction stage will be described with reference to Fig. 1.

In Step A in Fig. 1, succinyl-CoA and acetyl-CoA are condensed and converted into 3-oxoadipyl-CoA. Examples of the enzyme that can catalyze the present conversion include a β-ketothiolase. Furthermore, for example, enzymes classified into the group such as EC 2.3.1.9 (acetoacetyl-CoA thiolase), EC 2.3.1.16 (3-ketoacyl-CoA thiolase), or EC 2.3.1.174 (3-oxoadipyl-CoA thiolase) can also be exemplified as enzymes that can have an activity for the present conversion. The enzyme that can be used in the present conversion is not limited as long as it has an activity for the present conversion, and examples thereof include a succinyl-CoA:acetyl-CoA acyltransferase and a 3-oxoadipyl-CoA thiolase. In one embodiment, Escherichia coli-derived PaaJ consisting of an amino acid sequence set forth in SEQ ID NO: 1 is used.

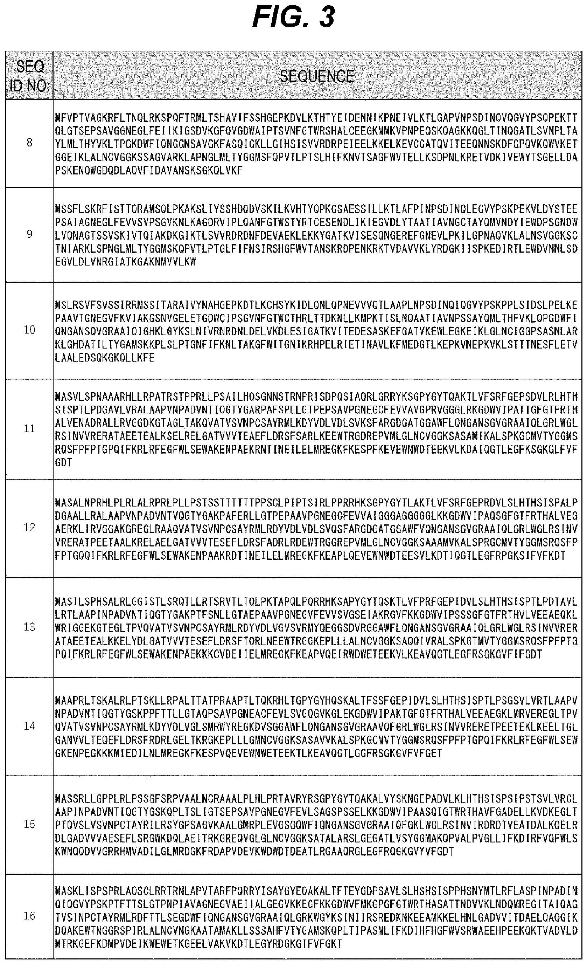

In Step B in Fig. 1, 3-oxoadipyl-CoA is converted into 3-hydroxyadipyl-CoA. Examples of the enzyme that can catalyze the present conversion include oxidoreductases classified into the group of EC 1.1.1. For example, enzymes classified into the group of EC 1.1.1.35 (3-hydroxyacyl-CoA dehydrogenase), EC 1.1.1.36 (acetoacetyl-CoA dehydrogenase), EC 1.1.1.157 (3-hydroxybutanoyl-CoA dehydrogenase), EC 1.1.1.211 (long chain 3-hydroxyacyl-CoA dehydrogenase), or EC 1.1.1.259 (3-hydroxypimeloyl-CoA dehydrogenase) can be exemplified as enzymes that can have an activity for the present conversion. The enzyme that can be used in the present invention is not limited as long as it has an activity for the present conversion, and examples thereof include a 3-hydroxyadipyl-CoA dehydrogenase. In one embodiment, Escherichia coli-derived PaaH consisting of an amino acid sequence set forth in SEQ ID NO: 2 is used.

In Step C in Fig. 1, 3-hydroxyadipyl-CoA is converted into 2,3-dehydroadipyl-CoA. Examples of the enzyme that can catalyze the present conversion include hydrolyases classified into the group of EC 4.2.1. For example, enzymes classified into the group of EC 4.2.1.17 (enoyl-CoA hydratase), EC 4.2.1.55 (3-hydroxybutanoyl-CoA dehydratase), or EC 4.2.1.74 (long chain enoyl-CoA hydratase) can be exemplified as enzymes that can have an activity for the present conversion. The enzyme that can be used in the present invention is not limited as long as it has an activity for the present conversion, and examples thereof include a 3-hydroxyadipyl-CoA dehydratase. In one embodiment, Escherichia coli-derived PaaF consisting of an amino acid sequence set forth in SEQ ID NO: 3 is used. In addition, as another embodiment, as the enzyme, 3-hydroxyadipyl-CoA dehydratase PaaF(L3) encoded by a base sequence (783 bp) of a PCR amplification product obtained using a nucleotide set forth in SEQ ID NO: 4 and a nucleotide set forth in SEQ ID NO: 46 as primers and a chromosomal DNA of the genus Burkholderia (Burkholderia sp.) LEBP-3 strain as a template, is used. The Burkholderia sp. LEBP-3 strain (hereinafter, also abbreviated as "L3 strain") has been deposited with National Institute of Technology and Evaluation, Patent Microorganisms Depositary (NPMD) (Address: Room 122, 2-5-8 Kazusa-Kamatari, Kisarazu, Chiba) on December 4, 2020 (Receipt Number: NITE ABP-03334), and then internationally deposited under "Accession Number: NITE BP-03334".

Here, as the DNA polymerase used for preparing the PCR amplification product, a known DNA polymerase in the art is used, and examples thereof include, but are not limited to, a Taq DNA polymerase, a hot start adjusted DNA polymerase, and a proofreading DNA polymerase having a 3'-5' exonuclease activity separately from a polymerase activity. In a preferred embodiment of the conditions for preparing the PCR amplification product, a PCR amplification product is prepared by performing 30 cycles of a treatment with an amount of 25 µL of a solution under conditions of a heat treatment at 98°C for 10 seconds, annealing at 55°C for 15 seconds, and elongation at 72°C and 5 sec/kb using a 1 µM of specific nucleotide as a forward primer and a reverse primer, and using PrimeSTAR Max DNA Polymerase (trade name, manufactured by TAKARA BIO INC.) as an enzyme.

In Step D in Fig. 1, 2,3-dehydroadipyl-CoA is converted into adipyl-CoA. Examples of the enzyme that can catalyze the present conversion include oxidoreductases classified into the group of EC 1.3.1. For example, enzymes classified into the group of EC 1.3.1.8 (acyl-CoA dehydrogenase (NADP⁺)), EC 1.3.1.9 (enoyl-ACP reductase (NADH)), EC 1.3.1.38 (trans-2-enoyl-CoA reductase (NADP⁺)), EC 1.3.1.44 (trans-2-enoyl-CoA reductase (NAD⁺)), EC 1.3.1.86 (crotonyl-CoA reductase), EC 1.3.1.93 (long chain acyl-CoA reductase), or EC 1.3.1.104 (enoyl-ACP reductase (NADPH)) can be exemplified as enzymes that can have an activity for the present conversion. In the present invention, the 2,3-dehydroadipyl-CoA reductase described above is used for the conversion of Step D.

The 2,3-dehydroadipyl-CoA reductase used in the present invention is, for example, an enzyme derived from the following biological species such as Candida auris (SEQ ID NO: 8), Kluyveromyces marxianus (SEQ ID NO:9), Pichia kudriavzevii (SEQ ID NO:10), Thermothelomyces thermophilus (SEQ ID NO:11), Thermothielavioides terrestris (SEQ ID NO:12), Chaetomium thermophilum (SEQ ID NO:13), Podospora anserina (SEQ ID NO:14), Purpureocillium lilacinum (SEQ ID NO:15), and Pyrenophora teres (SEQ ID NO:16). Preferably, at least one of the enzymes consisting of the amino acid sequences set forth in SEQ ID NOs: 8 to 16 is used, and more preferably, at least one of the enzymes consisting of the amino acid sequences set forth in SEQ ID NOs: 11, 12, 13, 14, and 16 is used (Fig. 3).

In Step E in Fig. 1, adipyl-CoA is converted into adipic acid. Examples of the enzyme that can catalyze the present conversion include thioester hydrolases classified into the group of EC 3.1.2. For example, enzymes classified into the group of EC 3.1.2.1 (acetyl-CoA hydrolase) or EC 3.1.2.20 (acyl-CoA hydrolase) can be exemplified as enzymes that can have an activity for the present conversion. The enzyme that can be used in the present invention is not limited as long as it has an activity for the present conversion, and examples thereof include an adipyl-CoA hydrolase.

In addition, examples of another enzyme that can catalyze the reaction of Step E in Fig. 1 include a CoA-transferase classified into the group of EC 2.8.3. For example, enzymes classified into the group of EC 2.8.3.5 (3-oxoacid CoA transferase), EC 2.8.3.6 (3-oxoadipate CoA-transferase), or EC 2.8.3.18 (succinyl-CoA: acetyl-CoA-transferase) can be exemplified as enzymes that can have an activity for the present conversion. The enzyme that can be used in the present invention is not limited as long as it has an activity for the present conversion, and examples thereof include an adipate-CoA transferase.

Furthermore, examples of conversion of another enzyme that can catalyze the reaction of Step E in Fig. 1 include a pathway that transfers the adipyl group of adipyl-CoA to a phosphate to form an adipyl phosphate by acyltransferases classified into the group of EC 2.3.1 and then undergoes dephosphorylation by phosphotransferases classified into group EC 2.7.2. For example, as an acyltransferase, enzymes classified into the group of EC 2.3.1.8 (phosphate acetyltransferase) or EC 2.3.1.19 (butyryl phosphate transferase) can be exemplified as enzymes that can have an activity for the present conversion. As a phosphotransferase, enzymes classified into the group of EC 2.7.2.1 (acetate kinase) or EC 2.7.2.7 (butanoate kinase) can be exemplified as enzymes that can have an activity for the present conversion. The enzyme that can be used in the present invention is not limited as long as it has an activity for the present conversion, and examples thereof include a phosphate adipyltransferase and an adipate kinase.

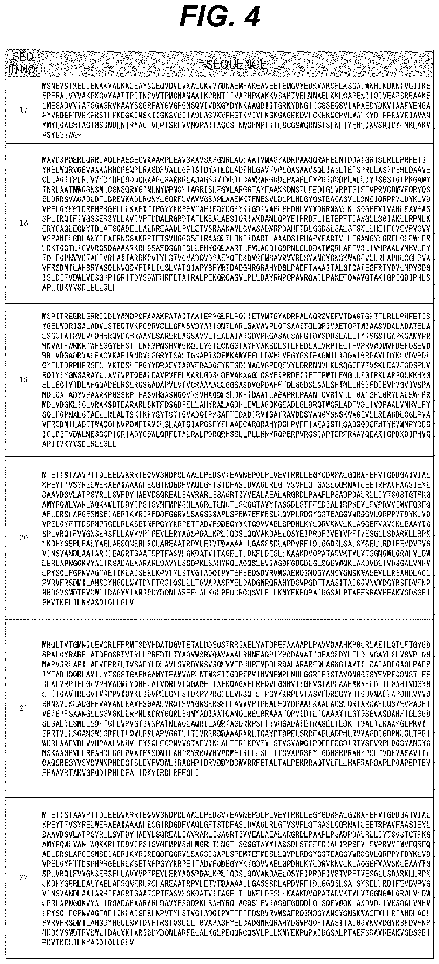

In Steps F, U, and V in Fig. 1, acyl-CoA is converted into an aldehyde. Examples of the enzyme that can catalyze the present conversion include enzymes classified into the group of EC 1.2.1. For example, enzymes classified into the group of EC 1.2.1.10 (acetaldehyde dehydrogenase (acetylation)), EC 1.2.1.17 (glyoxylate dehydrogenase (acylation)), EC 1.2.1.42 (hexadecanal dehydrogenase (acylation)), EC 1.2.1.44 (cinnamoyl-CoA reductase (acylation)), EC 1.2.1.75 (malonyl-CoA reductase (malonic semialdehyde formation)), or EC 1.2.1.76 (succinic semialdehyde dehydrogenase (acylation)) can be exemplified as enzymes that can have an activity for the present conversion because these enzymes catalyze a conversion reaction in which CoA is removed to produce an aldehyde similarly to the present conversion. The enzyme that can be used in the present invention is not limited as long as it has an activity for the present conversion, and for example, Clostridium kluyveri-derived sucD consisting of an amino acid sequence set forth in SEQ ID NO: 17 is used (Fig. 4).

In Steps G, I, K, and N in Fig. 1, a carboxyl group is converted into an aldehyde. Examples of the enzyme that can catalyze the present conversion include a carboxylic acid reductase (CAR). For example, enzymes classified into the group of EC 1.2.1.30 (carboxylic acid reductase (NADP⁺)), EC 1.2.1.31 (L-aminoadipate semialdehyde dehydrogenase), EC 1.2.1.95 (L-2-aminoadipate reductase), or EC 1.2.99.6 (carboxylic acid reductase) can be exemplified as enzymes that can have an activity for the present conversion because these enzymes catalyze a conversion reaction in which an aldehyde is generated from a carboxylic acid similarly to the present conversion. Typical examples of the biological species from which the enzyme is derived include, but are not limited to, Nocardia iowensis, Nocardia asteroides, Nocardia brasiliensis, Nocardia farcinica, Segniliparus rugosus, Segniliparus rotundus, Tsukamurella paurometabola, Mycobacterium marinum, Mycobacterium neoaurum, Mycobacterium abscessus, Mycobacterium avium, Mycobacterium chelonae, Mycobacterium immunogenum, Mycobacterium smegmatis, Serpula lacrymans, Heterobasidion annosum, Coprinopsis cinerea, Aspergillus flavus, Aspergillus terreus, Neurospora crassa, and Saccharomyces cerevisiae. The enzyme that can be used in the present invention is not limited as long as it has an activity for the present conversion, and for example, at least one of enzymes consisting of an amino acid sequence set forth in any of SEQ ID NOs: 18 to 22 may be used, at least one of enzyme MaCar derived from Mycobacterium abscessus consisting of an amino acid sequence set forth in SEQ ID NO: 20 and MaCar(m) that is a variant of MaCar consisting of an amino acid sequence set forth in SEQ ID NO: 22 is preferably used, and MaCar(m) consisting of an amino acid sequence set forth in SEQ ID NO: 22 is more preferably used (Fig. 4).

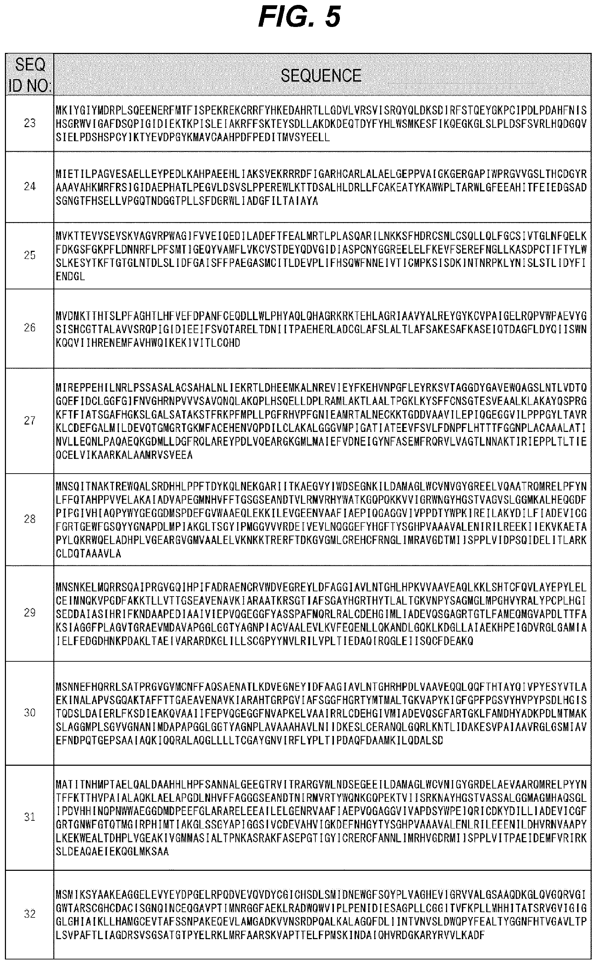

In addition, the carboxylic acid reductase can be converted into an active holoenzyme by phosphopantetinylation (Venkitasubramanian et al., Journal of Biological Chemistry, Vol. 282, No. 1, 478-485 (2007)). The phosphopantetinylation is catalyzed by a phosphopantetheinyl transferase (PT). Examples of the enzyme that can catalyze the present reaction include enzymes classified into EC 2.7.8.7. Therefore, the microorganism of the present invention may be further modified to increase an activity of the phosphopantetheinyl transferase. Examples of a method of increasing the activity of the phosphopantetheinyl transferase include, but are not limited to, a method of introducing an exogenous phosphopantetheinyl transferase and a method of enhancing expression of an endogenous phosphopantetheinyl transferase. The enzyme that can be used in the present invention is not particularly limited as long as it has a pheosphopantetheinyl group transfer activity, and typical examples thereof include EntD of Escherichia coli, Sfp of Bacillus subtilis, Npt of Nocardia iowensis (Venkitasubramanian et al., Journal of Biological Chemistry, Vol. 282, No.1, 478-485 (2007)), and Lys5 of Saccharomyces cerevisiae (Ehmann et al., Biochemistry 38.19 (1999): 6171-6177). The enzyme that can be used in the present invention is not limited as long as it has an activity for the present conversion, and for example, at least one of enzymes consisting of amino acid sequences set forth in SEQ ID NOs: 23 to 26 may be used, and Npt of Nocardia iowensis derived from Nocardia iowensis consisting of an amino acid sequence set forth in SEQ ID NO: 24 is preferably used (Fig. 5).

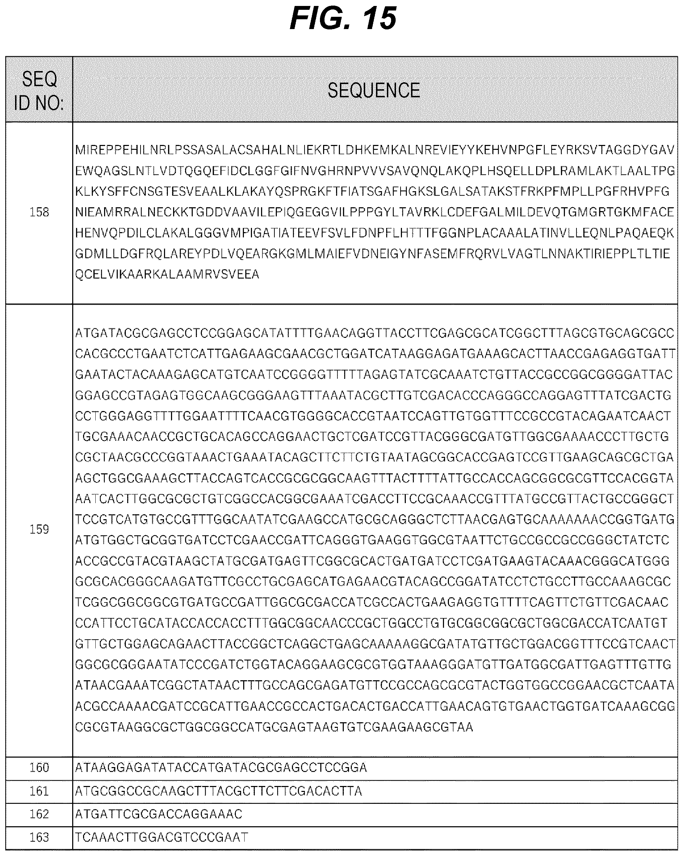

The conversions of Steps J, M, P, and R in Fig. 1 are transamination reactions. Examples of the enzyme that can catalyze the conversion include a transaminase (aminotransferase) classified in the group of EC 2.6.1. For example, enzymes classified into the group of EC 2.6.1.19 (4-aminobutanoate-2-oxoglutarate transaminase), EC 2.6.1.29 (diamine transaminase), or EC 2.6.1.48 (5-aminovalerate transaminase) can be exemplified as enzymes that can have an activity for the present conversion. The enzyme that can be used in the present invention is not particularly limited as long as it has the conversion activity of each step, for example, YgjG that is a putrescine aminotransferase of Escherichia coli reported to transaminate cadaverine and spermidine (Samsonova., et al., BMC microbiology 3.1 (2003): 2), SpuC that is a putrescine aminotransferase of the genus Pseudomonas (Lu et al., Journal of bacteriology 184.14 (2002): 3765-3773, Galman et al., Green Chemistry 19.2 (2017): 361-366), or GABA aminotransferase GabT or PuuE of Escherichia coli may also be used. Furthermore, it is reported that an ω-transaminase derived from a biological species such as Ruegeria pomeroyi, Chromobacterium violaceum, Arthrobacter citreus, Sphaerobacter thermophilus, Aspergillus fischeri, Vibrio fluvialis, Agrobacterium tumefaciens, or Mesorhizobium loti also has a transamination activity to a diamine compound such as 1,8-diaminooctane or 1,10-diaminodecane, and these enzymes may be used in the present invention (Sung et al., Green Chemistry 20.20 (2018): 4591-4595, Sattler et al., Angewandte Chemie 124.36 (2012): 9290-9293). The enzyme that can be used in the present invention is not limited as long as it has an activity for the present conversion, and for example, at least one of enzymes consisting of amino acid sequences set forth in SEQ ID NOs: 27 to 31 and 158 may be used (Figs. 5 and 15). Examples of a typical amino group donor include, but are not limited to, L-glutamic acid, L-alanine, and glycine.

In Steps H, L, O, and Q in Fig. 1, aldehydes are converted into alcohols. Examples of the enzyme that can catalyze the present conversion include oxidoreductases classified into the group of EC 1.1.1. For example, enzymes classified in the group of EC 1.1.1.1 (alcohol dehydrogenase), EC 1.1.1.2 (alcohol dehydrogenase (NADP⁺)), or EC 1.1.1.71 (alcohol dehydrogenase [NAD(P)⁺]) can be exemplified as enzymes that can have an activity for the present conversion because these enzymes catalyze a reaction in which an aldehyde is converted into an alcohol similarly to the present conversion. The enzyme used in the present invention is not limited as long as it has an activity for the present conversion, and for example, is Escherichia coli-derived Ahr set forth in SEQ ID NO: 32 (Fig. 5).

In the reactions of Steps S and T in Fig. 1, CoA is added to a carboxyl group. Examples of the enzyme that can catalyze the present conversion include CoA transferases classified into the group of EC 2.8.3 and acid-thiol ligases classified into the group of EC 6.2.1. The enzyme used in the present invention is not limited as long as it has an activity for the present conversion.

The gene encoding the enzyme that can be used in the present invention may be derived from an organism other than the exemplified organism or artificially synthesized, and may be any gene that can express a substantial enzyme activity in a host microorganism cell.

The recombinant microorganism according to the present invention may be a recombinant microorganism obtained by disrupting any gene as appropriate in a host microorganism. The target gene is disrupted by a method known in the art.

In addition, the amino acid sequence of the enzyme or the base sequence of the gene encoding the enzyme that can be used in the present invention can contain all mutations that can occur in nature or artificially introduced mutations and modifications as long as a substantial enzyme activity can be expressed by the host microorganism, and can contain, for example, mutations such as deletion, substitution, insertion, and addition. For example, the enzyme may have an amino acid sequence in which 1 or more, preferably 1 to 20, more preferably 1 to 10, still more preferably 1 to 7, further still more preferably 1 to 5, and particularly preferably 1 to 3 amino acids are deleted, substituted, inserted, and/or added in the amino acid sequence of the enzyme described above.

Furthermore, as the base sequence of the gene encoding the enzyme that can be used in the present invention, a DNA that hybridizes with a DNA having a complementary base sequence under a stringent condition can also be used as long as it can express a substantial enzymatic activity in the host microorganism cell. The "stringent condition" is, for example, a condition of about "1xSSC, 0.1%SDS, 60°C", a more severe condition of about "0.1xSSC, 0.1%SDS, 60°C", and a still more severe condition of about "0.1xSSC, 0.1%SDS, 68°C".

In addition, it is known that there are extra codons in various codons encoding a specific amino acid, and therefore, alternative codons that are to be finally translated to the same amino acid may also be used in the present invention. That is, since a genetic code degenerates, a plurality of codons can be used to encode a specific amino acid, such that the amino acid sequence can be encoded by an arbitrary set of similar DNA oligonucleotides. It should be noted that since most organisms are known to preferentially use a subset of a specific codon (optimal codon) (Gene, Vol. 105, pp. 61-72, 1991, and the like), performing of "codon optimization" in accordance with a host microorganism can also be useful in the present invention.

Therefore, the genetically modified microorganism according to the present invention can have a base sequence having a sequence identity of, for example, 80% or higher, 85% or higher, 88% or higher, 90% or higher, 93% or higher, 95% or higher, 97% or higher, 98% or higher, or 99% or higher with the base sequence of the enzyme gene under a condition in which an enzymatic activity can be expressed. Alternatively, the genetically modified microorganism can contain a gene encoding a protein having an amino acid sequence having a sequence identity, for example, 80% or higher, 85% or higher, 88% or higher, 90% or higher, 95% or higher, 97% or higher, 98% or higher, or 99% or higher with the amino acid sequence of the enzyme.

In the present specification, a proportion (%) of a "sequence identity" of a comparative amino acid sequence to a reference amino acid sequence is defined as a percentage of amino acid residues in the comparative sequence that are identical to the amino acid residues in the reference sequence when the sequences are aligned so that the identity between the two sequences is maximized, and if necessary, a gap is introduced into one or both of the two sequences. In this case, a conservative substitution is not considered as a part of the sequence identity. The sequence identity can be determined by using publicly available computer software, for example, using an alignment search tool such as Basic Local Alignment Search Tool (abbreviated as BLAST, registered trademark). In an alignment, those skilled in the art can determine appropriate parameters to obtain the maximum alignment of the comparative sequence. The "sequence identity" of the nucleotide sequence can also be determined by a similar method.

In the present invention, when the C6 compound biosynthesis enzyme gene is introduced into a host microorganism cell as an "expression cassette", a more stable and high level of enzymatic activity can be obtained. In the present specification, the "expression cassette" refers to a nucleotide having a nucleic acid sequence that is functionally linked to a nucleic acid to be expressed or a gene to be expressed and regulates transcription and translation. Typically, the expression cassette of the present invention contains a promoter sequence on the 5' upstream from the coding sequence, a terminator sequence on the 3' downstream, and an optionally additional normal regulatory element in a functionally linked state, and in such a case, a nucleic acid to be expressed or a gene to be expressed is introduced into a host microorganism.

The promoter is defined as a DNA sequence that allows RNA polymerase to bind to DNA to initiate RNA synthesis, regardless of whether the promoter is a constitutive expression promoter or an inductive expression promoter. A strong promoter is a promoter that initiates mRNA synthesis at a high frequency, and is also suitably used in the present invention. In Escherichia coli, a lac system, a trp system, a tac or trc system, or a major operator and promoter region of a λ-phage, a regulatory region for fd coat protein, a promoter for a glycolytic enzyme (for example, 3-phosphoglycerate kinase or glyceraldehyde-3-phosphate dehydrogenase), glutamate decarboxylase A, or serine hydroxymethyltransferase, a promoter region of RNA polymerase derived from T7 phage, and the like can be used. In Corynebacterium glutamicum, a high-level constitutive expression (HCE) promoter, a cspB promoter, a sodA promoter, an elongation factor (EF-Tu) promoter, and the like can be used. As a terminator, a T7 terminator, an rrnBT1T2 terminator, a lac terminator, and the like can be used. In addition to the promoter and terminator sequences, other examples of regulatory elements include a selection marker, an amplification signal, and a replication point. A suitable regulatory sequence is described in, for example, "Gene Expression Technology: Methods in Enzymology 185" Academic Press (1990)".

The expression cassette described above is incorporated into a vector consisting of, for example, a plasmid, a phage, a transposon, an IS element, a fosmid, a cosmid, or a linear or cyclic DNA and is inserted into a host microorganism. A plasmid and a phage are preferred. The vector may be self-replicated in a host microorganism or may be replicated by a chromosome. Examples of a preferred plasmid include pLG338, pACYC184, pBR322, pUC18, pUC19, pKC30, pRep4, pHS1, pKK223-3, pDHE19.2, pHS2, pPLc236, pMBL24, pLG200, pUR290, pIN-III113-B1, Agt11, or pBdCI of Escherichia coli; pUB110, pC194, or pBD214 of a bacillus; and pSA77 or pAJ667 of the genus Corynebacterium. Examples of a bacillus such as the genus Bacillus include pUB110, pC194, and pBD214. Other usable plasmids and the like are described in "Gene Cloning and DNA analysis 7th edition", Wiley-Blackwell (2016). The expression cassette can be introduced into the vector by a conventional method of including cutting with an appropriate restriction enzyme, cloning, and ligation. Each expression cassette may be located on one vector or on two or more vectors.

After the vector having the expression cassette of the present invention is constructed as described above, a conventional method can be used as a method that can be applied when the vector is introduced into a host microorganism. Examples of the method include, but are not limited to, a calcium chloride method, an electroporation method, a conjugation transfer method, and a protoplast fusion method, and a method suitable for a host microorganism can be selected.

A second aspect of the present invention relates to a method for producing a target compound, the method including a culture step of culturing the recombinant microorganism described above.

Here, the compound is preferably a C6 compound. The C6 compound is at least one selected from the group consisting of adipic acid, hexamethylenediamine, 1,6-hexanediol, 6-aminohexanoic acid, 6-amino-1-hexanol, and 6-hydroxyhexanoic acid.

The production method of a C6 compound includes a culture step of culturing the genetically modified microorganism according to the present invention. In the culture step, a culture containing microbial cells is obtained by culturing the recombinant microorganism in a medium containing a carbon source and a nitrogen source. The genetically modified microorganism of the present invention is cultured under conditions suitable for the C6 compound production and the growth and maintenance of the microorganism, and suitable medium composition, culture time, and culture conditions can be easily set by those skilled in the art.

Examples of the carbon source include D-glucose, sucrose, lactose, fructose, maltose, oligosaccharides, polysaccharides, starch, cellulose, rice bran, blackstrap molasses, fats and oils (for example, soybean oil, sunflower oil, peanut oil, palm oil, and the like), fatty acids (for example, palmitic acid, linoleic acid, oleic acid, linolenic acid, and the like), alcohols (for example, glycerol, ethanol, and the like), organic acids (for example, acetic acid, lactic acid, succinic acid, and the like), corn decomposition liquids, and cellulose decomposition liquids. Preferably, the carbon source is D-glucose, sucrose, or glycerol. These carbon sources can be used alone or as a mixture.

The C6 compound produced using a biomass-derived raw material can be clearly distinguished from a synthetic raw material derived from, for example, petroleum, natural gas, or coal, by measurement of a biomass carbon content based on Carbon-14 (radiocarbon) analysis defined in ISO 16620-2 or ASTM D6866.

Examples of the nitrogen source include nitrogen-containing organic compounds (for example, peptone, casamino acid, tryptone, a yeast extract, a meat extract, a malt extract, a corn steep liquor, soy flour, an amino acid, urea, and the like) and inorganic compounds (for example, an aqueous ammonia solution, ammonium sulfate, ammonium chloride, ammonium phosphate, ammonium carbonate, sodium nitrate, ammonium nitrate, and the like). These nitrogen sources can be used alone or as a mixture.

In addition, the medium may contain a corresponding antibiotic in a case where a recombinant microorganism expresses a useful additional trait, for example, in a case where a recombinant microorganism contains a marker resistant to an antibiotic. Therefore, a risk of contamination by various bacteria during culture is reduced. Examples of the antibiotic include, but are not limited to, a β-lactam antibiotic such as ampicillin, an aminoglycoside antibiotic such as kanamycin, a macrolide antibiotic such as erythromycin, a tetracycline antibiotic, and chloramphenicol.

The culture may be batch or continuous. In addition, in any case, an additional carbon source or the like may be supplied at an appropriate time point of culture. Furthermore, the culture may be performed while conditions such as a preferred temperature, oxygen concentration, and pH are controlled. A suitable culture temperature of a general transformant derived from a microbial host cell is usually 15°C to 55°C and preferably 25°C to 40°C. In a case where the host microorganism is aerobic, shaking (flask culture or the like), agitation/aeration (jar fermenter culture or the like) may be performed to ensure a suitable oxygen concentration during fermentation. These culture conditions can be easily set by those skilled in the art.

The culture step may include obtaining a culture of the recombinant microorganism and/or an extract of the culture.

The production method according to the present invention may further include a mixing step of mixing the culture and/or the extract of the culture with a substrate compound to obtain a mixed solution. The substrate compound may be appropriately selected according to the enzyme and the target compound.

The production method of a compound according to the present invention may include a step of separating and/or purifying a C6 compound that is a target compound. In the separation and/or purification step, examples of any technique include, but are not limited to, centrifugation, filtration, membrane separation, crystallization, extraction, distillation, adsorption, phase separation, ion exchange, and various types of chromatography. In the separation and/or purification step, one type of method may be selected, or a plurality of methods may be combined.

A further aspect of the present invention relates to a protein. Specifically, the protein is a recombinant protein:
(a) having an enzymatic activity to reduce 2,3-dehydroadipyl-CoA for conversion into adipyl-CoA, and
(b) satisfying any one of the followings:
   (i) consisting of an amino acid sequence having a sequence identity of 80% or higher, 85% or higher, 88% or higher, 90% or higher, 93% or higher, 95% or higher, 97% or higher, 98% or higher, or 99% or higher with an amino acid sequence set forth in SEQ ID NO: 8, 9, 10, 11, 12, 13, 14, 15, or 16;
   (ii) consisting of an amino acid sequence in which 1 to 10, 1 to 7, 1 to 5, or 1 to 3 amino acids are deleted, substituted, inserted, and/or added in an amino acid sequence set forth in SEQ ID NO: 8, 9, 10, 11, 12, 13, 14, 15, or 16;
   (iii) encoded by a DNA consisting of a polynucleotide sequence having a sequence identity of 80% or higher, 85% or higher, 88% or higher, 90% or higher, 93% or higher, 95% or higher, 97% or higher, 98% or higher, or 99% or higher with a polynucleotide sequence set forth in SEQ ID NO: 57, 58, 59, 60, 61, 62, 63, 64, or 65;
   (iv) consisting of an amino acid sequence in which 1 to 10, 1 to 7, 1 to 5, or 1 to 3 amino acids are deleted, substituted, inserted, and/or added in an amino acid sequence of a protein encoded by a polynucleotide sequence set forth in SEQ ID NO: 57, 58, 59, 60, 61, 62, 63, 64, or 65;
   (v) encoded by a DNA hybridizing with a DNA consisting of a polynucleotide sequence complementary to a polynucleotide sequence set forth in SEQ ID NO: 57, 58, 59, 60, 61, 62, 63, 64, or 65 under a stringent condition; and
   (vi) encoded by a DNA consisting of a degenerate isomer of a polynucleotide sequence set forth in SEQ ID NO: 57, 58, 59, 60, 61, 62, 63, 64, or 65.

The protein is preferably a recombinant protein:
(a) having an enzymatic activity to reduce 2,3-dehydroadipyl-CoA for conversion into adipyl-CoA, and
(b) satisfying any one of the followings:
   (i) consisting of an amino acid sequence having a sequence identity of 80% or higher with an amino acid sequence set forth in SEQ ID NO: 8, 9, 10, 11, 12, 13, 14, 15, or 16; (ii) consisting of an amino acid sequence in which 1 to 10 amino acids are deleted, substituted, inserted, and/or added in an amino acid sequence set forth in SEQ ID NO: 8, 9, 10, 11, 12, 13, 14, 15, or 16;
   (iii) encoded by a DNA consisting of a polynucleotide sequence having a sequence identity of 80% or higher with a polynucleotide sequence set forth in SEQ ID NO: 57, 58, 59, 60, 61, 62, 63, 64, or 65;
   (iv) consisting of an amino acid sequence in which 1 to 10 amino acids are deleted, substituted, inserted, and/or added in an amino acid sequence of a protein encoded by a polynucleotide sequence set forth in SEQ ID NO: 57, 58, 59, 60, 61, 62, 63, 64, or 65;
   (v) encoded by a DNA hybridizing with a DNA consisting of a polynucleotide sequence complementary to a polynucleotide sequence set forth in SEQ ID NO: 57, 58, 59, 60, 61, 62, 63, 64, or 65 under a stringent condition; and
   (vi) encoded by a DNA consisting of a degenerate isomer of a polynucleotide sequence set forth in SEQ ID NO: 57, 58, 59, 60, 61, 62, 63, 64, or 65.

In a preferred embodiment, the recombinant protein:
(xi) consisting of an amino acid sequence set forth in SEQ ID NO: 8, 9, 10, 11, 12, 13, 14, 15, or 16; or
(xiii) encoded by a DNA consisting of a polynucleotide sequence set forth in SEQ ID NO: 57, 58, 59, 60, 61, 62, 63, 64, or 65.

Still another aspect of the present invention relates to a method for producing a C6 compound, the method including using the protein described above.

As described above, according to the present invention, a recombinant microorganism having an excellent 2,3-dehydroadipyl-CoA reductase activity can be obtained by introducing a gene encoding a protein having the 2,3-dehydroadipyl-CoA reductase activity into a host microorganism. In addition, a protein having an excellent 2,3-dehydroadipyl-CoA reductase activity is also provided.

A target C6 compound can be efficiently produced using the aforementioned recombinant microorganism or the aforementioned protein. In addition, various C6 compounds can be obtained by performing conversion using an additional enzyme according to a desired C6 compound. Furthermore, since the recombinant microorganism according to the present invention has an excellent C6 compound production ability, it is expected that the recombinant microorganism can be used for production of the target compound on an industrial scale.

### <2> Invention B: Recombinant Microorganism and Production Method of Adipic Acid or Derivative Thereof

The recombinant microorganism according to the present invention is a microorganism obtained by genetically modifying a host microorganism so that adipic acid (ADA) (CAS No. 124-04-9) or an adipic acid derivative can be efficiently produced.

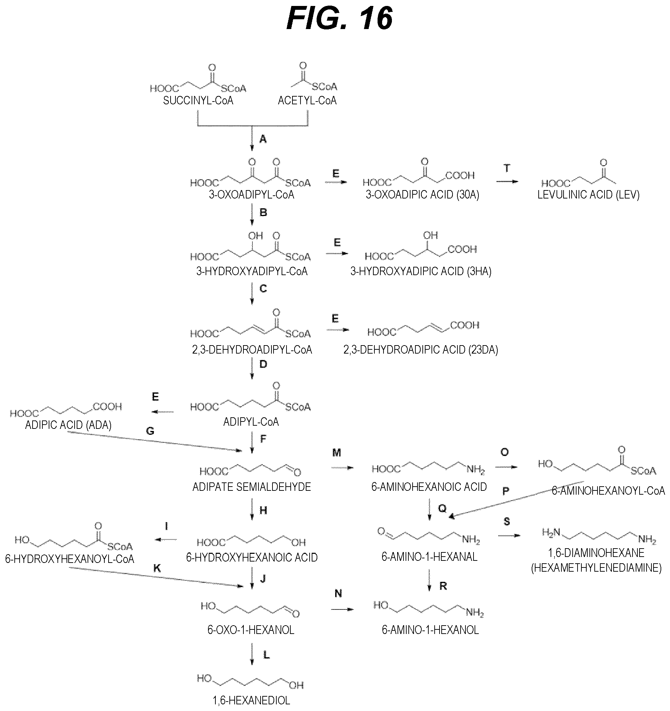

In the present specification, the "adipic acid derivative" is an aliphatic carboxylic acid having an aliphatic hydrocarbon group having 3 to 5 carbon atoms and one or a plurality of carboxyl groups. The aliphatic hydrocarbon group of the adipic acid derivative is saturated or unsaturated. In the aliphatic hydrocarbon group, carbon may be substituted with a substituent such as a hydroxyl group, a carboxyl group, a ketone group, or an amino group. The number of carbon atoms of the aliphatic hydrocarbon group of the "adipic acid derivative" is preferably 3 or 4. The number of carboxyl groups of the "adipic acid derivative" is preferably 1 or 2. Specific examples of the "adipic acid derivative" include 3-oxoadipic acid (3-OA) having an aliphatic hydrocarbon group having 4 carbon atoms and two carboxylic acids, levulinic acid (LEV) having an aliphatic hydrocarbon group 3 carbon atoms and one carboxylic acid, 3-hydroxyadipic acid (3HA) having an aliphatic hydrocarbon group 4 carbon atoms and two carboxylic acids, and 2,3-dehydroadipic acid (23DA) having an aliphatic hydrocarbon group having 4 carbon atoms and two carboxylic acids (see Fig. 16).

It is understood by those skilled in the art that the adipic acid and the adipic acid derivative in the present specification can take a neutral or ionized form including any salt form and this form is pH-dependent. Therefore, the terms "adipic acid" and "adipic acid derivative" encompass any salt form and ionized form in addition to the free acid form. In the present specification, the adipic acid and adipic acid derivative may be collectively referred to as "adipic acid" or an "adipic acid derivative".

It is also possible to synthesize 6-aminocaproic acid, hexamethylenediamine, 1,6-hexanediol, and 6-amino-1-hexanol using adipic acid produced by the recombinant microorganism according to the present invention as a raw material. The synthesis method may be a chemical synthesis method, a biological synthesis method using an enzyme or a microorganism in which the enzyme is present, or the like. In addition, when the microorganism has a metabolic pathway for converting adipic acid into the above compound, metabolic conversion from a raw material using adipic acid as an intermediate in the cell is also possible.

The genetically modified microorganism according to the present invention contains at least one of an exogenous gene encoding a 3-hydroxyadipyl-CoA dehydratase and an exogenous gene encoding a 2,3-dehydroadipyl-CoA reductase. By containing at least one of such exogenous genes, the recombinant microorganism obtains an adipic acid production pathway, and adipic acid or an adipic acid derivative can be thus produced. In the present specification, the "genetically modified microorganism" is simply referred to as "recombinant microorganism".

At least one of the 3-hydroxyadipyl-CoA dehydratase and the 2,3-dehydroadipyl-CoA reductase is derived from the genus Burkholderia (Burkholderia sp.) LEBP-3 strain (Accession Number: NITE BP-03334). Preferably, both the 3-hydroxyadipyl-CoA dehydratase and the 2,3-dehydroadipyl-CoA reductase are derived from the genus Burkholderia (Burkholderia sp.) LEBP-3 strain.

At least one of the exogenous gene encoding a 3-hydroxyadipyl-CoA dehydratase and the exogenous gene encoding a 2,3-dehydroadipyl-CoA reductase is derived from the genus Burkholderia (Burkholderia sp.) LEBP-3 strain (Accession Number: NITE BP-03334). Preferably, both the exogenous gene encoding a 3-hydroxyadipyl-CoA dehydratase and the exogenous gene encoding a 2,3-dehydroadipyl-CoA reductase are derived from the genus Burkholderia (Burkholderia sp.) LEBP-3 strain (Accession Number: NITE BP-03334) .

In the genetically modified microorganism of the present invention, at least one of the exogenous genes encoding the enzyme may be introduced. In the genetically modified microorganism of the present invention, preferably, at least one of exogenous genes encoding an enzyme that catalyzes each reaction step of the adipic acid production pathway is introduced, and an exogenous enzyme is expressed. In addition, in a case where the genetically modified microorganism of the present invention expresses a sufficient amount of enzyme that catalyzes a reaction stage of an adipic acid production pathway without introduction of an exogenous gene, the reaction may proceed by the enzyme encoded by the endogenous gene.

The recombinant microorganism according to the present invention preferably expresses at least one of a 3-hydroxyadipyl-CoA dehydratase and a 2,3-dehydroadipyl-CoA reductase. The recombinant microorganism according to the present invention expresses at least one of these enzymes, such that the reaction stage of the adipic acid production pathway can proceed, and a target compound can be produced.

More preferably, the recombinant microorganism according to the present invention expresses both a 3-hydroxyadipyl-CoA dehydratase and a 2,3-dehydroadipyl-CoA reductase. The recombinant microorganism according to the present invention expresses these enzymes, such that each reaction stage of the adipic acid production pathway can proceed, and a target compound can be produced.

Still more preferably, the recombinant microorganism according to the present invention expresses an oxidoreductase and/or a β-ketothiolase, in addition to the 3-hydroxyadipyl-CoA dehydratase and the 2,3-dehydroadipyl-CoA reductase. The recombinant microorganism according to the present invention expresses these enzymes, such that each reaction stage of the adipic acid production pathway can proceed, and a target compound can be more efficiently produced (see Fig. 16).

Hereinafter, each of the enzymes encoded by the gene contained in the recombinant microorganism according to the present invention will be described in detail.

### <3-Hydroxyadipyl-CoA Dehydratase>

The 3-hydroxyadipyl-CoA dehydratase is an enzyme that catalyzes the reaction of converting 3-hydroxyadipyl-CoA into 2,3-dehydroadipyl-CoA (Step C in Fig. 16).

In one embodiment, the 3-hydroxyadipyl-CoA dehydratase is encoded by a DNA having a sequence identity of 65% or higher, 70% or higher, 75% or higher, 80% or higher, 85% or higher, 88% or higher, 90% or higher, 93% or higher, 95% or higher, 97% or higher, 98% or higher, or 99% or higher with a PCR amplification product obtained using a chromosomal DNA of the genus Burkholderia (Burkholderia sp.) LEBP-3 strain as a template and using a predetermined nucleotide as a primer. Here, the DNA has, for example, 626 to 940 bp, preferably 740 to 862 bp, more preferably 743 to 823 bp, still more preferably 767 to 799 bp, particularly preferably 775 to 791 bp, and most preferably 778 to 788 bp. The primer used in PCR will be described below.

In a preferred embodiment, the 3-hydroxyadipyl-CoA dehydratase is encoded by a DNA that is a PCR amplification product obtained using a chromosomal DNA of the genus Burkholderia (Burkholderia sp.) LEBP-3 strain as a template and using a predetermined nucleotide as a primer. The DNA has, for example, 626 to 940 bp, preferably 740 to 862 bp, more preferably 743 to 823 bp, still more preferably 767 to 799 bp, particularly preferably 775 to 791 bp, and most preferably 778 to 788 bp.

In a further embodiment, the 3-hydroxyadipyl-CoA dehydratase is selected from:
(A-1) a recombinant polypeptide encoded by a DNA having a sequence identity of 65% or higher, 70% or higher, 75% or higher, 80% or higher, 85% or higher, 88% or higher, 90% or higher, 93% or higher, 95% or higher, 97% or higher, 98% or higher, or 99% or higher with a PCR amplification product obtained using a chromosomal DNA of the genus Burkholderia (Burkholderia sp.) LEBP-3 strain as a template and using a predetermined nucleotide as a primer,
   in which the recombinant polypeptide has a 3-hydroxyadipyl-CoA dehydratase activity, and
   the DNA has the base pair number (bp) described above;
(A-2) a recombinant polypeptide consisting of an amino acid sequence in which 1 to 10, preferably 1 to 7, more preferably 1 to 5, and still more preferably 1 to 3 amino acids are deleted, substituted, inserted, and/or added in an amino acid sequence of a polypeptide encoded by a DNA that is a PCR amplification product obtained using a chromosomal DNA of the genus Burkholderia (Burkholderia sp.) LEBP-3 strain as a template and using a predetermined nucleotide as a primer,
   in which the recombinant polypeptide has a 3-hydroxyadipyl-CoA dehydratase activity, and
   the DNA has the base pair number (bp) described above;
   and
(A-3) a recombinant polypeptide encoded by a DNA consisting of a degenerate isomer of a PCR amplification product obtained using a chromosomal DNA of the genus Burkholderia (Burkholderia sp.) LEBP-3 strain as a template and using a predetermined nucleotide as a primer,
   in which the recombinant polypeptide has a 3-hydroxyadipyl-CoA dehydratase activity, and
   the DNA has the base pair number (bp) described above.

In a more preferred embodiment, the 3-hydroxyadipyl-CoA dehydratase is a recombinant polypeptide encoded by a PCR amplification product obtained using a chromosomal DNA of the genus Burkholderia (Burkholderia sp.) LEBP-3 strain as a template and using a predetermined nucleotide as a primer, and a base pair number of the amplification product is 783 or 784 bp.

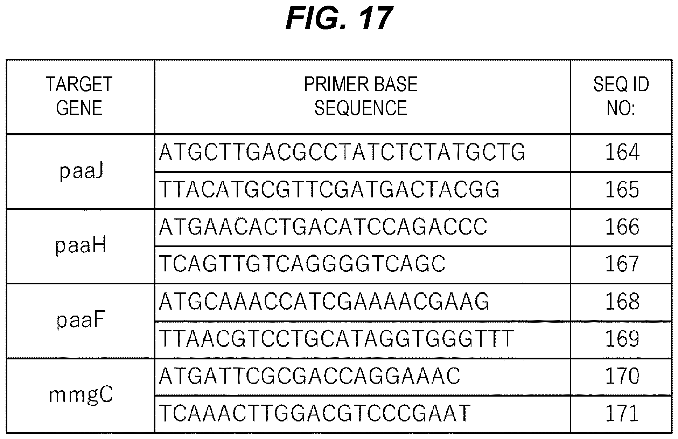

Regarding the 3-hydroxyadipyl-CoA dehydratase, primers (forward and reverse) used in PCR include the followings (see Fig. 17):
(1a) a nucleotide consisting of a base sequence set forth in SEQ ID NO: 168 and a nucleotide consisting of a base sequence set forth in SEQ ID NO: 169;
(1b) a nucleotide having a base sequence set forth in SEQ ID NO: 168 and a nucleotide having a base sequence set forth in SEQ ID NO: 169;
(1c) a nucleotide consisting of a base sequence obtained by codon-optimizing a base sequence set forth in SEQ ID NO: 168 and a nucleotide consisting of a base sequence obtained by codon-optimizing a base sequence set forth in SEQ ID NO: 169; and
(1d) a nucleotide having a base sequence obtained by codon-optimizing a base sequence set forth in SEQ ID NO: 168 and a nucleotide having a base sequence obtained by codon-optimizing a base sequence set forth in SEQ ID NO: 169.

The "nucleotide having a base sequence set forth in SEQ ID NO: 168 and the nucleotide having a base sequence set forth in SEQ ID NO: 169" of (1b) have additional nucleotide lengths of, for example, 1 to 20 bp, preferably 1 to 15 bp, more preferably 5 to 15 bp, and still more preferably 10 to 15 bp, in addition to the "base sequence set forth in SEQ ID NO: 168" and the "base sequence set forth in SEQ ID NO: 169", respectively.

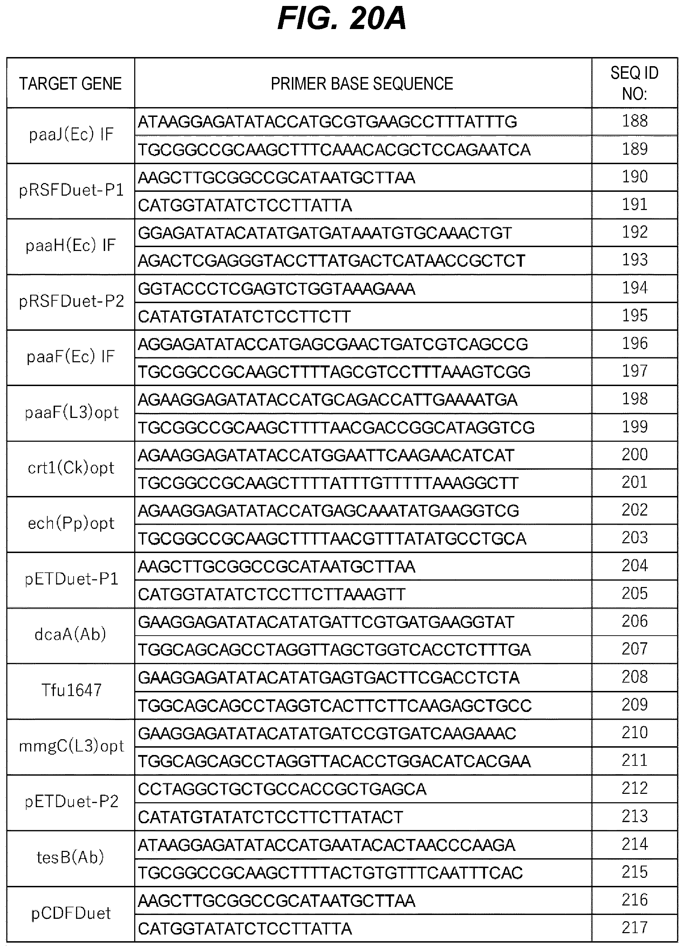

The "nucleotide having a base sequence obtained by codon-optimizing a base sequence set forth in SEQ ID NO: 168 and the nucleotide having a base sequence obtained by codon-optimizing a base sequence set forth in SEQ ID NO: 169" of (1d) have sequences homologous to vector ends, and have additional nucleotide lengths of, for example, 1 to 20 bp, preferably 1 to 15 bp, more preferably 5 to 15 bp, and still more preferably 10 to 15 bp, in addition to the "base sequence obtained by codon-optimizing the base sequence set forth in SEQ ID NO: 168" and the "base sequence obtained by codon-optimizing the base sequence set forth in SEQ ID NO: 169", respectively. Examples of the "nucleotide having a base sequence obtained by codon-optimizing a base sequence set forth in SEQ ID NO: 168 and the nucleotide having a base sequence obtained by codon-optimizing a base sequence set forth in SEQ ID NO: 169" of (1d) include:
(1d') a nucleotide consisting of a base sequence set forth in SEQ ID NO: 198 and a nucleotide consisting of a base sequence set forth in SEQ ID NO: 199 (Fig. 20A).

Regarding the 3-hydroxyadipyl-CoA dehydratase, preferred primers are:
(1a) a nucleotide consisting of a base sequence set forth in SEQ ID NO: 168 and a nucleotide consisting of a base sequence set forth in SEQ ID NO: 169, or
(1b) a nucleotide having a base sequence set forth in SEQ ID NO: 168 and a nucleotide having a base sequence set forth in SEQ ID NO: 169.

A DNA that is a PCR amplification product obtained using the nucleotide as a primer and a chromosomal DNA of the genus Burkholderia (Burkholderia sp.) LEBP-3 strain as a template has preferably 783 or 784 bp. Here, as the DNA polymerase used in PCR, a known DNA polymerase in the art is used, and examples thereof include a Taq DNA polymerase, a hot start adjusted DNA polymerase, and a proofreading DNA polymerase having a 3'-5' exonuclease activity separately from a polymerase activity.

### <2,3-Dehydroadipyl-CoA Reductase>

The 2,3-dehydroadipyl-CoA reductase is an enzyme that catalyzes the reaction of converting 2,3-dehydroadipyl-CoA into adipyl-CoA (see Step D in Fig. 16).

In one embodiment, the 2,3-dehydroadipyl-CoA reductase is encoded by a DNA having a sequence identity of 65% or higher, 70% or higher, 75% or higher, 80% or higher, 85% or higher, 88% or higher, 90% or higher, 93% or higher, 95% or higher, 97% or higher, 98% or higher, or 99% or higher with a PCR amplification product obtained using a chromosomal DNA of the genus Burkholderia (Burkholderia sp.) LEBP-3 strain as a template and using a predetermined nucleotide as a primer. The DNA has, for example, 924 to 1,386 bp, preferably 1,039 to 1,249 bp, more preferably 1,097 to 1,213 bp, still more preferably 1,131 to 1,179 bp, particularly preferably 1,143 to 1,167 bp, more particularly 1,145 to 1,165 bp, and most preferably 1,150 to 1,160 bp.

In a preferred embodiment, the 2,3-dehydroadipyl-CoA reductase is encoded by a DNA that is a PCR amplification product obtained using a chromosomal DNA of the genus Burkholderia (Burkholderia sp.) LEBP-3 strain as a template and using a predetermined nucleotide as a primer. The DNA has, for example, 924 to 1,386 bp, preferably 1,039 to 1,249 bp, more preferably 1,097 to 1,213 bp, still more preferably 1,131 to 1,179 bp, particularly preferably 1,143 to 1,167 bp, more particularly 1,145 to 1,165 bp, and most preferably 1,150 to 1,160 bp.

In a further aspect, the 2,3-dehydroadipyl-CoA reductase is selected from:
(B-1) a recombinant polypeptide encoded by a DNA having a sequence identity of 65% or higher, 70% or higher, 75% or higher, 80% or higher, 85% or higher, 88% or higher, 90% or higher, 93% or higher, 95% or higher, 97% or higher, 98% or higher, or 99% or higher with a PCR amplification product obtained using a chromosomal DNA of the genus Burkholderia (Burkholderia sp.) LEBP-3 strain as a template and using a predetermined nucleotide as a primer,
   in which the recombinant polypeptide has a 2,3-dehydroadipyl-CoA reductase activity, and
   the DNA has the base pair number (bp) described above;
(B-2) a recombinant polypeptide consisting of an amino acid sequence in which 1 to 10, preferably 1 to 7, more preferably 1 to 5, and still more preferably 1 to 3 amino acids are deleted, substituted, inserted, and/or added in an amino acid sequence of a polypeptide encoded by a DNA that is a PCR amplification product obtained using a chromosomal DNA of the genus Burkholderia (Burkholderia sp.) LEBP-3 strain as a template and using a predetermined nucleotide as a primer,
   in which the recombinant polypeptide has a 2,3-dehydroadipyl-CoA reductase activity, and
   the DNA has the base pair number (bp) described above;
   and
(B-3) a recombinant polypeptide encoded by a DNA consisting of a degenerate isomer of a PCR amplification product obtained using a chromosomal DNA of the genus Burkholderia (Burkholderia sp.) LEBP-3 strain as a template and using a predetermined nucleotide as a primer,
   in which the recombinant polypeptide has a 2,3-dehydroadipyl-CoA reductase activity, and
   the DNA has the base pair number (bp) described above.

In a more preferred aspect, the exogenous 2,3-dehydroadipyl-CoA reductase is a PCR amplification product obtained using a chromosomal DNA of the genus Burkholderia (Burkholderia sp.) LEBP-3 strain as a template and using a predetermined nucleotide as a primer, and a base pair number of the amplification product is 1,155 or 1,156 bp.

Regarding the 2,3-dehydroadipyl-CoA reductase, primers (forward and reverse) used in PCR include the followings (see Fig. 17):
(2a) a nucleotide consisting of a base sequence set forth in SEQ ID NO: 170 and a nucleotide consisting of a base sequence set forth in SEQ ID NO: 171;
(2b) a nucleotide having a base sequence set forth in SEQ ID NO: 170 and a nucleotide having a base sequence set forth in SEQ ID NO: 171;
(2c) a nucleotide consisting of a base sequence obtained by codon-optimizing a base sequence set forth in SEQ ID NO: 170 and a nucleotide consisting of a base sequence obtained by codon-optimizing a base sequence set forth in SEQ ID NO: 171; and
(2d) a nucleotide having a base sequence obtained by codon-optimizing a base sequence set forth in SEQ ID NO: 170 and a nucleotide having a base sequence obtained by codon-optimizing a base sequence set forth in SEQ ID NO: 171.

The "nucleotide having a base sequence set forth in SEQ ID NO: 170 and the nucleotide having a base sequence set forth in SEQ ID NO: 171" of (2b) have additional nucleotide lengths of, for example, 1 to 20 bp, preferably 1 to 15 bp, more preferably 5 to 15 bp, and still more preferably 10 to 15 bp, in addition to the "base sequence set forth in SEQ ID NO: 170" and the "base sequence set forth in SEQ ID NO: 171", respectively.

The "nucleotide having a base sequence obtained by codon-optimizing a base sequence set forth in SEQ ID NO: 170 and the nucleotide having a base sequence obtained by codon-optimizing a base sequence set forth in SEQ ID NO: 171" of (2d) have sequences homologous to vector ends, and have additional nucleotide lengths of, for example, 1 to 20 bp, preferably 1 to 15 bp, more preferably 5 to 15 bp, and still more preferably 10 to 15 bp, in addition to the "base sequence obtained by codon-optimizing the base sequence set forth in SEQ ID NO: 170" and the "base sequence obtained by codon-optimizing the base sequence set forth in SEQ ID NO: 171", respectively. Examples of the "nucleotide having a base sequence obtained by codon-optimizing a base sequence set forth in SEQ ID NO: 170 and the nucleotide having a base sequence obtained by codon-optimizing a base sequence set forth in SEQ ID NO: 171" of (2d) include:
(2d') a nucleotide consisting of a base sequence set forth in SEQ ID NO: 210 and a nucleotide consisting of a base sequence set forth in SEQ ID NO: 211 (Fig. 20A).

Regarding the 2,3-dehydroadipyl-CoA reductase, preferred primers are: (2a) a nucleotide consisting of a base sequence set forth in SEQ ID NO: 170 and a nucleotide consisting of a base sequence set forth in SEQ ID NO: 171, or
(2b) a nucleotide having a base sequence set forth in SEQ ID NO: 170 and a nucleotide having a base sequence set forth in SEQ ID NO: 171.

A DNA that is a PCR amplification product obtained using the nucleotide as a primer and a chromosomal DNA of the genus Burkholderia (Burkholderia sp.) LEBP-3 strain as a template has preferably 1,155 or 1,156 bp. Here, as the DNA polymerase used in PCR, a known DNA polymerase in the art is used, and examples thereof include a Taq DNA polymerase, a hot start adjusted DNA polymerase, and a proofreading DNA polymerase having a 3'-5' exonuclease activity separately from a polymerase activity.

In the present specification, the "PCR amplification product" refers to a product obtained as a result of performing PCR using a specific nucleotide as a primer. Unless otherwise indicated, in the present specification, in PCR amplification, a treatment with an amount of 25 µL of a solution is performed 30 cycles under conditions of a heat treatment at 98°C for 10 seconds, annealing at 55°C for 15 seconds, and elongation at 72°C and 5 sec/kb using 1 µM of each of specific nucleotides as a forward primer and a reverse primer, and using PrimeSTAR Max DNA Polymerase (trade name, manufactured by TAKARA BIO INC.) as an enzyme.

In relation to the present invention, a host microorganism into which a target exogenous enzyme gene is introduced is not particularly limited, and may be either a prokaryote or a eukaryote. Any one of a microorganism isolated and preserved in advance, a microorganism newly isolated from nature, and a microorganism subjected to a genetic modification can be arbitrarily selected. The host microorganism belongs to, for example, a genus selected from the genus Escherichia, the genus Corynebacterium, the genus Bacillus, the genus Acinetobacter, the genus Burkholderia, the genus Pseudomonas, the genus Clostridium, the genus Saccharomyces, the genus Schizosaccharomyces, the genus Yarrowia, the genus Candida, the genus Pichia, the genus Aspergillus, the genus Klebsiella, the genus Gluconobacter, the genus Zymomonas, the genus Lactobacillus, the genus Lactococcus, the genus Streptococcus, and the genus Streptomyces. The host microorganism of the present invention preferably belongs to, for example, a genus selected from the group consisting of the genus Escherichia, the genus Corynebacterium, the genus Bacillus, the genus Acinetobacter, the genus Burkholderia, the genus Pseudomonas, the genus Clostridium, the genus Saccharomyces, the genus Schizosaccharomyces, the genus Yarrowia, the genus Candida, the genus Pichia, and the genus Aspergillus. The host microorganism is more preferably Escherichia coli.

Therefore, the recombinant microorganism according to the present invention into which an exogenous gene has been introduced into the host microorganism belongs to, for example, a genus selected from the group consisting of the genus Escherichia, the genus Corynebacterium, the genus Bacillus, the genus Acinetobacter, the genus Burkholderia, the genus Pseudomonas, the genus Clostridium, the genus Saccharomyces, the genus Schizosaccharomyces, the genus Yarrowia, the genus Candida, the genus Pichia, the genus Aspergillus, the genus Klebsiella, the genus Gluconobacter, the genus Zymomonas, the genus Lactobacillus, the genus Lactococcus, the genus Streptococcus, and the genus Streptomyces, and preferably belongs to a genus selected from the group consisting of the genus Escherichia, the genus Corynebacterium, the genus Bacillus, the genus Acinetobacter, the genus Burkholderia, the genus Pseudomonas, the genus Clostridium, the genus Saccharomyces, the genus Schizosaccharomyces, the genus Yarrowia, the genus Candida, the genus Pichia, and the genus Aspergillus. The recombinant microorganism according to the present invention is more preferably Escherichia coli.

The adipic acid, adipic acid derivative, hexamethylenediamine, 1,6-hexanediol, and 6-amino-1-hexanol production pathways of the genetically modified microorganism according to the present invention and the enzymes that catalyze the respective reaction stages of the pathways will be described below with reference to Fig. 16.

In the conversion of Step A in Fig. 16, for example, a succinyl-CoA:acetyl-CoA acyltransferase, or a 3-oxoadipyl-CoA thiolase is involved, and succinyl-CoA and acetyl-CoA are condensed and converted into 3-oxoadipyl-CoA. Other examples of the enzyme that can catalyze the present conversion include a β-ketothiolase. Furthermore, for example, enzymes classified into the group such as EC 2.3.1.9 (acetoacetyl-CoA thiolase), EC 2.3.1.16 (3-ketoacyl-CoA thiolase), or EC 2.3.1.174 (3-oxoadipyl-CoA thiolase) can also be exemplified as enzymes that can have an activity for the present conversion. The enzyme used in the present invention is not limited as long as it has an activity for the present conversion, and in one embodiment, Escherichia coli-derived PaaJ consisting of an amino acid sequence set forth in SEQ ID NO: 172 is used. In a preferred embodiment, the enzyme is a succinyl-CoA:acetyl-CoA acyltransferase encoded by a DNA sequence (1,203 bp) that is a PCR amplification product obtained using a nucleotide set forth in SEQ ID NO: 164 and a nucleotide set forth in SEQ ID NO: 165 as primers and a chromosomal DNA of the genus Burkholderia (Burkholderia sp.) LEBP-3 strain as a template.

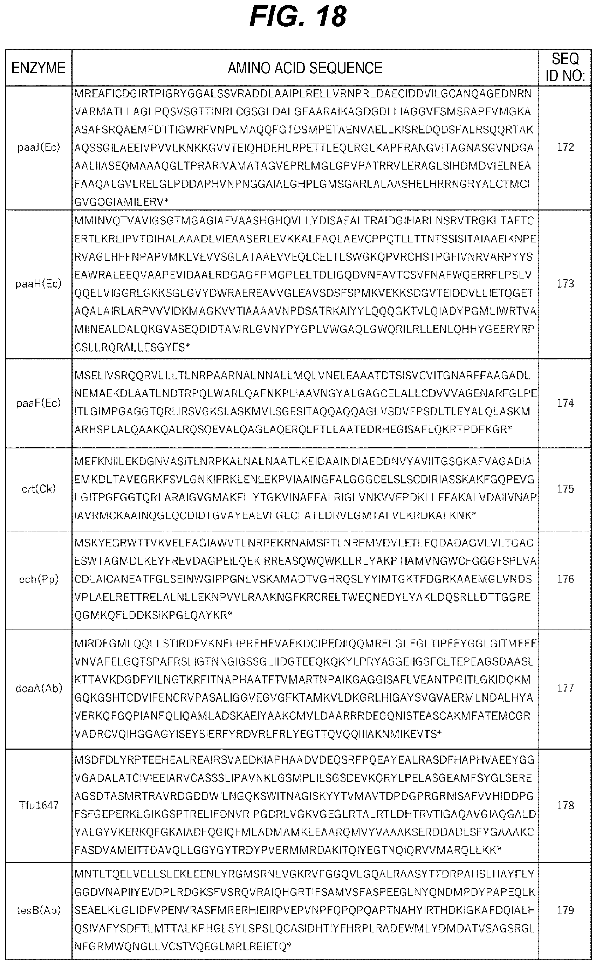

In the conversion of Step B in Fig. 16, the 3-hydroxyadipyl-CoA dehydrogenase is involved and 3-oxoadipyl-CoA is converted into 3-hydroxyadipyl-CoA. Examples of the other enzymes that can catalyze the present conversion include oxidoreductases classified into the group of EC 1.1.1. Specifically, for example, enzymes classified into the group such as EC 1.1.1.35 (3-hydroxyacyl-CoA dehydrogenase), EC 1.1.1.36 (acetoacetyl-CoA dehydrogenase), EC 1.1.1.157 (3-hydroxybutanoyl-CoA dehydrogenase), EC 1.1.1.211 (long chain 3-hydroxyacyl-CoA dehydrogenase), or EC 1.1.1.259 (3-hydroxypimeloyl-CoA dehydrogenase) can be exemplified as enzymes that can have an activity for the present conversion. The enzyme used in the present invention is not limited as long as it has an activity for the present conversion, and for example, Escherichia coli-derived PaaH consisting of an amino acid sequence set forth in SEQ ID NO: 173 is used (Fig. 18). In a preferred embodiment, the enzyme is a 3-hydroxyadipyl-CoA dehydrogenase encoded by a DNA sequence (1,521 bp) of a PCR amplification product obtained using a nucleotide set forth in SEQ ID NO: 166 and a nucleotide set forth in SEQ ID NO: 167 as primers and a chromosomal DNA of the genus Burkholderia (Burkholderia sp.) LEBP-3 strain as a template.

In the conversion of Step C in Fig. 16, for example, the 3-hydroxyadipyl-CoA dehydratase is involved and 3-hydroxyadipyl-CoA is converted into 2,3-dehydroadipyl-CoA. Examples of the other enzymes that can catalyze the present conversion include hydrolyases classified into the group of EC 4.2.1. Specifically, for example, enzymes classified into the group such as EC 4.2.1.17 (enoyl-CoA hydratase), EC 4.2.1.55 (3-hydroxybutanoyl-CoA dehydratase), or EC 4.2.1.74 (long chain enoyl-CoA hydratase) can be exemplified as enzymes that can have an activity for the present conversion. In a preferred embodiment, the enzyme is the 3-hydroxyadipyl-CoA dehydratase described above.

In the conversion of Step D in Fig. 16, for example, the 2,3-dehydroadipyl-CoA reductase is involved and 2,3-dehydroadipyl-CoA is converted into adipyl-CoA. Examples of the other enzymes that can catalyze the present conversion include oxidoreductases classified into the group of EC 1.3.1. Specifically, for example, enzymes classified into the group such as EC 1.3.1.8 (acyl-CoA dehydrogenase (NADP⁺)), EC 1.3.1.9 (enoyl-ACP reductase (NADH)), EC 1.3.1.38 (trans-2-enoyl-CoA reductase (NADP⁺)), EC 1.3.1.44 (trans-2-enoyl-CoA reductase (NAD⁺)), EC 1.3.1.86 (crotonyl-CoA reductase), EC 1.3.1.93 (long chain acyl-CoA reductase), or EC 1.3.1.104 (enoyl-ACP reductase (NADPH)) can be exemplified as enzymes that can have an activity for the present conversion. In a preferred embodiment, the enzyme is the 2,3-dehydroadipyl-CoA reductase described above.

In the conversion of Step E in Fig. 16, adipyl-CoA is converted into adipic acid. Examples of the enzyme that can catalyze the present conversion include thioester hydratases classified into the group of EC 3.1.2. For example, enzymes classified into the group such as EC 3.1.2.1 (acetyl-CoA hydratase) or EC 3.1.2.20 (acyl-CoA hydratase) can be exemplified as enzymes that can have an activity for the present conversion. In a preferred embodiment, the enzyme is not particularly limited as long as it has an activity for the present conversion, and Acinetobacter ADP1 strain-derived tesB(Ab) set forth in SEQ ID NO: 179 is used. Depending on the substrate specificity of the enzyme used, CoAs of 3-oxoadipyl-CoA, 3-hydroxyadipyl-CoA, and 2,3-dehydroadipyl-CoA are eliminated to produce 3-oxoadipic acid, 3-hydroxyadipic acid, and 2,3-dehydroadipic acid, respectively.

In addition, examples of another enzyme that can catalyze the conversion of Step E in Fig. 16 include CoA-transferases classified into the group of EC 2.8.3. For example, enzymes classified into the group of EC 2.8.3.5 (3-oxoacid CoA-transferase), EC 2.8.3.6 (3-oxoadipate CoA-transferase), or EC 2.8.3.18 (succinyl-CoA:acetate CoA-transferase) can be exemplified as enzymes that can have an activity for the present conversion.

Furthermore, examples of conversion of another enzyme that can catalyze the reaction of Step E in Fig. 16 include a pathway that transfers the adipyl group of adipyl-CoA to a phosphate to form an adipyl phosphate by phosphate acyltransferases classified into the group of EC 2.3.1 and then undergoes dephosphorylation by phosphotransferases classified into group EC 2.7.2. For example, enzymes classified into the group of EC 2.3.1.8 (phosphate acetyltransferase) or EC 2.3.1.19 (butyryl phosphate transferase) as an acyltransferase and the group of EC 2.7.2.1 (acetate kinase) or EC 2.7.2.7 (butanoate kinase) as phosphotransferases can be exemplified as enzymes that can have an activity for the present conversion.

In Step T in Fig. 16, 3-oxoadipic acid is converted into levulinic acid. The present conversion is catalyzed by an enzyme (3-oxoadipate decarboxylase) or occurs spontaneously.

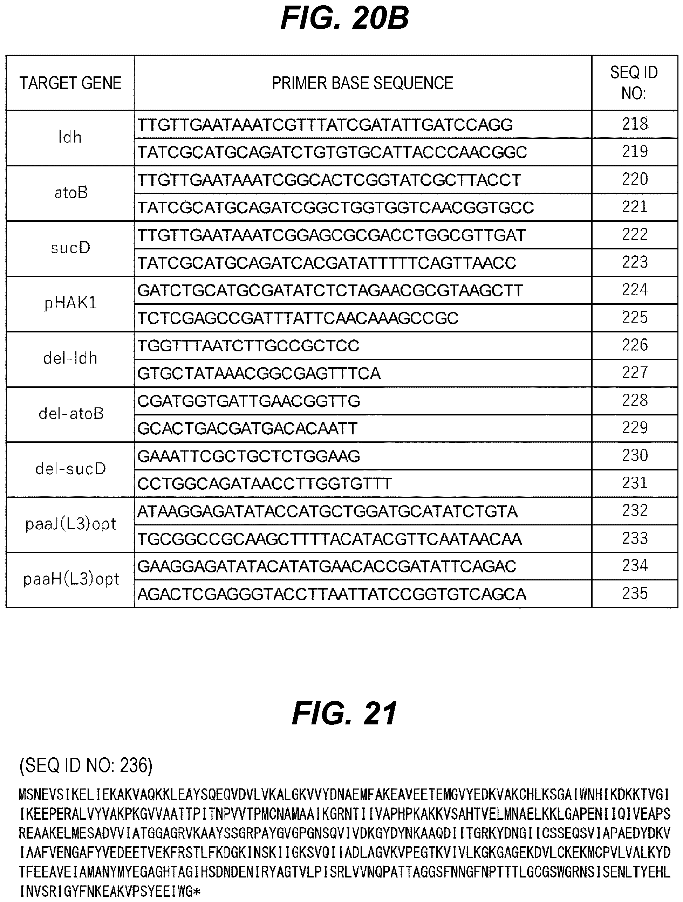

In the conversion of Step F in Fig. 16, adipyl-CoA is converted into an adipate semialdehyde. Examples of the enzyme that can catalyze the present conversion include enzymes classified into the group of EC 1.2.1. For example, enzymes classified into the group of EC 1.2.1.10 (acetaldehyde dehydrogenase (acetylation)), EC 1.2.1.17 (glyoxylate dehydrogenase (acylation)), EC 1.2.1.42 (hexadecanal dehydrogenase (acylation)), EC 1.2.1.44 (cinnamoyl-CoA reductase (acylation)), EC 1.2.1.75 (malonyl-CoA reductase (malonic semialdehyde formation)), or EC 1.2.1.76 (succinic semialdehyde dehydrogenase (acylation)) can be exemplified as enzymes that can have an activity for the present conversion because these enzymes catalyze a conversion reaction in which CoA is removed and an aldehyde is produced similarly to the present conversion. The enzyme used in the present invention is not limited as long as it has an activity for the present conversion, and for example, Clostridium kluyveri-derived sucD consisting of an amino acid sequence set forth in SEQ ID NO: 236 is used (Fig. 21).

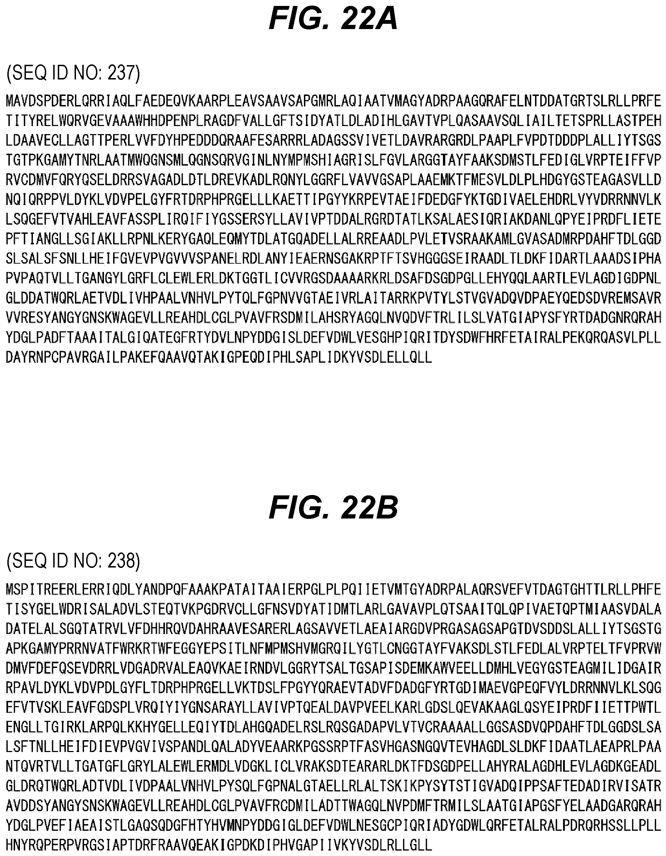

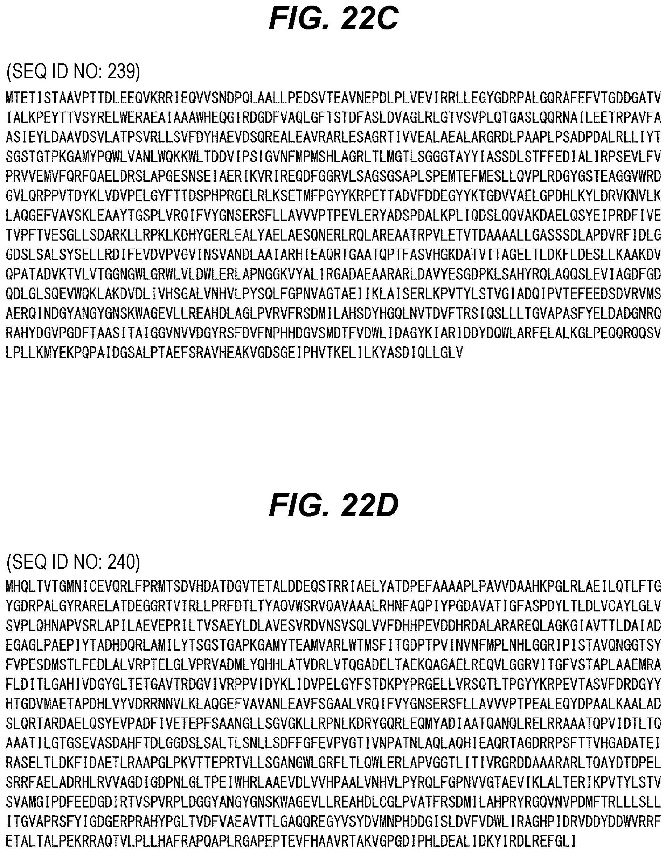

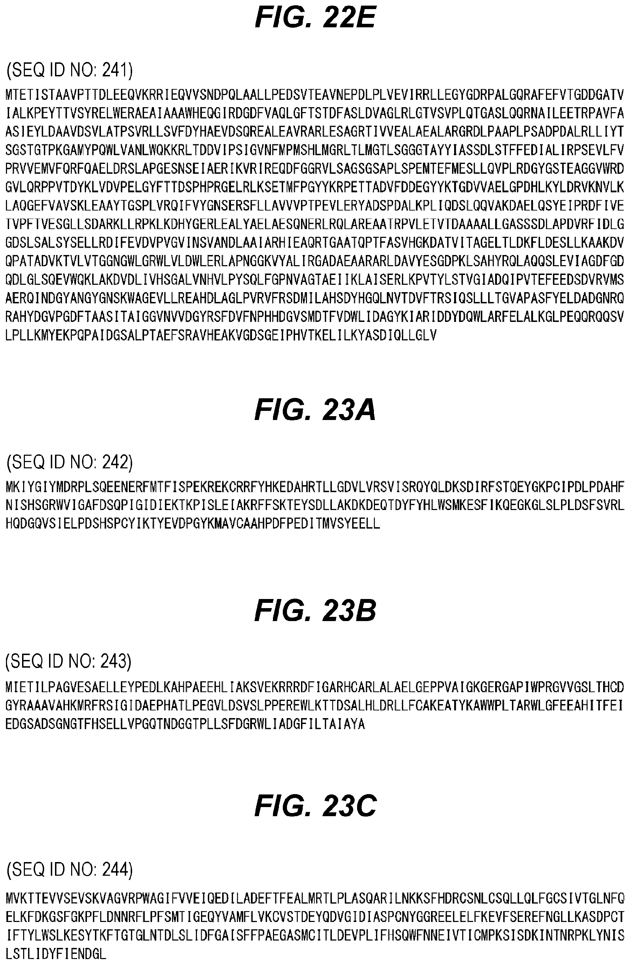

In the conversions of Steps G, J, and Q in Fig. 16, a carboxyl group is converted into an aldehyde. Examples of the enzyme that can catalyze the present conversion include a carboxylic acid reductase (CAR). For example, enzymes classified into the group of EC 1.2.1.30 (carboxylic acid reductase (NADP⁺)), EC 1.2.1.31 (L-aminoadipate semialdehyde dehydrogenase), EC 1.2.1.95 (L-2-aminoadipate reductase), or EC 1.2.99.6 (carboxylic acid reductase) can be exemplified as enzymes that can have an activity for the present conversion because these enzymes catalyze a conversion reaction in which an aldehyde is generated from a carboxylic acid similarly to the present conversion. Typical examples of the biological species from which the enzyme is derived include, but are not limited to, Nocardia iowensis, Nocardia asteroides, Nocardia brasiliensis, Nocardia farcinica, Segniliparus rugosus, Segniliparus rotundus, Tsukamurella paurometabola, Mycobacterium marinum, Mycobacterium neoaurum, Mycobacterium abscessus, Mycobacterium avium, Mycobacterium chelonae, Mycobacterium immunogenum, Mycobacterium smegmatis, Serpula lacrymans, Heterobasidion annosum, Coprinopsis cinerea, Aspergillus flavus, Aspergillus terreus, Neurospora crassa, and Saccharomyces cerevisiae. The enzyme that can be used in the present invention is not limited as long as it has an activity for the present conversion, and for example, at least one of enzymes consisting of an amino acid sequence set forth in any of SEQ ID NOs: 237 to 241 (Figs. 22A to 22E) may be used, at least one of Mycobacterium abscessus-derived enzyme MaCar consisting of an amino acid sequence set forth in SEQ ID NO: 239 and MaCar(m) that is a variant of MaCar consisting of an amino acid sequence set forth in SEQ ID NO: 241 is preferably used, and MaCar(m) consisting of an amino acid sequence set forth in SEQ ID NO: 241 is more preferably used.

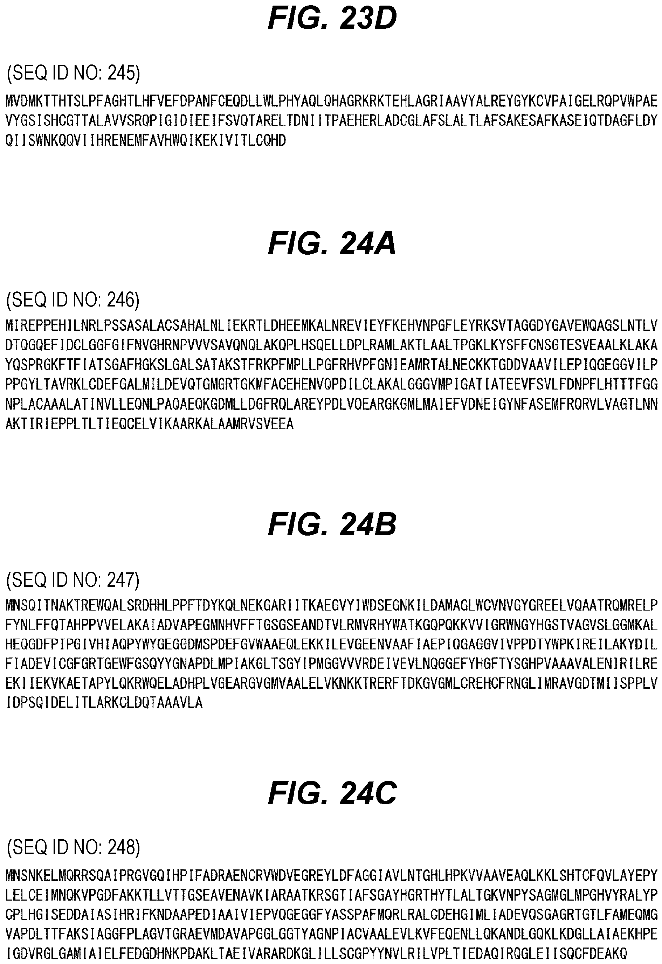

In addition, the carboxylic acid reductase can be converted into an active holoenzyme by phosphopantetinylation (Venkitasubramanian et al., Journal of Biological Chemistry, Vol. 282, No. 1, 478-485 (2007)). The phosphopantetinylation is catalyzed by a phosphopantetheinyl transferase (PT). Examples of the enzyme that can catalyze the present reaction include enzymes classified into EC 2.7.8.7. Therefore, the microorganism of the present invention may be further modified to increase an activity of the phosphopantetheinyl transferase. Examples of a method of increasing the activity of the phosphopantetheinyl transferase include, but are not limited to, a method of introducing an exogenous phosphopantetheinyl transferase and a method of enhancing expression of an endogenous phosphopantetheinyl transferase. The enzyme that can be used in the present invention is not particularly limited as long as it has a pheosphopantetheinyl group transfer activity, and typical examples thereof include EntD of Escherichia coli, Sfp of Bacillus subtilis, Npt of Nocardia iowensis (Venkitasubramanian et al., Journal of Biological Chemistry, Vol. 282, No.1, 478-485 (2007)), and Lys5 of Saccharomyces cerevisiae (Ehmann et al., Biochemistry 38.19 (1999): 6171-6177). The enzyme used in the present invention is not limited as long as it has an activity for the present conversion, and for example, at least one of enzymes consisting of amino acid sequences set forth in SEQ ID NOs: 242 to 245 may be used (Figs. 23A to 23D), and Npt of Nocardia iowensis derived from Nocardia iowensis consisting of an amino acid sequence set forth in SEQ ID NO: 243 is preferably used.

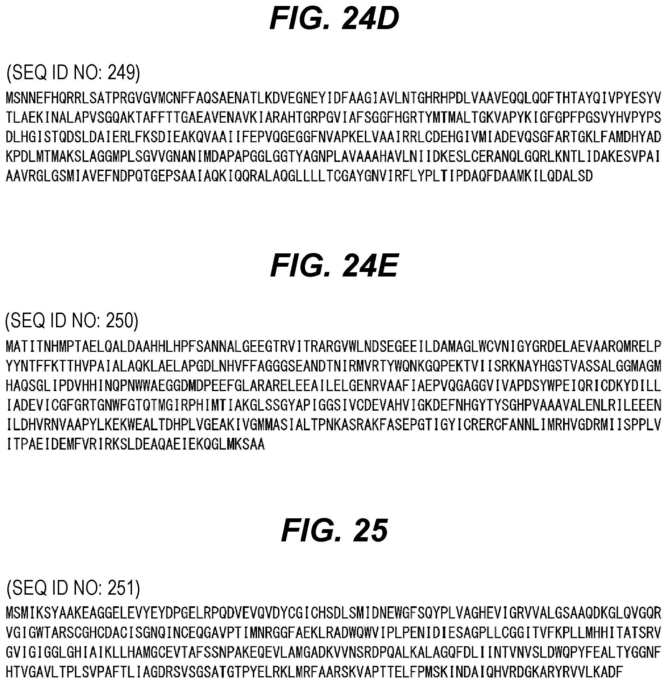

The conversions of Steps M, N, and S in Fig. 16 are transamination reactions. Examples of the enzyme that can catalyze the conversion include a transaminase (aminotransferase) classified in the group of EC 2.6.1. For example, enzymes classified into the group of EC 2.6.1.19 (4-aminobutanoate-2-oxoglutarate transaminase), EC 2.6.1.29 (diamine transaminase), or EC 2.6.1.48 (5-aminovalerate transaminase) can be exemplified as enzymes that can have an activity for the present conversion. The enzyme that can be used in the present invention is not particularly limited as long as it has the conversion activity of each step, for example, YgjG that is a putrescine aminotransferase of Escherichia coli reported to transaminate cadaverine and spermidine (Samsonova., et al., BMC microbiology 3.1 (2003): 2), SpuC that is a putrescine aminotransferase of the genus Pseudomonas (Lu et al., Journal of bacteriology 184.14 (2002): 3765-3773, Galman et al., Green Chemistry 19.2 (2017): 361-366), or GABA aminotransferase GabT or PuuE of Escherichia coli may also be used. Furthermore, it is reported that an ω-transaminase derived from a biological species such as Ruegeria pomeroyi, Chromobacterium violaceum, Arthrobacter citreus, Sphaerobacter thermophilus, Aspergillus fischeri, Vibrio fluvialis, Agrobacterium tumefaciens, or Mesorhizobium loti also has a transamination activity to a diamine compound such as 1,8-diaminooctane or 1,10-diaminodecane, and these enzymes may be used in the present invention (Sung et al., Green Chemistry 20.20 (2018): 4591-4595, Sattler et al., Angewandte Chemie 124.36 (2012): 9290-9293). The enzyme used in the present invention is not limited as long as it has an activity for the present conversion, and for example, at least one of enzymes consisting of amino acid sequences set forth in SEQ ID NOs: 246 to 250 may be used (Figs. 24A to 24E). Examples of a typical amino group donor include, but are not limited to, L-glutamic acid, L-alanine, and glycine.

In the conversions of Steps H, L, and R in Fig. 16, aldehydes are converted into alcohols. Examples of the enzyme that can catalyze the present conversion include oxidoreductases classified into the group of EC 1.1.1. For example, enzymes classified in the group of EC 1.1.1.1 (alcohol dehydrogenase), EC 1.1.1.2 (alcohol dehydrogenase (NADP⁺)), or EC 1.1.1.71 (alcohol dehydrogenase [NAD(P)⁺]) can be exemplified as enzymes that can have an activity for the present conversion because these enzymes catalyze a reaction in which an aldehyde is converted into an alcohol similarly to the present conversion. The enzyme that can be used in the present invention is not limited as long as it has an activity for the present conversion, and for example, Escherichia coli-derived Ahr set forth in SEQ ID NO: 251 is used (Fig. 25).

In the reactions of Steps I and O in Fig. 16, CoA is added to a carboxyl group. Examples of the enzyme that can catalyze the present conversion include CoA transferases classified into the group of EC 2.8.3 and acid-thiol ligases classified into the group of EC 6.2.1. The enzyme used in the present invention is not limited as long as it has an activity for the present conversion.

The gene encoding the enzyme that can be used in the present invention may be derived from an organism other than the exemplified organism or artificially synthesized, and may be any gene that can express a substantial enzyme activity in a host microorganism cell.

In the genetically modified microorganism according to the present invention, it is preferable that an exogenous gene encoding at least one enzyme selected from the group consisting of a succinyl-CoA:acetyl-CoA acyltransferase and a 3-hydroxyadipyl-CoA dehydrogenase is introduced, in addition to the exogenous gene encoding a 3-hydroxyadipyl-CoA dehydratase and/or a 2,3-dehydroadipyl-CoA reductase described above.

That is, the recombinant microorganism according to the present invention preferably further contains at least one of an exogenous gene encoding a succinyl-CoA:acetyl-CoA acyltransferase and an exogenous gene encoding a 3-hydroxyadipyl-CoA dehydrogenase.

Other exogenous genes may be introduced into the recombinant microorganism according to the present invention, in addition to the exogenous genes described above, that is, the 3-hydroxyadipyl-CoA dehydratase, the 2,3-dehydroadipyl-CoA reductase, the succinyl-CoA:acetyl-CoA acyltransferase, and the 3-hydroxyadipyl-CoA dehydrogenase.

The recombinant microorganism according to the present invention may be a recombinant microorganism in which any gene has been disrupted as appropriate in a host microorganism. The target gene may be disrupted by a method known in the art.

In addition, the enzyme gene that can be used for the present invention may have all naturally occurring mutations or artificially introduced mutations and modifications as long as it can express a substantial enzyme activity in the host microorganism cell. For example, it is known that extra codons are present in various codons encoding a specific amino acid. Therefore, in the present invention, alternative codons that are to be finally translated into the same amino acid may also be used. That is, since a genetic code degenerates, a plurality of codons can be used to encode a specific amino acid, such that the amino acid sequence can be encoded by an arbitrary set of similar DNA oligonucleotides. The only member of the set is identical to the gene sequence of the natural enzyme; however, even mismatched DNA oligonucleotides can hybridize to natural sequences under an appropriate stringent condition (for example, hybridize at 3xSSC at 68°C and wash at 2xSSC, 0.1%SDS at 68°C), DNA encoding a natural sequence can by identified and isolated, and such a gene can also be used in the present invention. In particular, since most organisms are known to preferentially use a subset of a specific codon (optimal codon) (Gene, Vol. 105, pp. 61-72, 1991, and the like), performing of "codon optimization" depending on a host microorganism can also be useful in the present invention.

Therefore, the genetically modified microorganism according to the present invention can have a base sequence having a sequence identity of, for example, 65% or higher, 70% or higher, 75% or higher, 80% or higher, 85% or higher, 90% or higher, 95% or higher, 97% or higher, 98% or higher, or 99% or higher with the base sequence of the enzyme gene under a condition in which a substantial enzymatic activity can be expressed. Alternatively, the genetically modified microorganism according to the present invention can have a base sequence having a sequence identity of, for example, 65% or higher, 70% or higher, 75% or higher, 80% or higher, 85% or higher, 90% or higher, 95% or higher, 97% or higher, 98% or higher, or 99% or higher with a base sequence encoding the amino acid sequence of the enzyme described above.

In the present invention, when the biosynthetic enzyme gene is introduced into a host microorganism cell as an "expression cassette", a more stable and high level of enzymatic activity can be obtained. In the present specification, the "expression cassette" refers to a nucleotide having a nucleic acid sequence that is functionally linked to a nucleic acid to be expressed or a gene to be expressed and regulates transcription and translation. Typically, the expression cassette of the present invention contains a promoter sequence on the 5' upstream from the coding sequence, a terminator sequence on the 3' downstream, and an optionally additional normal regulatory element in a functionally linked state, and in such a case, a nucleic acid to be expressed or a gene to be expressed is introduced into a host microorganism.

In the present specification, the promoter is defined as a DNA sequence that allows RNA polymerase to bind to DNA to initiate RNA synthesis, regardless of whether the promoter is a constitutive expression promoter or an inductive expression promoter. A strong promoter is a promoter that initiates mRNA synthesis at a high frequency, and is also suitably used in the present invention. In Escherichia coli, a lac system, a trp system, a tac or trc system, or a major operator and promoter region of a λ-phage, a regulatory region for fd coat protein, a promoter for a glycolytic enzyme (for example, 3-phosphoglycerate kinase or glyceraldehyde-3-phosphate dehydrogenase), glutamate decarboxylase A, or serine hydroxymethyltransferase, a promoter region of RNA polymerase derived from T7 phage, and the like can be used. In Corynebacterium glutamicum, a high-level constitutive expression (HCE) promoter, a cspB promoter, a sodA promoter, an elongation factor (EF-Tu) promoter, and the like can be used.

As the terminator, a T7 terminator, an rrnBT1T2 terminator, a lac terminator, and the like can be used.

In addition to the promoter and terminator sequences, other examples of regulatory elements include a selection marker, an amplification signal, and a replication point. A preferred regulatory element is described in, for example, "Gene Expression Technology: Methods in Enzymology 185" Academic Press (1990)".

The host microorganism used in the present invention may be a microorganism obtained by disrupting a gene encoding an enzyme originally possessed by the microorganism. Examples of the gene encoding the enzyme include an ldh gene encoding lactate dehydrogenase, an atoB gene encoding an acetyl-CoA acetyltransferase, and a sucD gene encoding a succinyl-CoA synthetase α-subunit. As described above, by using the host microorganism in which a part of the gene of the enzyme originally possessed by the host microorganism is disrupted, contamination of impurities can be avoided, and progress of an unintended reaction can be suppressed. Specifically, the ldh gene encoding lactate dehydrogenase is disrupted, such that the production of lactic acid as an impurity can be reduced. The atoB gene encoding an acetyl-CoA acetyltransferase and the sucD gene encoding a succinyl-CoA synthetase α-subunit are disrupted, such that the yield of adipic acid can be improved.

The expression cassette described above is incorporated into a vector consisting of, for example, a plasmid, a phage, a transposon, an IS element, a fosmid, a cosmid, or a linear or cyclic DNA and is inserted into a host microorganism. A plasmid and a phage are preferred. The vector may be self-replicated in a host microorganism or may be replicated by a chromosome. Examples of a preferred plasmid include pLG338, pACYC184, pBR322, pUC18, pUC19, pKC30, pRep4, pHS1, pKK223-3, pDHE19.2, pHS2, pPLc236, pMBL24, pLG200, pUR290, pIN-III113-B1, Agt11, or pBdCI of Escherichia coli; pUB110, pC194, or pBD214 of a bacillus; and pSA77 or pAJ667 of the genus Corynebacterium. Examples of a bacillus such as the genus Bacillus include pUB110, pC194, and pBD214. Other usable plasmids and the like are described in "Gene Cloning and DNA nalysis 6th edition", Wiley-Blackwell (2016). The expression cassette can be introduced into the vector by a conventional method of including cutting with an appropriate restriction enzyme, cloning, and ligation. Each expression cassette may be located on one vector or on two or more vectors.

After the vector having the expression cassette of the present invention is constructed as described above, a conventional method can be used as a method that can be applied when the vector is introduced into a host microorganism. Examples of the method include, but are not limited to, a calcium chloride method, an electroporation method, a conjugation transfer method, and a protoplast fusion method, and a method suitable for a host microorganism can be selected.

Another embodiment of the present invention relates to a production method of a target compound using the recombinant microorganism. More specifically, the present invention relates to a method for producing a target compound, the method including a culture step of culturing the recombinant microorganism.

Here, the target compound is selected from the group consisting of adipic acid, an adipic acid derivative, hexamethylenediamine, 1,6-hexanediol, and 6-amino-1-hexanol. Examples of the adipic acid derivative include 3-oxoadipic acid (3-OA), levulinic acid (LEV), 3-hydroxyadipic acid (3HA), and 2,3-dehydroadipic acid (23DA) as described above.

In one embodiment, the recombinant microorganism according to the present invention has an adipic acid production pathway. In this case, the present invention relates to a method for producing adipic acid, the method including a culture step of culturing the recombinant microorganism.

In one embodiment, the recombinant microorganism according to the present invention has a hexamethylenediamine production pathway. In this case, the present invention relates to a method for producing hexamethylenediamine, the method including a culture step of culturing the recombinant microorganism.

In one embodiment, the recombinant microorganism according to the present invention has a 1,6-hexanediol production pathway. In this case, the present invention relates to a method for producing 1,6-hexanediol, the method including a culture step of culturing the recombinant microorganism.

In one embodiment, the recombinant microorganism according to the present invention has a 6-amino-1-hexanol production pathway. In this case, the present invention relates to a method for producing 6-amino-1-hexanol, the method including a culture step of culturing the recombinant microorganism.

In the culture step, a culture containing microbial cells is obtained by culturing the recombinant microorganism in a medium containing a carbon source and a nitrogen source. The genetically modified microorganism of the present invention is cultured under conditions suitable for the adipic acid production and the growth and maintenance of the microorganism, and suitable medium composition, culture time, and culture conditions can be easily set by those skilled in the art.

Examples of the carbon source include D-glucose, sucrose, lactose, fructose, maltose, oligosaccharides, polysaccharides, starch, cellulose, rice bran, blackstrap molasses, fats and oils (for example, soybean oil, sunflower oil, peanut oil, palm oil, and the like), fatty acids (for example, palmitic acid, linoleic acid, oleic acid, linolenic acid, and the like), alcohols (for example, glycerol, ethanol, and the like), organic acids (for example, acetic acid, lactic acid, succinic acid, and the like), corn decomposition liquids, cellulose decomposition liquids, carbon dioxide, and carbon monoxide. Preferably, the carbon source is D-glucose, sucrose, or glycerol. These carbon sources can be used alone or as a mixture.

The adipic acid produced using a biomass-derived raw material can be clearly distinguished from a synthetic raw material derived from, for example, petroleum, natural gas, or coal, by measurement of a biomass carbon content based on Carbon-14 (radiocarbon) analysis defined in ISO 16620-2 or ASTM D6866.

Examples of the nitrogen source include nitrogen-containing organic compounds (for example, peptone, casamino acid, tryptone, a yeast extract, a meat extract, a malt extract, a corn steep liquor, soy flour, an amino acid, urea, and the like) and inorganic compounds (for example, an aqueous ammonia solution, ammonium sulfate, ammonium chloride, ammonium phosphate, ammonium carbonate, sodium nitrate, ammonium nitrate, and the like). These nitrogen sources can be used alone or as a mixture.

In addition, the medium may contain a corresponding antibiotic in a case where a recombinant microorganism expresses a useful additional trait, for example, in a case where a recombinant microorganism contains a marker resistant to an antibiotic. Therefore, a risk of contamination by various bacteria during culture is reduced. Examples of the antibiotic include, but are not limited to, a β-lactam antibiotic such as ampicillin, an aminoglycoside antibiotic such as kanamycin, a macrolide antibiotic such as erythromycin, a tetracycline antibiotic, and chloramphenicol.

The culture may be batch or continuous. In addition, in any case, an additional carbon source or the like may be supplied at an appropriate time point of culture. Furthermore, the culture may be performed while conditions such as a suitable temperature, oxygen concentration, and pH are controlled. A suitable culture temperature of a general transformant derived from a microbial host cell is usually 15°C to 45°C and preferably 25°C to 37°C. In a case where the host microorganism is aerobic, shaking (flask culture or the like), agitation/aeration (jar fermenter culture or the like) may be performed to ensure a suitable oxygen concentration during fermentation. These culture conditions can be easily set by those skilled in the art.

The production method of a target compound according to the present invention may include a separation and purification step of separating and purifying a produced compound from a culture. In the step, as any technique, for example, techniques such as centrifugation, membrane filtration, membrane separation, crystallization, extraction, distillation, adsorption, phase separation, and various types of chromatography can be used, but are not limited thereto. The separation and purification step may include one type of step, or may include a combination of a plurality of steps.

Those skilled in the art given the above description can sufficiently implement the present invention. Hereinafter, examples are given for the purpose of further description, and the present invention is not limited to the examples.

### [Examples]

### <1> Invention A

Hereinafter, the present invention A will be described based on examples, and the present invention A is not limited to these examples.

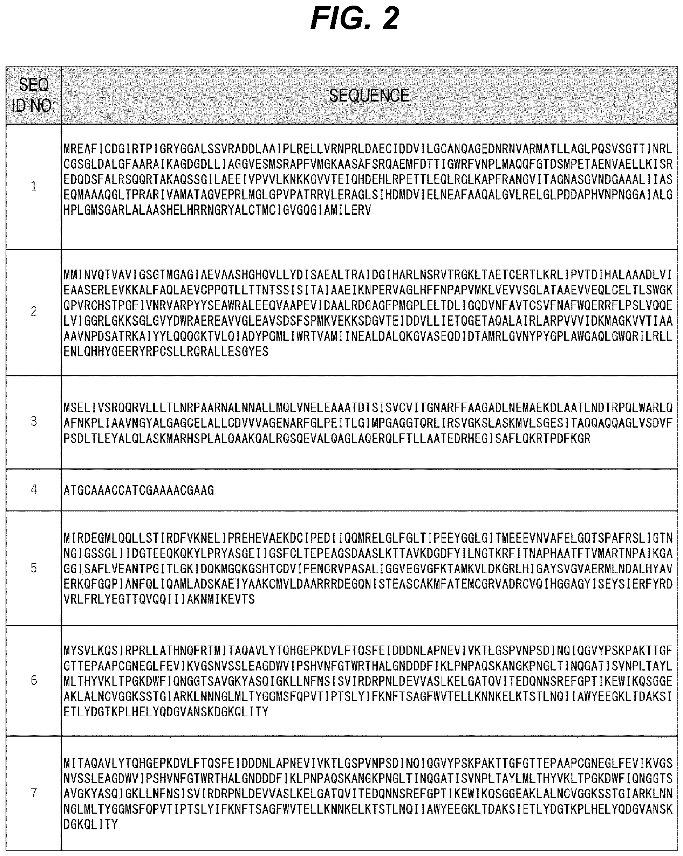

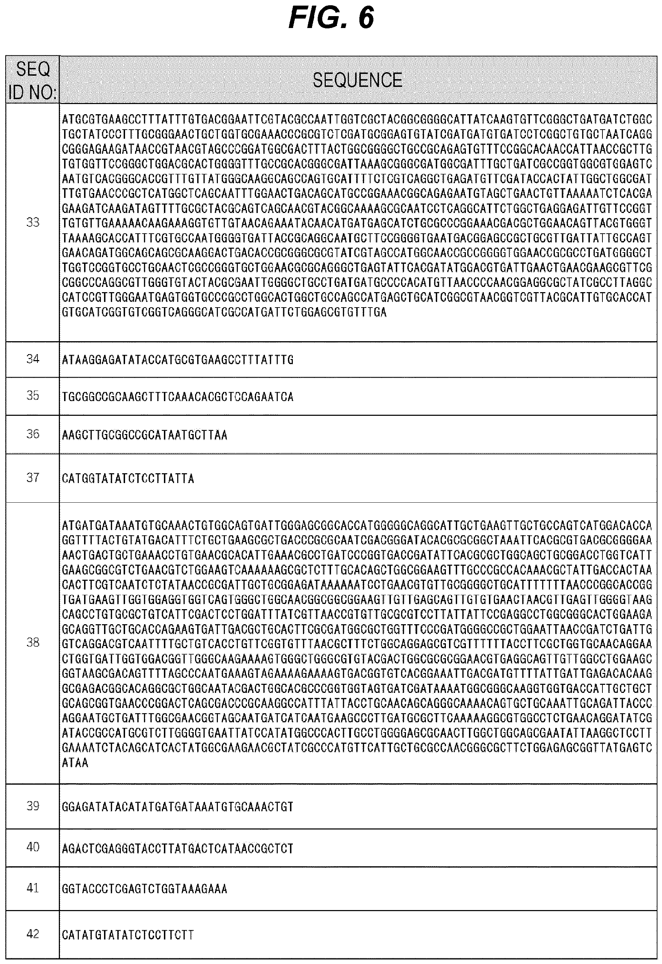

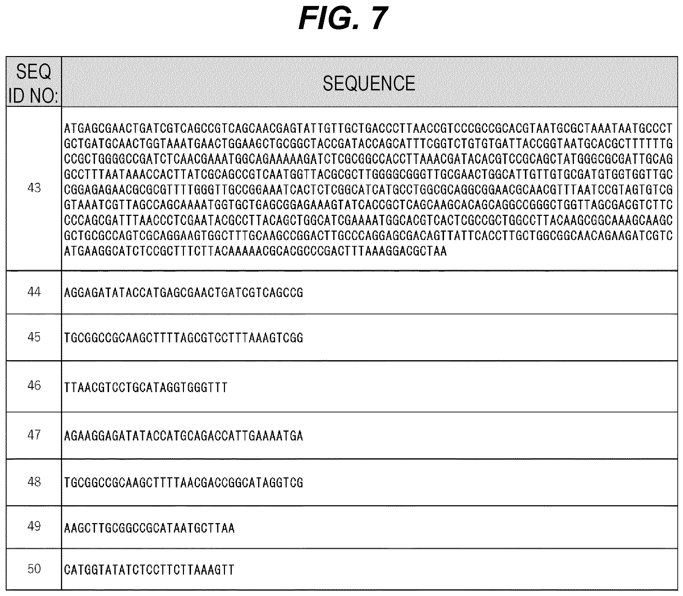

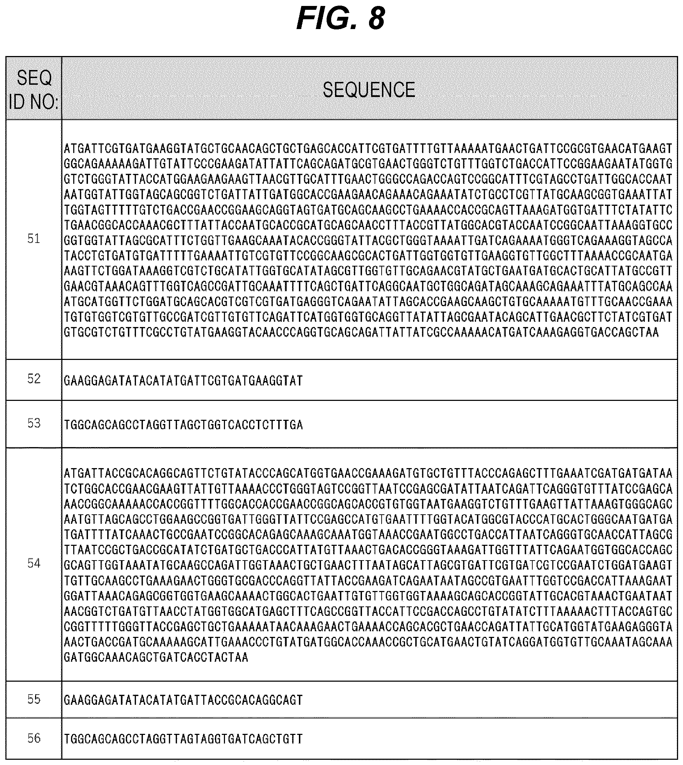

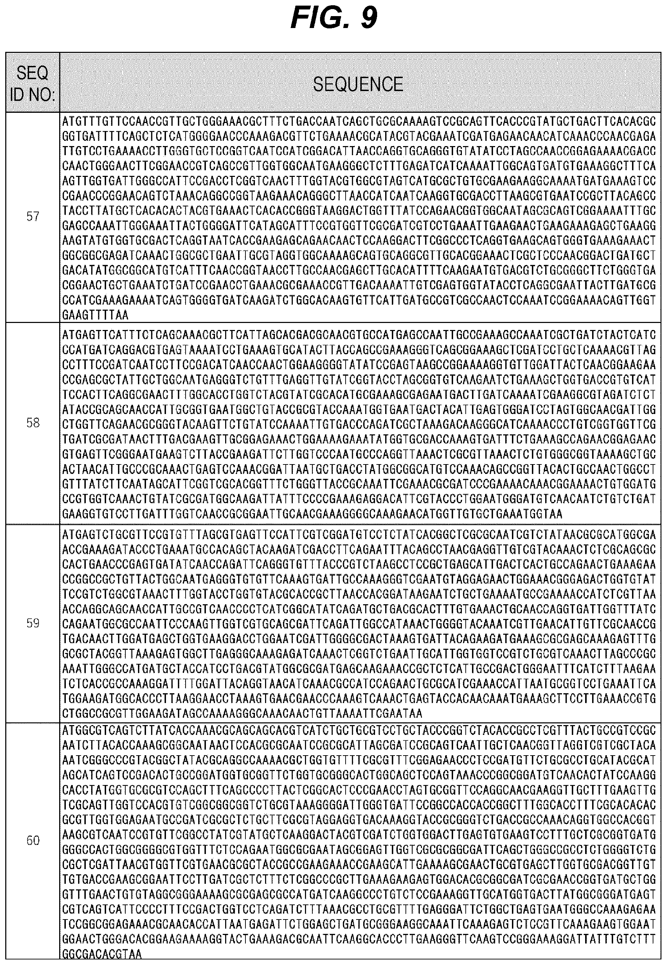

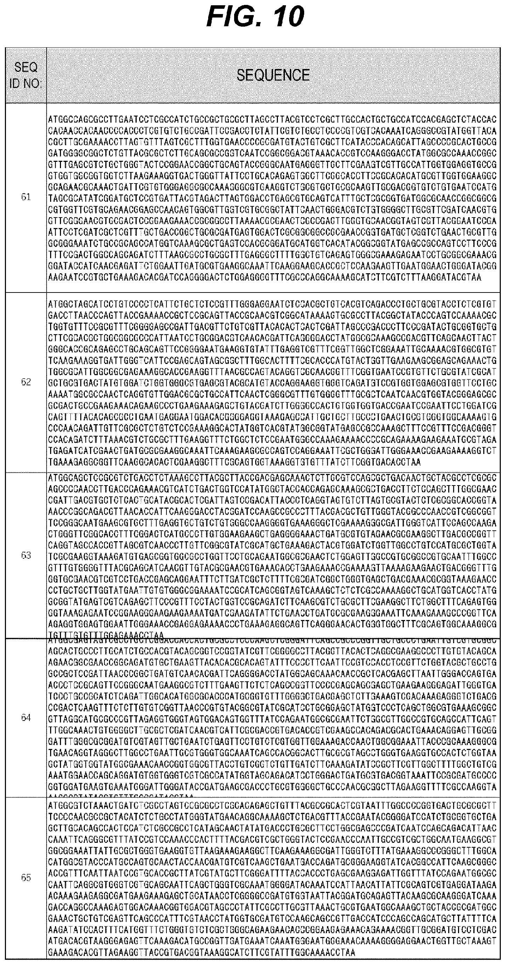

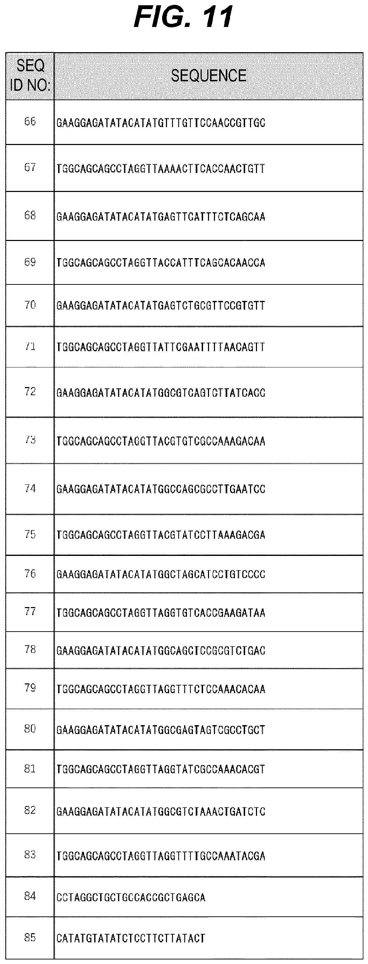

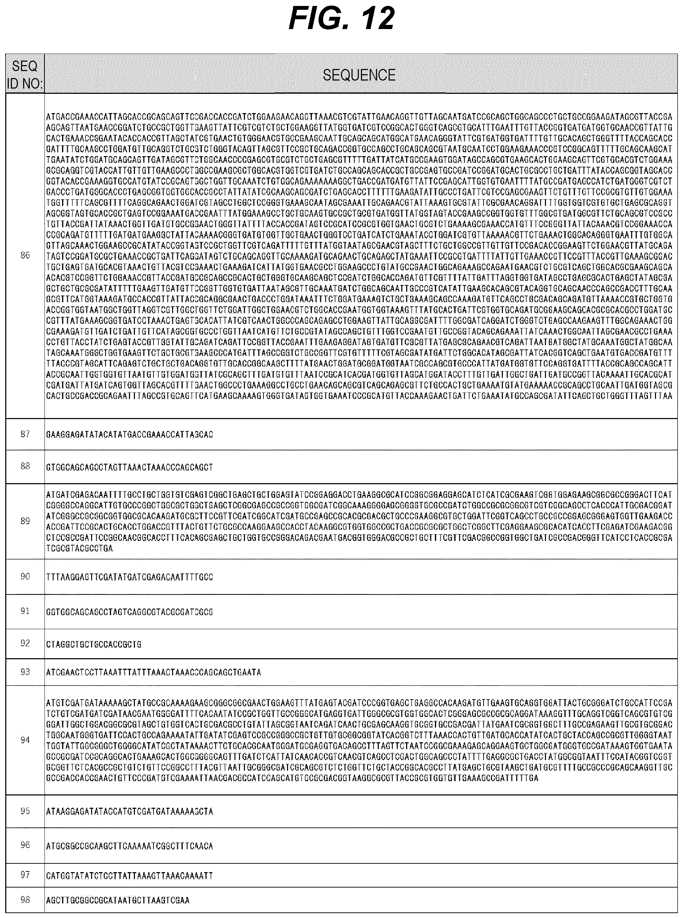

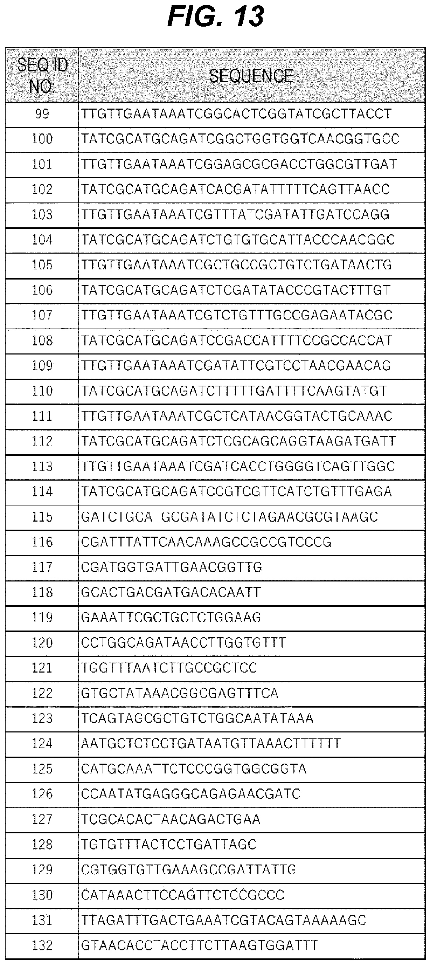

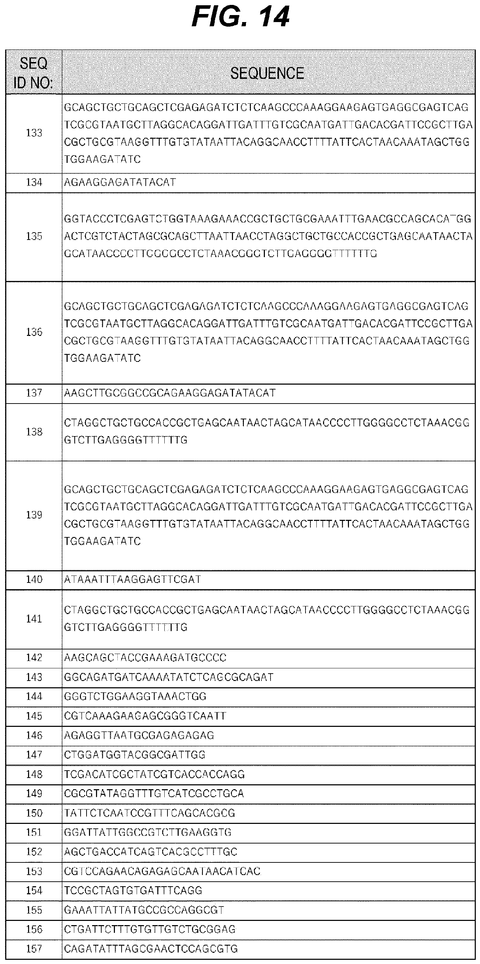

The amino acid sequences and the base sequences of the samples used in the examples are illustrated in Figs. 2 to 15.

### <Construction of Plasmid for Expressing C6 Compound Production Pathway Enzyme Gene>

The PCR fragment was amplified using PrimeSTAR Max DNA Polymerase (trade name, manufactured by TAKARA BIO INC.) or KOD FX Neo (trade name, manufactured by Toyobo Co., Ltd.), and the plasmid was prepared using an Escherichia coli JM109 strain. For the optimization of the base sequence, GeneArt GeneOptimizer (Software name, Thermo Fisher Scientific Inc.) or an artificial gene synthesis service of Eurofins Genomics LLC was used.

A polynucleotide encoding PaaJ(Ec) (SEQ ID NO: 1) of Eshcherichia coli was obtained by cloning from Eshcherichia coli W3110 strain (NBRC12713) genomic DNA. PCR was performed using oligonucleotides of SEQ ID NOs: 34 and 35 as primers to obtain a PCR product containing the coding region (SEQ ID NO: 33) of paaJ(Ec) gene. Next, PCR was performed using pRSFDuet-1 (trade name, manufactured by Merck & Co., Inc.) as a template and oligonucleotides of SEQ ID NOs: 36 and 37 as primers to obtain a pRSFDuet-1 fragment. The DNA fragment containing a paaJ(Ec) gene coding region and the pRSFDuet-1 fragment were connected using In-Fusion (registered trademark, hereinafter omitted) HD cloning kit (manufactured by Clontech Laboratories, Inc.). An Escherichia coli JM109 strain was transformed, and a plasmid was extracted from the resulting transformant. "paaJ(Ec)-pRSFDuet" was obtained as a PaaJ(Ec) expression plasmid.

A polynucleotide encoding PaaH(Ec) (SEQ ID NO: 2) of Eshcherichia coli was obtained by cloning from Eshcherichia coli W3110 strain (NBRC12713) genomic DNA. PCR was performed using oligonucleotides of SEQ ID NOs: 39 and 40 as primers to obtain a PCR product containing a coding region (SEQ ID NO: 38) of paaH(Ec) gene. Next, PCR was performed using the "paaJ(Ec)-pRSFDuet" as a template and oligonucleotides of SEQ ID NOs: 41 and 42 as primers to obtain a "paaJ(Ec)-pRSFDuet" fragment. The DNA fragment containing a coding region of paaH(Ec) gene and the "paaJ(Ec)-pRSFDuet" fragment were connected using In-Fusion HD cloning kit (trade name, manufactured by Clontech Laboratories, Inc.). An Escherichia coli JM109 strain was transformed, and a plasmid was extracted from the resulting transformant. "paaJ(Ec)-paaH(Ec)-pRSFDuet" was obtained as a PaaJ(Ec) and PaaH(Ec) co-expression plasmid.

A polynucleotide encoding PaaF(Ec) (SEQ ID NO: 3) of Eshcherichia coli was obtained by cloning from Eshcherichia coli W3110 strain (NBRC12713) genomic DNA. PCR was performed using oligonucleotides of SEQ ID NOs: 44 and 45 as primers to obtain a PCR product containing a coding region (SEQ ID NO: 43) of paaF(Ec) gene. The nucleotide set forth in SEQ ID NO: 4 and the nucleotide set forth in SEQ ID NO: 46 were used as primers, and the nucleotide encoding PaaF(L3) of the L3 strain encoded by the base sequence of the PCR amplification product obtained using the chromosomal DNA of the L3 strain as a template was obtained by optimizing the base sequence for expressing Escherichia coli using the artificial gene synthesis service of Eurofins Genomics LLC. PCR was performed using oligonucleotides of SEQ ID NOs: 47 and 48 as primers to obtain a PCR product containing the optimized sequence of the coding region of paaF(L3) gene. PCR was performed using pETDuet-1 (trade name, manufactured by Merck & Co., Inc.) as a template and oligonucleotides of SEQ ID NOs: 49 and 50 as primers to obtain a pETDuet-1 fragment. The DNA fragments containing the coding regions of paaF(Ec) and paaF(L3), respectively, and the pETDuet-1 fragment were connected using In-Fusion HD cloning kit (trade name, manufactured by Clontech Laboratories, Inc.). An Escherichia coli JM109 strain was transformed, and a plasmid was extracted from the resulting transformant. "paaF(Ec)-pETDuet" was obtained as a PaaF(Ec) expression plasmid and "paaF(L3)-pETDuet" was obtained as a PaaF(L3) expression plasmid.

The polynucleotide encoding dcaA(Ab) (SEQ ID NO: 5) of Acinetobacter baylyi was obtained by optimizing the base sequence for expressing Escherichia coli using the artificial gene synthesis service of Eurofins Genomics LLC. PCR was performed using oligonucleotides of SEQ ID NOs: 52 and 53 as primers to obtain a PCR product containing a coding region (SEQ ID NO: 51) of dcaA(Ab) gene. PCR was performed using "paaF(Ec)-pETDuet" as a template and oligonucleotides of SEQ ID NOs: 84 and 85 as primers to obtain a "paaF(Ec)-pETDuet" fragment. The DNA fragment containing a coding region of dcaA(Ab) gene and the "paaF(Ec)-pETDuet" fragment were connected using In-Fusion HD cloning kit (trade name, manufactured by Clontech Laboratories, Inc.). An Escherichia coli JM109 strain was transformed, and a plasmid was extracted from the resulting transformant. "paaF(Ec)-dcaA(Ab)-pETDuet" was obtained as a PaaF(Ec) and DcaA(Ab) co-expression plasmid.

Ter(Ct) (SEQ ID NO: 6) of Candida tropicalis was cloned as a sequence (SEQ ID NO: 7) with the N-terminal region removed by 22 residues. The polynucleotide for encoding was obtained by optimizing the base sequence for expressing Escherichia coli using the artificial gene synthesis service of Eurofins Genomics LLC. PCR was performed using oligonucleotides of SEQ ID NOs: 55 and 56 as primers to obtain a PCR product containing a coding region (SEQ ID NO: 54) of ter(Ct) gene. PCR was performed using "paaF(L3)-pETDuet" as a template and oligonucleotides of SEQ ID NOs: 84 and 85 as primers to obtain a "paaF(L3)-pETDuet" fragment. The DNA fragment containing a coding region of ter(Ct) gene and the "paaF(L3)-pETDuet" fragment were connected using In-Fusion HD cloning kit (trade name, manufactured by Clontech Laboratories, Inc.). An Escherichia coli JM109 strain was transformed, and a plasmid was extracted from the resulting transformant. "paaF(L3)-ter(Ct)-pETDuet" was obtained as a PaaF(L3) and Ter(Ct) co-expression plasmid.

Using BLAST Search (https://www.genome.jp/tools/blast/) BLASTP, a protein presumed to have a function of a 2,3-dehydroadipyl-CoA reductase was selected from the KEGG GENES database. The selected candidate enzyme proteins are shown in Table A-1.

**[Table A-1]**

| Table A-1 Candidate 2,3-dehydroadipyl-CoA reductase protein | | | |
|---|---|---|---|
| Enzyme | Amino acid sequence SEQ ID NO: | Derived species | Accession Number |
| Ter(caur) | 8 | Candida *auris* | XP_028888110 |
| Ter(kmx) | 9 | *Kluyveromyces marxianus* | XP_022677345 |
| Ter(pkz} | 10 | *Pichia kudriavzevii* | XP_029321276 |
| Ter(mtm) | 11 | *Thermothelomyces thermophilus* | XP_003660060 |
| Ter(ttt) | 12 | *Thermothielavioides terrestris* | XP_003651510 |
| Ter(cthr) | 13 | *Chaetomium thermophilum* | XP_006691841 |
| Ter(pan) | 14 | *Podospora anserina* | XP_001907468 |
| Ter(plj) | 15 | *Purpureocillium lilacinum* | XP_018182887 |
| Ter(pte) | 16 | *Pyrenophorateres* | EFQ85170 |

The polynucleotide encoding the selected 2,3-dehydroadipyl-CoA reductase candidate proteins was obtained by optimizing the base sequence for Escherichia coli expression and using the artificial gene synthesis service of Eurofins Genomics LLC, and a DNA fragment containing the coding region of each enzyme gene was obtained by PCR. The base sequence of the coding region of each enzyme gene and the sequence number of the base sequence of the primer set used for PCR are shown in Table 2. PCR was performed using "paaF(L3)-pETDuet" as a template and oligonucleotides of SEQ ID NOs: 84 and 85 as primers to obtain a "paaF(L3)-pETDuet" fragment. The DNA fragment containing a coding region of each gene and the "paaF(L3)-pETDuet" fragment were connected using In-Fusion HD cloning kit (trade name, manufactured by Clontech Laboratories, Inc.). An Escherichia coli JM109 strain was transformed, and a plasmid was extracted from the resulting transformant. Co-expression plasmids of PaaF (L3) and each 2,3-dehydroadipyl-CoA reductase candidate protein were obtained.

**[Table A-2]**

| Table A-2: Candidate 2,3-dehydroadipyl-CoA reductase gene base sequence and primer setCandidate 2,3-dehydroadipyl-CoA reductase gene base sequence and primer set | | |
|---|---|---|
| Enzyme | Coding region Base sequence SEQ ID NO: | Primer set SEQ ID NO: |
| Ter(caur) | 57 | 66,67 |
| Ter(kmx) | 58 | 68,69 |
| Ter(pkz) | 59 | 70,71 |
| Ter(mtm) | 60 | 72,73 |
| Ter(ttt) | 61 | 74,75 |
| Ter(cthr) | 62 | 76,77 |
| Ter(pan) | 63 | 78,79 |
| Ter(plj) | 64 | 80,81 |
| Ter(pie) | 65 | 82,83 |

A carboxylic acid reductase MaCar (SEQ ID NO: 20) of Mycobacterium abscessus was used as a variant MaCar(m) in which the 283rd tryptophan residue of the amino acid sequence was substituted with an arginine residue and the 303rd alanine residue was substituted with a methionine residue (SEQ ID NO: 22). The polynucleotide encoding MaCar(m) was obtained by optimizing the base sequence for expressing Escherichia coli using the artificial gene synthesis service of Eurofins Genomics LLC. PCR was performed using oligonucleotides of SEQ ID NOs: 87 and 88 as primers to obtain a PCR product containing a coding region (SEQ ID NO: 86) of Macar(m) gene.

Next, the present PCR product was inserted into the cleavage site of restriction enzymes NdeI and AvrII of pACYCDuet-1 (trade name, manufactured by Merck & Co., Inc.) using In-Fusion HD cloning kit (trade name, manufactured by Clontech Laboratories, Inc.). An Escherichia coli JM109 strain was transformed, and a plasmid was extracted from the resulting transformant. "Macar(m)-pACYCDuet" was obtained as an MaCar(m) expression plasmid.

A polynucleotide encoding Npt (SEQ ID NO: 24) of Nocardia iowensis was obtained by cloning from the genomic DNA of the Nocardia iowensis JCM18299 strain (provided by RIKEN BRC through the National BioResource Project of the Ministry of Education, Culture, Sports, Science and Technology). PCR was performed using oligonucleotides of SEQ ID NOs: 90 and 91 as primers to obtain a PCR product containing a coding region (SEQ ID NO: 89) of npt gene. Next, PCR was performed using "MaCar(m)-pACYCDuet" as a template and oligonucleotides of SEQ ID NOs: 92 and 93 as primers to obtain an "Macar(m)-pACYCDuet" fragment. The PCR products were connected to each other using In-Fusion HD cloning kit (trade name, manufactured by Clontech Laboratories, Inc.). An Escherichia coli JM109 strain was transformed, and a plasmid was extracted from the resulting transformant. "Macar(m)-npt-pACYCDuet" was obtained as an MaCar(m) and Npt co-expression plasmid.

A polynucleotide encoding Ahr (SEQ ID NO: 32) of Eshcherichia coli was obtained by cloning from Eshcherichia coli W3110 strain (NBRC12713) genomic DNA. PCR was performed using oligonucleotides of SEQ ID NOs: 95 and 96 as primers to obtain a PCR product containing a coding region of ahr gene (SEQ ID NO: 94). Next, PCR was performed using "Macar(m)-npt-pACYCDuet" as a template and oligonucleotides of SEQ ID NOs: 97 and 98 as primers to obtain an "macar(m)-npt-pACYCDuet" fragment. The PCR product containing the coding region of each gene and the "macar(m)-npt-pACYCDuet" fragment were connected using In-Fusion HD cloning kit (trade name, manufactured by Clontech Laboratories, Inc.). An Escherichia coli JM109 strain was transformed, and a plasmid was extracted from the resulting transformant. "ahr-macar(m)-npt-pACYCDuet" was obtained as an Ahr, MaCar, and Npt co-expression plasmid.

### <Construction of C6 Compound Production Strain>

The constructed plasmid was transformed into Escherichia coli BL21(DE3) strain competent cells (manufactured by NIPPON GENE CO., LTD.), and applied to an LB agar medium (10 g/L of tryptone, 5 g/L of yeast extract, 5 g/L of sodium chloride, and 15 g/L of agar powder) containing 50 mg/L of ampicillin sodium and 30 mg/L of kanamycin sulfate (for construction of the strain HDO1, further containing 30 mg/L of chloramphenicol), and incubated at 37°C for 24 hours, thereby obtaining single colonies of transformants. The resulting transformants are shown in Table A-3.

**[Table A-3]**

| A-3: Transformants | | | |
|---|---|---|---|
| Transformant | Plasmid (1) | Plasmid (2) | Plasmid (3) |
| ADA1 | paaJ(Ec)-paaH(Ec)-pRSFDiret | paaF(Ec)-dcaA(Ab)-pETDuet | - |
| ADA2 | paaJ(Ec)-paaH(Ec)-pRSFDuet | paaF(L3)-ter(Ct)-pETDuet | - |
| ADA3 | paaJ(Ee)-paaH(Ec)-pRSFDuet | paaF(L3)-ter(caur)-pETDuet | - |
| ADA4 | paaJ(Ec)-paaH(Ec)-pRSPDnet | psaF(L3)-ter(kmx)-pETDuet | - |
| ADA5 | paaJ(Ec)-paaH(Ec)-pR3FDuet | paaF(L3)-ter(pkz)-pETDuet | - |
| ADA6 | paaJ(Ec)-paaH(Ec)-pRSFDuet | paaF(L3)-ter(mtm)-pETDuet | - |
| ADA7 | paaJ(Ec)-paaH(Ec)-pRSFDuet | paaF(L3)-ter(ttt)-pETDuet | - |
| ADA8 | paaJ(Ec)-paaH(Ec)-pRSFDuet | paaF(L3)-ter(cthr)-pETDuet | - |
| ADA9 | paaJ(Ec)-paaH(Ee)-pRSFDuet | paaF(L3)-ter(pan)-pETDuet | - |
| ADA10 | paaJ(Ec)-paaH(Ec)-pRSPDuet | paaF(L3)-ter(plj)-pETDuet | - |
| ADA11 | paaJ(Ec)-paaH(Ec)-pRSFDuet | paaF(L3)-ter(pte)-pETDuet | - |
| HDO1 | paaJ(Ec)-paaH(Ec)-pRSFDuet | paaF(L3)-ter(mtm)-pETDuet | ahr-macar(m)-npt-pACYCDuet |

### <Adipic Acid Production Test by Recombinant Escherichia Coli Culture>

The transformants ADA1 to 11 were inoculated with one platinum loop into 2 mL of an LB liquid medium (10 g/L of tryptone, 5 g/L of yeast extract, and 5g/L of sodium chloride) (14 mL capacity round bottom tube) containing 50 mg/L of ampicillin sodium and 30 mg/L of kanamycin sulfate, and shaking culture was performed at 37°C for 24 hours, thereby obtaining a pre-culture solution. 10 µL of the resulting pre-culture solution was inoculated into 1 mL of MM medium (96-well deep well plate) containing 50 mg/L of ampicillin sodium and 30 mg/L of kanamycin sulfate, and shaking culture was performed at 37°C for 48 hours. The composition of the MM medium is shown in Table A-4.

**[Table A-4]**

| Table A-4: MM medium composition | |
|---|---|
| KH₂PO₄ | 3 g/L |
| K₂HPO₄ | 6 g/L |
| (NH₄)₂SO₄ | 2.5 g/L |
| MgSO₄ · 7H₂O | 0.3 g/L |
| CaCl₂ · 2H₂O | 1 mg/L |
| FeSO₄ · 7H₂O | 6 mg/L |
| MnSO₄ · H₂O | 1.5 mg/L |
| AlCl₃ · 6H₂O | 1.5 mg/L |
| CoCl₂ | 0.6 mg/L |
| ZnSO₄ · 7H₂O | 0.3 mg/L |
| Na₂McO₂ · 2H₂O | 0.3 mg/L |
| CuCl₂ · 2H₂O | 0.2 mg/L |
| H₃BO₃ | 0.1 mg/L |
| MOPS (Adjusted to pH 7.4 with KOH) | 125 mM |
| Glycerol | 20 g/L |

After completion of the culture, the adipic acid concentration in the culture solution was analyzed. The analysis was performed by GC/MS analysis using trimethylsilyl derivatization. To 40 µL of the culture solution centrifugal supernatant (disodium sebacate was added as an internal standard so as to have a final concentration of 10 mM), 360 µL of an extract solution in which water, methanol, and chloroform were mixed so that water:methanol:chloroform was (5:2:2) (v/v/v), was added, and the mixture was thoroughly stirred with a vortex mixer. After centrifugation (16,000 × g, 5 minutes), 40 µL of the supernatant was collected in a separate microtube and centrifugally dried on a centrifugal evaporator for 1 hour. 100 µL of a 20 mg/mL pyridine solution of methoxyamine hydrochloride was added to the resulting dried solid, and the mixture was shaken at 30°C for 90 minutes. 50 µL of N-methyl-N-trimethylsilyltrifluoroacetamide was added and the mixture was shaken at 37°C for 30 minutes. The reaction solution was used as a sample for GC/MS measurement, and analysis was performed under the following conditions.

### GC/MS Analysis Conditions

Apparatus: GCMS-QP-2020NX (manufactured by Shimadzu Corporation)
Column: fused silica capillary tube inert treatment tube (length: 1 m, outer diameter: 0.35 mm, inner diameter: 0.25 mm, manufactured by GL Sciences Inc.), InertCap 5MS/NP (length: 30 m, inner diameter: 0.25 mm, film thickness: 0.25 um, manufactured by GL Sciences Inc.)
Sample injection amount: 1 µL
Sample introduction method: Split (split ratio: 25:1)
Vaporization chamber temperature: 230°C
Carrier gas: helium
Carrier gas linear velocity: 39.0 cm/sec
Oven temperature: kept at 80°C for 2 minutes → raised at 15°C/min → kept at 325°C for 13 minutes
Ionization method: electron ionization method (EI)
Ionization energy: 70 eV
Ion source temperature: 230°C
Scan range: m/z = 50 to 500

The results are shown in Table A-5. The transformants ADA1 to ADA11 are transformants into which expression plasmids of enzyme genes capable of progressing the reactions of Steps A, B, C, and D in Fig. 1 are introduced. The reaction of Step E proceeds by an endogenous enzyme of Escherichia coli.

In the culture of the transformant ADA1 in which PaaJ(Ec), PaaH(Ec), and PaaF(Ec) derived from Escherichia coli and dcaA(Ab) derived from Acinetobacter baylyi were expressed (Comparative Example 1), 0.14 g/L of adipic acid was produced. In the culture of the transformant ADA2 in which PaaJ(Ec) and PaaH(Ec) derived from Escherichia coli, PaaF(L3) derived from the L3 strain, and Ter(Ct) of Candida tropicalis were expressed (Comparative Example 2), 0.33 g/L of adipic acid was produced.

In the culture of the transformants ADA3 to ADA11 in which PaaJ(Ec) and PaaH(Ec) derived from Escherichia coli, PaaF(L3) derived from the L3 strain, and Ter(caur), Ter(kmx), Ter(pkz), Ter(mtm), Ter(ttt), Ter(cthr), Ter(pan), Ter(plj), or Ter(pte) as the enzyme that catalyzes the reaction of Step D in Fig. 1 were expressed (Examples 1 to 9), it was shown that each adipic acid was produced, and Ter(caur), Ter(kmx), Ter(pkz), Ter(mtm), Ter(ttt), Ter(cthr), Ter(pan), Ter(plj), or Ter(pte) had an enzymatic activity that promotes the adipic acid production pathway reaction.

In addition, in the culture of the transformants ADA6 (Example 4), ADA7 (Example 5), ADA8 (Example 6), ADA9 (Example 7), and ADA11 (Example 9), it was shown that adipic acid was produced more than in the comparative examples, and the amount of C6 compound produced was improved by expressing the enzyme Ter(mtm), Ter(ttt), Ter(cthr), Ter(pan), or Ter(pte). In addition, form these results, it was found that an excellent 2,3-dehydroadipyl-CoA reductase activity was exhibited in the microorganisms shown as examples.

**[Table A-5]**

| Table A-5: Adipic acid production test results | | | | | | |
|---|---|---|---|---|---|---|
| | Strain | Step A (3-oxoadipyl-CoA thiolase) | Step B (3-hydroxyadipyl-CoA dehydrogenase) | Step C (3-hydroxyadipyl-CoA dehydratase) | Step D (2,3-dehydroadipyl-CoA reductase) | Adipic acid concentration [g/L] |
| Comparative Example 1 | ADA1 | PaaJ (Ec) | PaaH (Ec) | PaaF (Ec) | DcaA (Ab) | 0.14 |
| Comparative Example 2 | ADA2 | PaaJ (Ec) | PaaH (Ec) | PaaF (L3) | Ter (Ct) | 0.33 |
| Example 1 | ADA3 | PaaJ (Ec) | PaaH (Ec) | PaaF (L3) | Ter (caur) | 0.18 |
| Example 2 | ADA4 | PaaJ (Ec) | PaaH (Ec) | PaaF (L3) | Ter (kmx) | 0.19 |
| Example 3 | ADA5 | PaaJ (Ec) | PaaH (Ec) | PaaF (L3) | Ter (pkz) | 0.10 |
| Example 4 | ADA6 | PaaJ (Ec) | PaaH (Ec) | PaaF (L3) | Ter (mtm) | 132 |
| Example 5 | ADA7 | PaaJ (Ec) | PaaH (Ec) | PaaF (L3) | Ter (ttt) | 0.94 |
| Example 6 | ADA8 | PaaJ (Ec) | PaaH (Ec) | PaaF (L3) | Ter (cthr) | 1.27 |
| Example 7 | ADA9 | PaaJ (Ec) | PaaH (Ec) | PaaF (L3) | Ter (pan) | 0.84 |
| Example 8 | ADA10 | PaaJ (Ec) | PaaH (Ec) | PaaF (L3) | Ter (plj) | 0.16 |
| Example 9 | ADA11 | PaaJ (Ec) | PaaH (Ec) | PaaF (L3) | Ter (nte) | 0.76 |

### <1,6-Hexanediol and 6-Hydroxyhexanoic Acid Production Test by Recombinant Escherichia Coli Culture>

The transformant HDO1 was inoculated with one platinum loop into 2 mL of an LB liquid medium (14 mL capacity round bottom tube) containing 50 mg/L of ampicillin sodium, 30 mg/L of kanamycin sulfate, and 30 mg/L of chloramphenicol, and shaking culture was performed at 37°C for 24 hours, thereby obtaining a pre-culture solution. 10 µL of the resulting pre-culture solution was inoculated into 1 mL of MM medium (96-well deep well plate) containing 50 mg/L of ampicillin sodium, 30 mg/L of kanamycin sulfate, and 30 mg/L of chloramphenicol, and shaking culture was performed at 37°C for 48 hours.

After completion of the culture, the 1,6-hexanediol concentration and 6-hydroxyhexanoic acid concentration in the culture solution were analyzed. The 6-hydroxyhexanoic acid concentration was analyzed using GC/MS under the same conditions as in the adipic acid concentration analysis. The analysis of the 1,6-hexanediol concentration was performed under the following conditions using a high performance liquid chromatography Prominence system (manufactured by Shimadzu Corporation).

### High Performance Liquid Chromatography (HPLC) Analysis Conditions

Detector: differential refractive index detector
Column: Shim-Pack Fast-OA(G), Fast-OA (manufactured by Shimadzu Corporation)
Oven temperature: 40°C
Mobile phase: 8 mM methanesulfonic acid aqueous solution
Flow rate: 0.6 mL/min
Injection amount: 10 µL

The results are shown in Table A-6. The transformant HDO1 is a transformant into which expression plasmids of a carboxylic acid reductase and an alcohol dehydrogenase are introduced in addition to the expression plasmids of the enzyme genes capable of progressing the reactions of Steps A, B, C, and D in Fig. 1. In the culture of the transformant HDO1 (Example 10), 6-hydroxyhexanoic acid and 1,6-hexanediol were produced.

**[Table A-6]**

| Table A-6: 6-Hydroxyhexanoic acid and 1,6-hexanediol production test results | | | | | | | | | |
|---|---|---|---|---|---|---|---|---|---|
| | Strain | Step A (3-oxoadipyl-CoA thiolase) | Step B (3-hydroxyadipyl-CoA dehydrogenase) | Step C (3-hydroxyadipyl-CoA dehydratase) | Step D (2,3-dehydroadipyl-CoA reductase) | Carboxylic acid reductase | Alcohol dehydrogenase | 1,6-Hexanediol concentration (mg/L) | 6-Hydroxyhexanoic acid concentration (mg/L) |
| Example 10 | HDO1 | PaaJ (Ec) | PaaH (Ec) | PaaF (L3) | Ter (mtm) | MaCar (m) | Ahr | 56 | 3 |

### <Construction of Plasmid for Gene Disruption>

Disruption and insertion of the gene of Escherichia coli were performed by a homologous recombination method using pHAK1 (deposited with biotechnology division of National Institute of Technology and Evaluation (NITE), Patent Microorganisms Depositary (NPMD) (address: Room 122, 2-5-8, Kazusa-Kamatari, Kisarazu, Chiba) with Accession Number: NITE P-02919, on March 18, 2019, International Deposit Number: NITE BP-02919). pHAK1 includes a temperature-sensitive variant repA gene, a kanamycin resistant gene, and a levansucrase gene sacB derived from Bacillus subtilis. The levansucrase gene lethally acts on a host microorganism under the presence of sucrose. The PCR fragment was amplified using PrimeSTAR Max DNA Polymerase (trade name, manufactured by TAKARA BIO INC.) or KOD FX Neo (trade name, manufactured by Toyobo Co., Ltd.), and the plasmid was prepared using an Escherichia coli HST08 strain.

Using the genomic DNA of the Escherichia coli BL21(DE3) strain as a template, a PCR product containing a 5' homologous region, a coding region, and a 3' homologous region of a disruption target gene was obtained. The combinations of the target gene and the primer sequence are shown in Table A-7.

**[Table A-7]**

| Table A-7: Primer set used in plasmid construction | |
|---|---|
| Gene name | SEQ ID NO: |
| atoB | 99,100 |
| sucD | 101,102 |
| IdhA | 103,104 |
| adhE | 105,106 |
| yqhD, dkgA | 107,108 |
| yahK | 109,110 |
| ahr | 111,112 |
| pflB | 113,114 |

Next, the present PCR product was inserted into the pHAK1 plasmid fragment amplified using primers of SEQ ID NOs: 115 and 116 using In-Fusion HD cloning kit (trade name, manufactured by Clontech Laboratories, Inc.) and the PCR product was circularized. An Escherichia coli HST08 strain was transformed, and a plasmid was extracted from the resulting transformant.

PCR was performed using the pHAK1 plasmid into which the DNA fragment containing the 5' homologous region, the coding region, and the 3' homologous region of the resulting disruption target gene was inserted as a template and using primers shown in Table A-8, thereby obtaining a pHAK1 plasmid fragment in which the coding region of the disruption target gene was partially or entirely removed and the 5' homologous region and the 3' homologous region were contained.

**[Table A-8]**

| Table A-8: Primer set used in plasmid construction | |
|---|---|
| Gene name | SEQ ID NO: |
| atoB | 117,118 |
| sucD | 119,120 |
| IdhA | 121,122 |
| adhe | 123,124 |
| yqhD, dkgA | 125,126 |
| yahK | 127,128 |
| ahr | 129,130 |
| pflB | 131,132 |

The resulting plasmid fragment was circularized by terminal phosphorylation and self-ligation. An Escherichia coli HST08 strain was transformed, and a plasmid was extracted from the resulting transformant to obtain a plasmid for gene disruption.

### <Construction of Plasmid for Inserting Hexamethylenediamine Production Pathway Enzyme Gene>

A polynucleotide (SEQ ID NO: 33) encoding PaaJ(Ec) (SEQ ID NO: 1) of Eshcherichia coli was subjected to cloning from Eshcherichia coli W3110 strain (NBRC12713) genomic DNA. A polynucleotide (SEQ ID NO: 38) encoding PaaH(Ec) (SEQ ID NO: 2) of Eshcherichia coli was obtained by cloning from Eshcherichia coli W3110 strain (NBRC12713) genomic DNA.

A plasmid for inserting a gene was designed so that the paaJ(Ec) and paaH(Ec) genes were inserted into a pflB gene region as an expression cassette. An expression cassette in which a promoter region (SEQ ID NO: 133), a coding region of paaJ(Ec) gene, a linker sequence (SEQ ID NO: 134), a coding region of paaH gene, and a terminator region (SEQ ID NO: 135) were sequentially arranged in this order was incorporated between the 5' homology region and the 3' homology region of the coding region of pflB gene contained in the previously constructed plasmid for disrupting the pflB gene.

The nucleotide set forth in SEQ ID NO: 4 and the nucleotide set forth in SEQ ID NO: 46 were used as primers, and the nucleotide encoding PaaF(L3) of the L3 strain encoded by the base sequence of the PCR amplification product obtained using the chromosomal DNA of the L3 strain as a template was obtained by optimizing the base sequence for expressing Escherichia coli using the artificial gene synthesis service of Eurofins Genomics LLC. The nucleotide set forth in SEQ ID NO: 162 and the nucleotide set forth in SEQ ID NO: 163 were used as primers, and the polynucleotide (1,155 bp) encoding MmgC(L3) of the L3 strain encoded by the base sequence of the PCR amplification product obtained using the chromosomal DNA of the L3 strain as a template was obtained by optimizing the base sequence for expressing Escherichia coli using the artificial gene synthesis service of Eurofins Genomics LLC.

A plasmid for inserting a gene was designed so that the optimized sequences of the coding regions of paaF(L3) and mmgC (L3) genes were inserted into a yahK gene region as an expression cassette. An expression cassette in which a promoter region (SEQ ID NO: 136), a coding region of paaF(L3) gene, a linker sequence (SEQ ID NO: 137), a coding region of mmgC(L3) gene, and a terminator region (SEQ ID NO: 138) were sequentially arranged in this order was incorporated between the 5' homology region and the 3' homology region of the coding region of yahK gene included in the previously constructed plasmid for disrupting the yahK gene.

As a carboxylic acid reductase MaCar (SEQ ID NO: 20) of Mycobacterium abscessus, a variant MaCar(m) in which the 283rd tryptophan residue of the amino acid sequence was substituted with an arginine residue and the 303rd alanine residue was substituted with a methionine residue was used (SEQ ID NO: 22). The polynucleotide (SEQ ID NO: 86) encoding MaCar(m) was obtained by optimizing the base sequence for expressing Escherichia coli using the artificial gene synthesis service of Eurofins Genomics LLC. A polynucleotide (SEQ ID NO: 89) encoding Npt (SEQ ID NO: 24) of Nocardia iowensis was obtained by cloning from the genomic DNA of the Nocardia iowensis JCM18299 strain (provided by RIKEN BRC through the National BioResource Project of the Ministry of Education, Culture, Sports, Science and Technology).

A plasmid for inserting a gene was designed so that the macar(m) and npt genes were inserted into an ldhA gene region as an expression cassette. An expression cassette in which a promoter region (SEQ ID NO: 139), a coding region of macar(m) gene, a linker sequence (SEQ ID NO: 140), a coding region of npt gene, and a terminator region (SEQ ID NO: 141) were sequentially arranged in this order was incorporated between the 5' homology region and the 3' homology region of the coding region of ldhA gene contained in the previously constructed plasmid for disrupting the ldhA gene.

### <Construction of Escherichia Coli Modified Strain>

A plasmid for disrupting or inserting a desired gene was transformed into the Escherichia coli BL21(DE3) strain by an electroporation method (see Genetic Engineering Laboratory Notebook, by Takaaki Tamura, Yodosha), and then applied to an LB agar medium (10 g/L of tryptone, 5 g/L of yeast extract, 5 g/L of sodium chloride, and 15 g/L of agar powder) containing 100 mg/L of kanamycin sulfate, and incubation was performed at 30°C overnight to obtain a single colony, thereby obtaining a transformant. The present transformant was inoculated into 1 mL of an LB liquid medium (10 g/L of tryptone, 5 g/L of yeast extract, and 5 g/L of sodium chloride) containing 100 mg/L of kanamycin sulfate with one platinum loop, and shaking culture was performed at 30°C. The resulting culture solution was applied to an LB agar medium containing 100 mg/L of kanamycin sulfate, and incubation was performed at 42°C overnight, thereby obtaining a colony. The resulting colony was inoculated into 1 mL of an LB liquid medium with one platinum loop, and shaking culture was performed at 30°C. The resulting culture solution was applied to an LB agar medium containing 20% sucrose, and incubation was performed at 30°C for 2 days. Disruption or insertion of a desired gene in the resulting colony was confirmed by colony direct PCR using the primer set shown in Table A-9.

The above operation was repeated to construct an Escherichia Coli strain "No. 060" shown in Table A-10 and containing a plurality of gene disruptions and gene insertions. In the table, Δ indicates that the enzyme gene is deleted, and the "Gene name A::Gene name B" indicates that the gene A region is substituted with a region including the gene B.

**[Table A-9]**

| Table A-9: Primer set for confirmation of target disruption and insertion | |
|---|---|
| Gene name | SEQ ID NO: |
| atoB | 142,143 |
| sucD | 144,145 |
| IdhA | 146,147 |
| adhe | 148,149 |
| yqhD, dkgA | 150,151 |
| yahK | 152,153 |
| ahr | 154,155 |
| pflB | 156,157 |

**[Table A-10]**

| Table A-10: Escherichia coli modified strain | |
|---|---|
| Strain No. | Gene disruption and insertion |
| No.060 | ΔatoB, ΔsucD, ΔldhA, ΔadhE, ΔyqhD, ΔdkgA, ΔyahK, Δahr, ΔpflB |
| | pflB::paaJ(Ec)-paaH(Ec) |
| | yahK::paaF(L3)-mmgC(L3) |
| | ldhA::macar(m)-npt |

### <Construction of Plasmid for Expressing Hexamethylenediamine Production Pathway Enzyme Gene>

The PCR fragment was amplified using PrimeSTAR Max DNA Polymerase (trade name, manufactured by TAKARA BIO INC.) or KOD FX Neo (trade name, manufactured by Toyobo Co., Ltd.), and the plasmid was prepared using an Escherichia coli JM109 strain.

As YgjG (SEQ ID NO: 27) of Eshcherichia coli, a variant YgjG(m) (SEQ ID NO: 158) in which some amino acid sequences were substituted was used. A polynucleotide encoding YgjG(m) was subjected to PCR using the oligonucleotides of SEQ ID NOs: 160 and 161 as primers to obtain a PCR product containing a coding region of ygjG(m) gene (SEQ ID NO: 159). Next, the present PCR product was inserted in the cleavage site of restriction enzymes NcoI and HindIII in pACYCDuet-1 (trade name, manufactured by Merck & Co., Inc.) using In-Fusion HD cloning kit (trade name, manufactured by Clontech Laboratories, Inc.). An Escherichia coli JM109 strain was transformed, and a plasmid was extracted from the resulting transformant. "ygjG(m)-pACYCDuet" was obtained as an MaCar(m) expression plasmid.

### <Hexamethylenediamine Production Test by Recombinant Escherichia Coli Culture>

An Escherichia coli modified strain No. 060 was transformed with "paaF(L3)-ter(mtm)-pETDuet" and "ygjG(m)-pACYCDuet" by an electroporation method, and culture was performed at 37°C for one day on an LB agar medium containing 50 mg/L of ampicillin sodium 30 mg/L of chloramphenicol to form a colony, thereby obtaining a transformant HMD1. The colony of the transformant HMD1 was inoculated with one platinum loop into 2 mL of an LB liquid medium (14 mL capacity round bottom tube) containing 50 mg/L of ampicillin sodium and 30 mg/L of chloramphenicol, and shaking culture was performed at 37°C for 3 to 5 hours, thereby obtaining a pre-culture solution. In a 250 mL capacity Jar culture device (instrument name: Bio Jr. 8, manufactured by ABLE Corporation & Biott Corporation), 0.5 mL of the pre-culture solution was added to a synthetic medium containing 50 mg/L of carbenicillin sodium, 30 mg/L of chloramphenicol, and 0.02 mM IPTG (shown in the following table), and a main culture was performed. Culture conditions are as follows: culture temperature 37°C, culture pH 7.0, pH adjustment 10% (w/v) ammonia water, stirring at 750 rpm, and air flow 1 vvm. Glucose was added so as to have a final concentration of 35 g/L at the time when 22 hours has passed from the inoculation of the pre-culture solution, and culture was performed until 41 hours has passed.

**[Table A-11]**

| Table A-11: Synthetic medium composition | |
|---|---|
| KH₃PO₄ | 3 g/L |
| K₂HPO₄ | 6 g/L |
| (NH₄)₂SO₄ | 2.5 g/L |
| MgSO₄ · 7H₂O | 0.3 g/L |
| CaCl₂ · 2H₂O | 1 mg/L |
| FeSO₄ · 7H₂O | 6 mg/L |
| MnSO₄ · H₂O | 1.5 mg/L |
| AlCl₃ · 6H₂O | 1.5 mg/L |
| CoCl₂ | 0.6 mg/L |
| ZnSO₄ · 7H₂O | 0.3 mg/L |
| Na₂MoO₂ . 2H₂O | 0.3 mg/L |
| CuCl₂ · 2H₂O | 0.2 mg/L |
| H₃BO₃ | 0.1 mg/L |
| MOPS (Adjusted to pH 7.4 with KOH) | 125 mM |
| Glucose | 20 g/L |

After completion of the culture, the culture solution was separated into the microbial cells and the supernatant by centrifugation, and a hexamethylenediamine concentration in the supernatant was analyzed. The analysis of the hexamethylenediamine concentration was performed under the following conditions using ion chromatograph.

### Ion Chromatograph Analysis Conditions

Apparatus: ICS-3000 (manufactured by Dionex Corporation)
Detector: electrical conductivity detector
Column: IonPac CG19 (2 × 50 mm)/CS19(2 × 250 mm) (manufactured by Thermo Fisher Scientific)
Oven temperature: 30°C
Mobile phase: 8 mM aqueous methanesulfonic acid solution (A), 70 mM aqueous methanesulfonic acid solution (B)
Gradient condition: (A: 100%, B: 0%)-(10 min)-(A: 0%, B: 100%)-(holding for 1 min)
Flow rate: 0.28 mL/min
Injection amount: 20 µL

The results are shown in Table A-12. The transformant HMD1 is a transformant into which a carboxylic acid reductase and an aminotransferase gene are introduced in addition to enzyme genes capable of proceeding the reactions of Steps A, B, C, and D in Fig. 1. Hexamethylenediamine was produced by culturing the transformant HMD1 (Example 11).

**[Table A-12]**

| Table A-12: Hexamethylenediamine production strain culture results | | | | | | | | |
|---|---|---|---|---|---|---|---|---|
| | Transformant name | Step A (3-oxoadipyl-CoA thiolase) | Step B (3-hydroxyadipyl-CoA dehydrogenase) | Step C (3-hydroxyadipyl-CoA dehydratase) | Step D (2,3-dehydroadipyl-CoA reductase) | Carboxylic acid reductase | Aminotransferase | Hexamethylenediamine concentration [mg/L] |
| Example 11 | HMD1 | PaaJ (Ec) | PaaH (Ec) | PaaF (L3) | Ter (mtm) | MaCar (m) | YgjG (m) | 16 |

### <2> Invention B

Hereinafter, the present invention B will be described based on examples, and the present invention B is not limited to these examples.

### <Isolation and Identification of the Genus Burkholderia (Burkholderia sp.) LEBP-3 Strain>

The genus Burkholderia (Burkholderia sp.) LEBP-3 strain was isolated from activated sludge in Japan using a medium containing adipic acid as the sole carbon source and energy source. The genus Burkholderia (Burkholderia sp.) LEBP-3 strain has been deposited with National Institute of Technology and Evaluation, Patent Microorganisms Depositary (NPMD) (Address: Room 122, 2-5-8 Kazusa-Kamatari, Kisarazu, Chiba) on December 4, 2020 (original date of deposit) (Receipt Number: NITE ABP-03334), and is internationally deposited under "Accession Number: NITE BP-03334". The classification characteristics of the Burkholderia sp. LEBP-3 strain are as follows.

### <Mycological Properties of LEBP-3 Strain>

Culture temperature: 37°C,
Cell morphology: Bacilli (0.7 × 1.7-3.0 um),
Gram staining properties: -,
   Presence or absence of spores: -,
Motility: +,
Colony morphology when cultured in LB agar medium for 48 h: diameter of 1.9 to 2.5 mm, light yellow, circular,
Lenticular, full edge, smooth, opaque, butter-like,
Growth at 30°C: +,
Growth at 45°C: +,
Catalase reaction: +,
Oxidase reaction: +,
Acid/gas production from glucose: +/-,
O/F test (oxidation/fermentation): +/-,
Nitrate reducing property: -,
Indole productivity: -,
Glucose acidification: -,
Urease: not present,
Aesculin degradability: +,
Glucose, L-arabinose, D-mannose, N-acetyl-D-glucosamine, gluconic acid, n-capric acid, adipic acid, malic acid, citric acid, and phenyl acetate: assimilable, and
Maltose: not assimilable.

### <Phylogenetic Analysis by 16S rRNA Gene Sequence Homology of LEBP-3 Strain>

The 16S rDNA gene was identified by base sequence analysis. The base sequence of the 16S rDNA gene was amplified by PCR and subjected to sequence analysis. DB-BA11.0 (TechnoSuruga Laboratory Co., Ltd.) and the international base sequence database were used as databases for BLAST homology search. As a result, the LEBP-3 strain was contained in a cluster composed of the genus Burkhlderia, but showed a molecular phylogenetic position different from that of any known species, such that the species name was not identified.

### <Acquisition of Enzyme Gene by PCR Amplification and Homology Evaluation>

Whole-genome draft analysis of Burkholderia sp. LEBP-3 strain was performed. The LEBP-3 strain was subjected to shaking culture at 37°C in 2 mL of an LB medium (10 g/L of tryptone, 5 g/L of yeast extract, and 10 g/L of sodium chloride).
After completion of the culture, microbial cells were recovered from the culture solution, and a genomic DNA was extracted using Nucleo Spin Tissue (trade name, manufactured by MACHEREY-NAGEL). Whole-genome draft analysis and annotation of the Burkholderia sp. LEBP-3 strain were performed, and 7679 open reading frames (ORFs) were identified. Based on this information, paaJ(L3) as a succinyl-CoA:acetyl-CoA acyltransferase, paaH(L3) as a 3-hydroxyadipyl-CoA dehydrogenase, paaF(L3) as a 3-hydroxyadipyl-CoA dehydratase, and mmgC(L3) as a 2,3-dehydroadipyl-CoA reductase were identified.

The results of performing the BLAST search using the PaaJ(L3) sequence as a query are shown in Table B-1, the results of performing the BLAST search using the PaaH(L3) sequence as a query are shown in Table B-2, the results of performing the BLAST search using the PaaF(L3) sequence as a query are shown in Table B-3, and the results of performing the BLAST search using the MmgC(L3) sequence as a query are shown in Table B-4. "Refseq_Protein" was designated as the database at the time of sequence search.

**[Table B-1]**

| Name | Cover rate (%) | Sequence identity (%) | Accession No. |
|---|---|---|---|
| 3-oxoadipyl-CoA thiolase [Sphingomonas sp. 3P27F8; | 99% | 94.0 | WP_137788344.1 |
| 3-oxoadipyl-CoA thiolase [Aromatoleum bremense] | 99% | 89.0 | WP_169204100.1 |
| 3-oxoadipyl-CoA thiolase [Aromatoleum tolulylicum] | 99% | 86.0 | WP_076601068.1 |
| 3-oxoadipyl-CoA thiolase [Noviherbaspirillum massiliense] | 99% | 86.3 | WP_019143081.1, |
| 3-oxoadipyl-CoA thiolase [Azoarcus sp. CIS] | 99% | 84.8 | WP_050416542.1 |
| 3-oxoadipyl-CoA thiolase [Aromatoleum diolicum] | 99% | 96.5 | WP_169259361.1 |
| 3-oxoadipyl-CoA thiolase [Saezia sanguinis] | 99% | 82.8 | WP_126979818.1 |
| 3-oxoadipyl-CoA thiolase [unclassified Massilia] | 99% | 82.7 | WP_047825632.1 |
| 3-oxoadipyl-CoA thiolase [Burkholderia cepacia] | 99% | 81.0 | WP_043186899.1 |
| 3-oxoadipyl-CoA thiolase [Burkholderia anthina] | 99% | 81.0 | WP_175781473.1 |

**[Table B-2]**

| Name | Cover rate (%) | Sequence identity (%) | Accession No. |
|---|---|---|---|
| 3-hydroxyacyl-CoA dehydrogenase [Aromatoleum diolicum] | 93% | 75.9 | WP_169259360.1 |
| 3-hydroxyacyl-CoA dehydrogenase [Aromatoleum bremense] | 93% | 76.0 | WP_169204099.1 |
| 3-hydroxyacyl-CoA dehydrogenase [Noviherbaspirillum massiliense] | 93% | 76.6 | WP_019143080.1 |
| 3-hydroxyacyl-CoA dehydrogenase [Saezia sanguinis] | 93% | 74.0 | WP_126979819.1 |
| 3-hydroxyacyl-CoA dehydrogenase [Aromatoleum tolulyticum] | 93% | 74.0 | WP_076601067.1 |
| 3-hydroxyacyl-CoA dehydrogenase [unclassified Massilia] | 93% | 74.1 | WP_047825631.1 |
| 3-hydroxyacyl-CoA dehydrogenase [Azoarcus sp. CIB] | 93% | 72.3 | WP_050416541.1 |
| 3-hydroxyacyl-CoA dehydrogenase [Massilia sp. HP4] | 93% | 68.0 | WP_137174141.1 |
| 3-hydroxyacyl-CoA dehydrogenase [Azoarcus sp. KH32C] | 93% | 61.4 | WP_041656320.1 |
| 3-hydroxyacyl-CoA dehydrogenase [Aromatoleum diolicum] | 93% | 66.6 | WP_169260331.1 |

**[Table B-3]**

| Name | Cover rate (%) | Sequence identity (%) | Accession No. |
|---|---|---|---|
| enoyl-CoA hydratase/isomerase family protein [Noviherbaspirillum massiliense] | 97% | 83.9 | WP_019143079.1 |
| enoyl-CoA hydratase/isomerase family protein [unclassified Massilia] | 97% | 82.8 | WP_047825630.1 |
| enoyi-CoA hydratase/isomerase family protein [Azoarcus sp. C18] | 97% | 81.2 | WP_050416540.1 |
| enoyl-CoA hydratase/isomerase family protein [Saezia sanguinis] | 97% | 78.0 | WP_126979820.1 |
| enoyl-CoA hydratase/isomerase family protein [Aromatoleum tolulyticum] | 97% | 79.2 | WP_076601066.1 |
| enoyl-CoA hydratase/isomerase family protein [Aromatoleum diolicum] | 99% | 73.9 | WP_169259359.1 |
| enoyl-CoA hydratase/isomerase family protein [Brachymonas denitrificans] | 93% | 78.7 | WP_091813494.1 |
| enoyl-CoA hydratase/isomerase family protein [Brachymonas chironomi] | 94% | 77.2 | WP_018715326.1 |
| enoyl-CoA hydratase/isomerase family protein [Pseudomonas alkylphenolica] | 93% | 75.8 | WP_157192348.1 |
| enoyl-CoA hydratase/isomerase family protein [Pseudomonas alkylphenolica] | 93% | 75.4 | WP_128326511.1 |

**[Table B-4]**

| Name | Cover rate (%) | Sequence identity (%) | Accession No. |
|---|---|---|---|
| acyl-CoA dehydrogenase family protein [Paraburkholderia diazotrophica] | 98% | 90.6 | WP_090867470.1 |
| acyl-CoA dehydrogenase family protein [Burkholderiaceae] | 98% | 90.6 | WP_028365992.1 |
| acyl-CoA dehydrogenase family protein [Massilia niastensis] | 99% | 90.4 | WP_026355172.1 |
| acyl-CoA dehydrogenase family protein [Paraburkholderia dipogonis] | 98% | 90.6 | WP_134455213.1 |
| acyl-CoA dehydrogenase family protein [Paraburkholderia piptadeniae] | 98% | 90.3 | WP_087738639.1 |
| acyl-CoA dehydrogenase family protein [Paraburkholderia sp. 5N] | 98% | 89.5 | WP_172166833.1 |
| acyl-CoA dehydrogenase family protein [Noviherbaspirillum massiliense] | 99% | 90.3 | WP_019143077.1 |
| acyl-CoA dehydrogenase family protein [Paraburkholderia steynii] | 98% | 90.6 | WP_131239939.1 |
| acyl-CoA dehydrogenase family protein [Paraburkholderia sp. PGU16] | 98% | 90.0 | WP_180723506.1 |
| acyl-CoA dehydrogenase family protein [unclassified Massilia] | 99% | 89.3 | WP_047825629.1 |

From the above results, the enzyme of the Burkholderia sp. LEBP-3 strain was identified as a novel sequence. The paaJ(L3) gene can be subjected to PCR amplification using the nucleotide set forth in SEQ ID NO: 164 and the nucleotide set forth in SEQ ID NO: 165 as primers. The paaH(L3) gene can be subjected to PCR amplification using the nucleotide set forth in SEQ ID NO: 166 and the nucleotide set forth in SEQ ID NO: 167 as primers. The paaF(L3) gene can be subjected to PCR amplification using the nucleotide set forth in SEQ ID NO: 168 and the nucleotide set forth in SEQ ID NO: 169 as primers. The mmgC(L3) gene can be subjected to PCR amplification using the nucleotide set forth in SEQ ID NO: 170 and the nucleotide set forth in SEQ ID NO: 171 as primers. The primers used for PCR, the respective enzymes and the base sequences encoding the enzymes are illustrated in Figs. 17 to 20.

Examples of the PCR amplification conditions are shown below. As the enzyme, PrimeSTAR Max DNA Polymerase (trade name, manufactured by TAKARA BIO INC.) was used. 30 cycles of a treatment was performed under conditions of a heat treatment at 98°C for 10 seconds, annealing at 55°C for 15 seconds, and elongation at 72°C and 5 sec/kb. Oligonucleotides used as primers were added at 1 µM each. The liquid volume was 25 µL.

### <Construction of Recombinant Escherichia Coli Expressing Acquired Enzyme Gene>

The PCR fragment was amplified using PrimeSTAR Max DNA Polymerase (trade name, manufactured by TAKARA BIO INC.), and the plasmid was prepared using an Escherichia coli JM109 strain. For the codon optimization of the base sequence, GeneArt GeneOptinizer (Software name, Thermo Fisher Scientific Inc.) or an artificial gene synthesis service of Eurofins Genomics LLC was used.

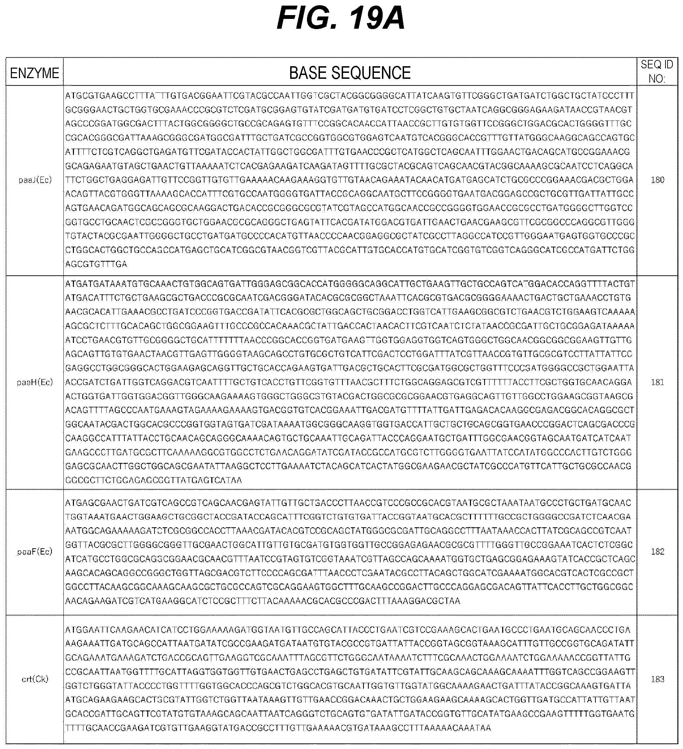

A polynucleotide encoding PaaJ(Ec) of Eshcherichia coli was subjected to cloning from Eshcherichia coli W3110 strain (NBRC12713) genomic DNA. PCR was performed using oligonucleotides (Fig. 20A) of SEQ ID NOs: 188 and 189 as primers to obtain a PCR amplification product containing a coding region (SEQ ID NO: 180) (Fig. 19A) of paaJ(Ec) gene. Next, PCR was performed using pRSFDuet-1 (trade name, manufactured by Merck & Co., Inc.) as a template and nucleotides (Fig. 20A) set forth in SEQ ID NOs: 190 and 191 as primers to obtain a pRSFDuet-1 fragment. The DNA fragment containing a gene coding region and the pRSFDuet-1 fragment were connected using In-Fusion HD cloning kit (trade name, manufactured by Clontech Laboratories, Inc.). An Escherichia coli JM109 strain was transformed, and a plasmid was extracted from the resulting transformant. "prm203" was obtained as a PaaJ(Ec) expression plasmid.

A polynucleotide encoding PaaH(Ec) of Eshcherichia coli was obtained by cloning from Eshcherichia coli W3110 strain (NBRC12713) genomic DNA. PCR was performed using nucleotides (Fig. 20A) set forth in SEQ ID NOs: 192 and 193 as primers to obtain a PCR amplification product containing a coding region (SEQ ID NO: 181) (Fig. 19A) of paaH(Ec) gene. Next, PCR was performed using prm203 as a template and using nucleotides (Fig. 20A) set forth in SEQ ID NOs: 194 and 195 as primers to obtain a prm203 fragment. The DNA fragment containing a coding region of each gene and the prm203 fragment were connected using In-Fusion HD cloning kit (trade name, manufactured by Clontech Laboratories, Inc.). An Escherichia coli JM109 strain was transformed, and a plasmid was extracted from the resulting transformant. "prm69" was obtained as a PaaJ(Ec)-PaaH(Ec) expression plasmid.

A polynucleotide encoding PaaF(Ec) of Eshcherichia coli was obtained by cloning from Eshcherichia coli W3110 strain (NBRC12713) genomic DNA. PCR was performed using oligonucleotides (Fig. 20A) set forth in SEQ ID NOs: 196 and 197 as primers to obtain a PCR amplification product containing a coding region (SEQ ID NO: 182) (Fig. 19A) of paaF(Ec) gene. The polynucleotide encoding PaaF(L3) of the L3 strain was obtained by optimizing the base sequence for expressing Escherichia coli using the artificial gene synthesis service of Eurofins Genomics LLC. PCR was performed using nucleotides (Fig. 20A) set forth in SEQ ID NOs: 198 and 199 as primers to obtain a PCR amplification product containing a coding region of paaF(L3) gene. PCR was performed using pETDuet-1 (trade name, manufactured by Merck & Co., Inc.) as a template and nucleotides (Fig. 20A) set forth in SEQ ID NOs: 204 and 205 as primers to obtain a pETDuet-1 fragment. The DNA fragment containing a coding region of each gene and the pETDuet-1 fragment were connected using In-Fusion HD cloning kit (trade name, manufactured by Clontech Laboratories, Inc.). An Escherichia coli JM109 strain was transformed, and a plasmid was extracted from the resulting transformant. "prm74" was obtained as a PaaF(Ec) expression plasmid and "prm194" was obtained as a PaaF(L3) expression plasmid.

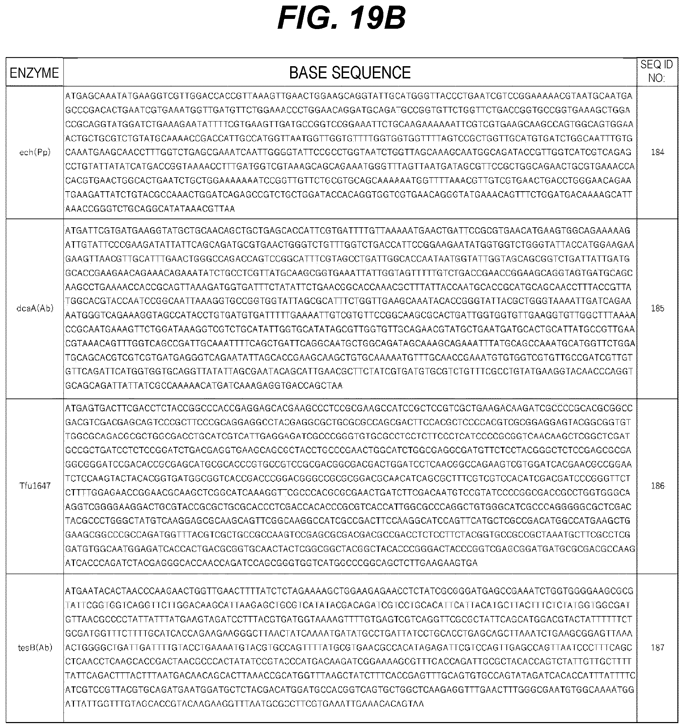

The polynucleotides encoding dcaA(Ab) of Acinetobacter baylyi, Tfu1647 of Thermobifida fusca, and MmgC(L3) of the L3 strain were obtained by optimizing the base sequences for expressing Escherichia coli using the artificial gene synthesis service of Eurofins Genomics. The dcaA(Ab) gene (SEQ ID NO: 185) (Fig. 19B) was subjected to PCR using the nucleotides set forth in SEQ ID NOs: 206 and 207 (Fig. 20A) as primers, the Tfu1647 gene (SEQ ID NO: 186) (Fig. 19B) of Thermobifida fusca was subjected to PCR using the nucleotides set forth in SEQ ID NOs: 208 and 209 (Fig. 20A) as primers, and the mmgC5(L3) gene was subjected to PCR using the nucleotides set forth in SEQ ID NOs: 210 and 211 (Fig. 20A) as primers, thereby obtaining PCR amplification products each containing a coding region of dcaA(Ab), Tfu1647, or mmgC5(L3) genes, respectively. PCR was performed using prm74 and prm194 as a template and the nucleotides set forth in SEQ ID NOs: 212 and 213 (Fig. 20A) as primers to obtain each of a prm74 fragment and a prm194 fragment. The DNA fragment containing the coding region of each gene, the prm74 fragment, and the prm194 fragment were connected using In-Fusion HD cloning kit (trade name, manufactured by Clontech Laboratories, Inc.). An Escherichia coli JM109 strain was transformed, and a plasmid was extracted from the resulting transformant. "prm127", "prm234", and "prm166" were obtained as a PaaF(Ec)-dcaA(Ab) expression plasmid, a PaaF(Ec)-mmgC(L3) expression plasmid, and a PaaF(L3)-mmgC(L3) expression plasmid, respectively.

The polynucleotide encoding tesB(Ab) of Acinetobacter baylyi was subjected to PCR from a chromosomal DNA of Acinetobacter baylyi using the nucleotides set forth in SEQ ID NOs: 214 and 215 (Fig. 20A) as primers, thereby obtaining PCR amplification products each containing a coding region of tesB(Ab) gene. PCR was performed using pCDFDuet-1 as a template and the nucleotides set forth in SEQ ID NOs: 216 and 217 (Fig. 20A) as primers to obtain a pCDFDuet-1 fragment. The DNA fragment containing a coding region of each tesB(Ab) gene and the pCDFDuet-1 fragment were connected using In-Fusion HD cloning kit (trade name, manufactured by Clontech Laboratories, Inc.). An Escherichia coli JM109 strain was transformed, and a plasmid was extracted from the resulting transformant. "prm92" was obtained as a TesB(Ab) expression plasmid.

A gene-disrupted strain of an Escherichia coli BL21(DE3) strain was produced as follows. Disruptions of the ldh gene encoding lactate dehydrogenase, the atoB gene encoding an acetyl-CoA acetyltransferase, and the sucD gene encoding a succinyl-CoA synthetase α-subunit were performed by a homologous recombination method using pHAK1 (deposited with biotechnology division of National Institute of Technology and Evaluation (NITE), Patent Microorganisms Depositary (NPMD) (address: Room 122, 2-5-8, Kazusa-Kamatari, Kisarazu, Chiba) with Accession Number: NITE P-02919, on March 18, 2019, International Deposit Number: NITE BP-02919). pHAK1 includes a temperature-sensitive variant repA gene, a kanamycin resistant gene, and a levansucrase gene SacB derived from Bacillus subtilis. The levansucrase gene lethally acts on a host microorganism under the presence of sucrose. The PCR fragment was amplified using PrimeSTAR Max DNA Polymerase (trade name, manufactured by TAKARA BIO INC.), and the plasmid was prepared using an Escherichia coli HST08 strain.

Using the genomic DNA of the Escherichia coli BL21(DE3) strain as a template, a PCR amplification product containing an upstream region, a coding region, and a downstream region of a disruption target gene was obtained. The ldh gene peripheral region was subjected to PCR using the nucleotides set forth in SEQ ID NOs: 218 and 219 (Fig. 20B) as primers, the atoB gene peripheral region was subjected to PCR using the nucleotides set forth in SEQ ID NOs: 220 and 221 (Fig. 20B) as primers, and the sucD gene peripheral region was subjected to PCR using the nucleotides set forth in SEQ ID NOs: 222 and 223 (Fig. 20B) as primers, thereby obtaining respective fragments.

Next, using In-Fusion HD cloning kit (trade name, manufactured by Clontech Laboratories, Inc.), the present PCR amplification product was inserted into the pHAK1 plasmid fragment amplified using primers (Fig. 20B) set forth in SEQ ID NOs: 224 and 225, and the PCR product was circularized.

Using, as a template, a pHAK1 plasmid into which the DNA fragment of the upstream region, the coding region, and the downstream region of the resulting disruption target gene was inserted, the ldh gene peripheral region was subjected to PCR using the nucleotides set forth in SEQ ID NOs: 226 and 227 (Fig. 20B) as primers, the atoB gene peripheral region was subjected to PCR using the nucleotides set forth in SEQ ID NOs: 228 and 229 (Fig. 20B) as primers, and the sucD gene peripheral region was subjected to PCR using the nucleotides set forth in SEQ ID NOs: 230 and 231 as primers (Fig. 20B), thereby obtaining plasmid fragments in which a partial region or the entire region of the coding region of the disruption target gene was removed.

The resulting plasmid fragment was circularized by terminal phosphorylation and self-ligation to obtain a plasmid for disrupting a gene.

A plasmid for disrupting a desired gene was transformed into the Escherichia coli BL21(DE3) strain by a calcium chloride method (refer to Genetic Engineering Laboratory Notebook, by Takaaki Tamura, Yodosha), and then applied to an LB agar medium (10 g/L of tryptone, 5 g/L of yeast extract, 5 g/L of sodium chloride, and 15 g/L of agar powder) containing 100 mg/L of kanamycin sulfate, and culture was performed at 30°C overnight to obtain a single colony, thereby obtaining a transformant. The present transformant was inoculated into 1 mL of an LB liquid medium (10 g/L of tryptone, 5 g/L of yeast extract, and 5 g/L of sodium chloride) containing 100 mg/L of kanamycin sulfate with one platinum loop, and shaking culture was performed at 30°C. The resulting culture solution was applied to an LB agar medium containing 100 mg/L of kanamycin sulfate, and culture was performed at 42°C overnight. In the resulting colony, a plasmid was inserted into the genome by single crossover. The colony was inoculated into 1 mL of an LB liquid medium with one platinum loop, and shaking culture was performed at 30°C. The resulting culture solution was applied to an LB agar medium containing 10% sucrose, and culture was performed overnight. Disruption of a desired gene in the resulting colony was observed by colony direct PCR. First, an ldh gene-disrupted strain was constructed, an atoB gene-disrupted strain was constructed based on the present ldh gene-disrupted strain, and a sucD gene-disrupted strain was constructed based on the ldh and atoB double-disrupted strain. The produced ldh, atoB, and sucD triple-disrupted strain was defined as an ASL000 strain.

The constructed plasmid was transformed into the ASL000 strain, and applied to an LB agar medium (10 g/L of tryptone, 5 g/L of yeast extract, 5 g/L of sodium chloride, and 15 g/L of agar powder) containing 50 mg/L of ampicillin sodium, 30 mg/L of kanamycin sulfate, and 50 mg/L of streptomycin sulfate, and incubated at 37°C for 24 hours, thereby obtaining single colonies of transformants. The resulting transformants are shown in Table B-5.

**[Table B-5]**

| Strain name | | Host | Plasmid (1) | pRSFDuet | | Plasmid (2) | pETDuet | | Plasmid (3) | pCDFDuet |
|---|---|---|---|---|---|---|---|---|---|---|
| | | | | step 1 | step 2 | | step 3 | step 4 | | step 5 |
| ADA197 | Control strain | ASL000 | prm69 | paaJ (Ec) | paaH (Ec) | prm127 | paaF (Ec) | dcaA (Ab) | prm92 | tesB (Ab) |
| ADA500 | Inventive strain | ASL000 | prm69 | paaJ (Ec) | paaH (Ec) | prm234 | paaF (Ec) | mmgC (L3) | prm92 | tesB (Ab) |
| ADA496 | Inventive strain | ASL000 | prm69 | paaJ (Ec) | paaH (Ec) | prm166 | paaF (L3) | mmgC (L3) | prm92 | tesB (Ab) |

### <Adipic Acid Production Test by Recombinant Escherichia Coli Expressing Acquired Enzyme Gene>

The strains shown in Table B-5 were inoculated with one platinum loop into 2 mL of an LB liquid medium (10 g/L of tryptone, 5 g/L of yeast extract, and 5g/L of sodium chloride) (14 mL capacity round bottom tube) containing 50 mg/L of ampicillin sodium, 30 mg/L of kanamycin sulfate, and 50 mg/L of streptomycin sulfate, and shaking culture was performed at 37°C for 24 hours, thereby obtaining a pre-culture solution. 10 µL of the resulting pre-culture solution was inoculated into 1 mL of MM medium (96-well deep well plate) containing 50 mg/L of ampicillin sodium, 30 mg/L of kanamycin sulfate, and 50 mg/L of streptomycin sulfate, and shaking culture was performed at 37°C for 48 hours. The composition of the MM medium is shown in Table B-6.

**[Table B-6]**

| MM medium | |
|---|---|
| KH2P04 | 3 g/L |
| K2HPO4 | 6 g/L |
| (NH4)2SO4 | 2.5 g/L |
| MgSO4 · 7H2O | 0.3 g/L |
| CaCl2 · 2H2O | 1 mg/L |
| FeSO4 · 7H24 | 6 mg/L |
| MnSO4 · H2O | 1.5 mg/L |
| AlCl3 · 6H2O | 1.5 mg/L |
| CoCl2 | 0.6 mg/L |
| ZnSO4 · 7H2O | 0.3 mg/L |
| Na2MoO2 · 2H2O | 0.3 mg/L |
| CuCl2 · 2H2O | 0.15 mg/L |
| H3BO3 | 0.075 mg/L |
| MOPS | 125 mM |
| **Glycerol** | 20 g/L |

After completion of the culture, the adipic acid and adipic acid analogue concentrations in the culture solution were analyzed. The analysis was performed by GC/MS analysis using trimethylsilyl derivatization. To 40 µL of the culture solution centrifugal supernatant (disodium sebacate was added as an internal standard so as to have a final concentration of 10 mM), 360 µL of an extract solution, in which water, methanol, and chloroform were mixed so that water:methanol:chloroform was 5:2:2 (v/v/v), was added, and the mixture was thoroughly stirred. After centrifugation (16,000 x g, 5 minutes), 100 µL of the supernatant was collected in a separate microtube and centrifugally concentrated on a centrifugal evaporator for 1 hour. 100 µL of a 20 mg/mL pyridine solution of methoxyamine hydrochloride was added to the resulting dried solid, and the mixture was shaken at 30°C for 90 minutes. 50 µL of N-methyl-N-trimethylsilyltrifluoroacetamide was added and the mixture was shaken at 37°C for 30 minutes. The reaction solution was used as a sample for GC/MS measurement, and analysis was performed under the following conditions.

### GC/MS Analysis Conditions

Apparatus: GCMS-QP-2020NX (manufactured by Shimadzu Corporation)
Column: fused silica capillary tube inert treatment tube (length: 1 m, outer diameter: 0.35 mm, inner diameter: 0.25 mm, manufactured by GL Sciences Inc.), InertCap 5MS/NP (length: 30 m, inner diameter: 0.25 mm, film thickness: 0.25 um, manufactured by GL Sciences Inc.)
   Sample injection amount: 1 µL
Sample introduction method: Split (split ratio: 25:1)
Vaporization chamber temperature: 230°C
Carrier gas: helium
Carrier gas linear velocity: 39.0 cm/sec
Oven temperature: kept at 80°C for 2 minutes → raised at 15°C/min → kept at 325°C for 13 minutes
Ionization method: electron ionization method (EI)
Ionization energy: 70 eV
Ion source temperature: 230°C
Scan range: m/z = 50 to 500
Injection amount: 1 µL

The results are shown in Table B-7. In the table, "3OA" represents 3-oxoadipic acid, "LEV" represents levulinic acid, "3HA" represents 3-hydroxyadipic acid, "23DA" represents 2,3-dehydroadipic acid, and "ADA" represents adipic acid.

**[Table B-7]**

| Strain name | | Concentration in culture solution (mg/L) | | | | | |
|---|---|---|---|---|---|---|---|
| | | 30A | LEV | 3HA | 23DA | ADA | Total |
| ADA197 | Control strain | 0 | 0 | 83 | 0 | 105 | 188 |
| ADA500 | Inventive strain | 112 | 21 | 113 | 40 | 69 | 355 |
| ADA496 | Inventive strain | 295 | 41 | 154 | 38 | 84 | 613 |

In the inventive strains expressing both PaaF(L3) and mmgC(L3) derived from the L3 strain, that is, the ADA500 strain and the ADA496 strain, 3-oxoadipic acid (3-OA), levulinic acid (LEV), 3-hydroxyadipic acid (3HA), 2,3-dehydroadipic acid (23DA), and adipic acid (ADA) were produced more than those in the control strain, and these products were produced in 355 mg/L and 613 mg/L, respectively, as the total values. From the above results, it was shown that the enzymes encoded by these genes had a high activity to progress the adipic acid pathway reaction, and improved the amounts of adipic acid and adipic acid derivative produced.

### Industrial Applicability

According to the present invention, for example, an efficient production process of a C6 compound can be provided, and is expected to be applied to production on an industrial scale.

The present invention can also be suitably utilized in the fermentative production of adipic acids directly from biomass raw materials.

## Claims

1. A recombinant microorganism comprising an exogenous gene encoding a protein having an enzymatic activity to reduce 2,3-dehydroadipyl-CoA for conversion into adipyl-CoA,
wherein the exogenous gene is:
(i) a DNA encoding a protein consisting of an amino acid sequence having a sequence identity of 80% or higher with an amino acid sequence set forth in SEQ ID NO: 8, 9, 10, 11, 12, 13, 14, 15, or 16,
(ii) a DNA encoding a protein consisting of an amino acid sequence in which 1 to 10 amino acids are deleted, substituted, inserted, and/or added in an amino acid sequence set forth in SEQ ID NO: 8, 9, 10, 11, 12, 13, 14, 15, or 16,
(iii) a DNA consisting of a polynucleotide sequence having a sequence identity of 80% or higher with a polynucleotide sequence set forth in SEQ ID NO: 57, 58, 59, 60, 61, 62, 63, 64, or 65,
(iv) a DNA encoding a protein consisting of an amino acid sequence in which 1 to 10 amino acids are deleted, substituted, inserted, and/or added in an amino acid sequence of a protein encoded by a polynucleotide sequence set forth in SEQ ID NO: 57, 58, 59, 60, 61, 62, 63, 64, or 65,
(v) a DNA hybridizing with a DNA consisting of a polynucleotide sequence complementary to a polynucleotide sequence set forth in SEQ ID NO: 57, 58, 59, 60, 61, 62, 63, 64, or 65 under a stringent condition, or
(vi) a DNA consisting of a degenerate isomer of a polynucleotide sequence set forth in SEQ ID NO: 57, 58, 59, 60, 61, 62, 63, 64, or 65.

2. The recombinant microorganism according to claim 1, wherein the exogenous gene is:
(xi) a DNA encoding a protein consisting of an amino acid sequence set forth in SEQ ID NO: 8, 9, 10, 11, 12, 13, 14, 15, or 16, or
(xiii) a DNA consisting of a polynucleotide sequence set forth in SEQ ID NO: 57, 58, 59, 60, 61, 62, 63, 64, or 65.

3. The recombinant microorganism according to claim 1 or 2, wherein the exogenous gene is derived from at least one selected from Candida auris, Kluyveromyces marxianus, Pichia kudriavzevii, Thermothelomyces thermophilus, Thermothielavioides terrestris, Chaetomium thermophilum, Podospora anserina, Purpureocillium lilacinum, and Pyrenophora teres.

4. The recombinant microorganism according to any one of claims 1 to 3, wherein the recombinant microorganism has a production pathway of a C6 compound, and wherein the C6 compound is at least one selected from the group consisting of adipic acid, hexamethylenediamine, 1,6-hexanediol, 6-aminohexanoic acid, 6-amino-1-hexanol, and 6-hydroxyhexanoic acid.

5. The recombinant microorganism according to any one of claims 1 to 4, wherein the recombinant microorganism belongs to the genus Escherichia, the genus Bacillus, the genus Corynebacterium, the genus Arthrobacter, the genus Brevibacterium, the genus Clostridium, the genus Zymomonas, the genus Pseudomonas, the genus Burkholderia, the genus Streptomyces, the genus Rhodococcus, the genus Synechocystis, the genus Alkalihalobacillus, the genus Saccharomyces, the genus Schizosaccharomyces, the genus Yarrowia, the genus Candida, the genus Pichia, or the genus Aspergillus.

6. The recombinant microorganism according to any one of claims 1 to 5, wherein the recombinant microorganism is Escherichia coli.

7. The recombinant microorganism according to any one of claims 1 to 6, further comprising at least one selected from the group consisting of:
• a gene encoding a 3-oxoadipyl-CoA thiolase;
• a gene encoding a 3-hydroxyadipyl-CoA dehydrogenase;
• a gene encoding a 3-hydroxyadipyl-CoA dehydratase;
• a gene encoding a carboxylic acid reductase;
• a gene encoding an alcohol dehydrogenase; and
• a gene encoding a transaminase (aminotransferase).

8. A method for producing a C6 compound, the method comprising a culture step of culturing the recombinant microorganism according to any one of claims 1 to 7,
wherein the C6 compound is at least one selected from the group consisting of adipic acid, hexamethylenediamine, 1,6-hexanediol, 6-aminohexanoic acid, 6-amino-1-hexanol, and 6-hydroxyhexanoic acid.

9. A recombinant protein:
(a) having an enzymatic activity to reduce 2,3-dehydroadipyl-CoA for conversion into adipyl-CoA, and
(b) satisfying any one of the followings:
(i) consisting of an amino acid sequence having a sequence identity of 80% or higher with an amino acid sequence set forth in SEQ ID NO: 8, 9, 10, 11, 12, 13, 14, 15, or 16;
(ii) consisting of an amino acid sequence in which 1 to 10 amino acids are deleted, substituted, inserted, and/or added in an amino acid sequence set forth in SEQ ID NO: 8, 9, 10, 11, 12, 13, 14, 15, or 16;
(iii) encoded by a DNA consisting of a polynucleotide sequence having a sequence identity of 80% or higher with a polynucleotide sequence set forth in SEQ ID NO: 57, 58, 59, 60, 61, 62, 63, 64, or 65;
(iv) consisting of an amino acid sequence in which 1 to 10 amino acids are deleted, substituted, inserted, and/or added in an amino acid sequence of a protein encoded by a polynucleotide sequence set forth in SEQ ID NO: 57, 58, 59, 60, 61, 62, 63, 64, or 65;
(v) encoded by a DNA hybridizing with a DNA consisting of a polynucleotide sequence complementary to a polynucleotide sequence set forth in SEQ ID NO: 57, 58, 59, 60, 61, 62, 63, 64, or 65 under a stringent condition; and
(vi) encoded by a DNA consisting of a degenerate isomer of a polynucleotide sequence set forth in SEQ ID NO: 57, 58, 59, 60, 61, 62, 63, 64, or 65.

10. A recombinant protein:
(xi) consisting of an amino acid sequence set forth in SEQ ID NO: 8, 9, 10, 11, 12, 13, 14, 15, or 16; or
(xiii) encoded by a DNA consisting of a polynucleotide sequence set forth in SEQ ID NO: 57, 58, 59, 60, 61, 62, 63, 64, or 65.

11. A method for producing a C6 compound, the method comprising using the recombinant protein according to claim 9 or 10.

12. A recombinant microorganism comprising at least one of an exogenous gene encoding a 3-hydroxyadipyl-CoA dehydratase and an exogenous gene encoding a 2,3-dehydroadipyl-CoA reductase.

13. The recombinant microorganism according to claim 12, wherein at least one of the 3-hydroxyadipyl-CoA dehydratase and the 2,3-dehydroadipyl-CoA reductase is derived from the genus Burkholderia (Burkholderia sp.) LEBP-3 strain (Accession Number: NITE BP-03334).

14. The recombinant microorganism according to claim 12 or 13, wherein the 3-hydroxyadipyl-CoA dehydratase is encoded by a DNA having a sequence identity of 80% or higher, 85% or higher, 88% or higher, 90% or higher, 93% or higher, 95% or higher, 97% or higher, 98% or higher, or 99% or higher with a PCR amplification product obtained using a chromosomal DNA of the genus Burkholderia (Burkholderia sp.) LEBP-3 strain as a template and using, as a primer,
(1a) a nucleotide consisting of a base sequence set forth in SEQ ID NO: 168 and a nucleotide consisting of a base sequence set forth in SEQ ID NO: 169, or
(1b) a nucleotide having a base sequence set forth in SEQ ID NO: 168 and a nucleotide having a base sequence set forth in SEQ ID NO: 169, and
The DNA has 626 to 940 bp.

15. The recombinant microorganism according to any one of claims 12 to 14, wherein the 2,3-dehydroadipyl-CoA reductase is encoded by a DNA having a sequence identity of 80% or higher, 85% or higher, 88% or higher, 90% or higher, 93% or higher, 95% or higher, 97% or higher, 98% or higher, or 99% or higher with a PCR amplification product obtained using a chromosomal DNA of the genus Burkholderia (Burkholderia sp.) LEBP-3 strain as a template and using, as a primer,
(2a) a nucleotide consisting of a base sequence set forth in SEQ ID NO: 170 and a nucleotide consisting of a base sequence set forth in SEQ ID NO: 171, or
(2b) a nucleotide having a base sequence set forth in SEQ ID NO: 170 and a nucleotide having a base sequence set forth in SEQ ID NO: 171, and
the DNA has 924 to 1,386 bp.

16. The recombinant microorganism according to claim 12 or 13, wherein the 3-hydroxyadipyl-CoA dehydratase is encoded by a DNA that is a PCR amplification product obtained using a chromosomal DNA of the genus Burkholderia (Burkholderia sp.) LEBP-3 strain as a template and using, as a primer,
(1a) a nucleotide consisting of a base sequence set forth in SEQ ID NO: 168 and a nucleotide consisting of a base sequence set forth in SEQ ID NO: 169, or
(1b) a nucleotide having a base sequence set forth in SEQ ID NO: 168 and a nucleotide having a base sequence set forth in SEQ ID NO: 169, and
the DNA has 783 or 784 bp.

17. The recombinant microorganism according to any one of claims 12 to 14, wherein the 2,3-dehydroadipyl-CoA reductase is encoded by a DNA that is a PCR amplification product obtained using a chromosomal DNA of the genus Burkholderia (Burkholderia sp.) LEBP-3 strain as a template and using, as a primer,
(2a) a nucleotide consisting of a base sequence set forth in SEQ ID NO: 170 and a nucleotide consisting of a base sequence set forth in SEQ ID NO: 171, or
(2b) a nucleotide having a base sequence set forth in SEQ ID NO: 170 and a nucleotide having a base sequence set forth in SEQ ID NO: 171, and
the DNA has 1,155 or 1,156 bp.

18. The recombinant microorganism according to any one of claims 12 to 17, wherein the recombinant microorganism belongs to a genus selected from the group consisting of the genus Escherichia, the genus Corynebacterium, the genus Bacillus, the genus Acinetobacter, the genus Burkholderia, the genus Pseudomonas, the genus Clostridium, the genus Saccharomyces, the genus Schizosaccharomyces, the genus Yarrowia, the genus Candida, the genus Pichia, and the genus Aspergillus.

19. The recombinant microorganism according to any one of claims 12 to 18, wherein the recombinant microorganism has an adipic acid production pathway.

20. The recombinant microorganism according to any one of claims 12 to 19, wherein the recombinant microorganism has a hexamethylenediamine production pathway.

21. The recombinant microorganism according to any one of claims 12 to 19, wherein the recombinant microorganism has a 1,6-hexanediol production pathway.

22. The recombinant microorganism according to any one of claims 12 to 19, wherein the recombinant microorganism has a 6-amino-1-hexanol production pathway.

23. A method for producing a target compound, the method comprising a culture step of culturing the recombinant microorganism according to any one of claims 12 to 18,
wherein the target compound is selected from the group consisting of adipic acid, an adipic acid derivative, hexamethylenediamine, 1,6-hexanediol, and 6-amino-1-hexanol, and
the adipic acid derivative is selected from the group consisting of oxoadipic acid, levulinic acid, 3-hydroxyadipic acid, and 2,3-dehydroadipic acid.

24. A method for producing adipic acid, the method comprising a culture step of culturing the recombinant microorganism according to claim 19.

25. A method for producing hexamethylenediamine, the method comprising a culture step of culturing the recombinant microorganism according to claim 20.

26. A method for producing 1,6-hexanediol, the method comprising a culture step of culturing the recombinant microorganism according to claim 21.

27. A method for producing 6-amino-1-hexanol, the method comprising a culture step of culturing the recombinant microorganism according to claim 22.

28. (A-1) A recombinant polypeptide encoded by a DNA having a sequence identity of 80% or higher, 85% or higher, 88% or higher, 90% or higher, 93% or higher, 95% or higher, 97% or higher, 98% or higher, or 99% or higher with a PCR amplification product obtained using a chromosomal DNA of the genus Burkholderia (Burkholderia sp.) LEBP-3 strain as a template, and using, as a primer,
(a1) a nucleotide consisting of a base sequence set forth in SEQ ID NO: 168 and a nucleotide consisting of a base sequence set forth in SEQ ID NO: 169, or
(a2) a nucleotide having a base sequence set forth in SEQ ID NO: 168 and a nucleotide having a base sequence set forth in SEQ ID NO: 169,
wherein the recombinant polypeptide has a 3-hydroxyadipyl-CoA dehydratase activity, and
the DNA has 626 to 940 bp;
(A-2) a recombinant polypeptide consisting of an amino acid sequence in which 1 to 10 amino acids are deleted, substituted, inserted, and/or added in an amino acid sequence of a polypeptide encoded by a DNA that is a PCR amplification product obtained using a chromosomal DNA of the genus Burkholderia (Burkholderia sp.) LEBP-3 strain as a template, and using, as a primer,
(a1) a nucleotide consisting of a base sequence set forth in SEQ ID NO: 168 and a nucleotide consisting of a base sequence set forth in SEQ ID NO: 169, or
(a2) a nucleotide having a base sequence set forth in SEQ ID NO: 168 and a nucleotide having a base sequence set forth in SEQ ID NO: 169,
wherein the recombinant polypeptide has a 3-hydroxyadipyl-CoA dehydratase activity, and
the DNA has 626 to 940 bp; or
(A-3) a recombinant polypeptide encoded by a DNA consisting of a degenerate isomer of a PCR amplification product obtained using a chromosomal DNA of the genus Burkholderia (Burkholderia sp.) LEBP-3 strain as a template, and using, as a primer,
(a1) a nucleotide consisting of a base sequence set forth in SEQ ID NO: 168 and a nucleotide consisting of a base sequence set forth in SEQ ID NO: 169, or
(a2) a nucleotide having a base sequence set forth in SEQ ID NO: 168 and a nucleotide having a base sequence set forth in SEQ ID NO: 169,
wherein the recombinant polypeptide has a 3-hydroxyadipyl-CoA dehydratase activity, and
the DNA has 626 to 940 bp.

29. (B-1) A recombinant polypeptide encoded by a DNA having a sequence identity of 80% or higher, 85% or higher, 88% or higher, 90% or higher, 93% or higher, 95% or higher, 97% or higher, 98% or higher, or 99% or higher with a PCR amplification product obtained using a chromosomal DNA of the genus Burkholderia (Burkholderia sp.) LEBP-3 strain as a template, and using, as a primer,
(b1) a nucleotide consisting of a base sequence set forth in SEQ ID NO: 170 and a nucleotide consisting of a base sequence set forth in SEQ ID NO: 171, or
(b2) a nucleotide having a base sequence set forth in SEQ ID NO: 170 and a nucleotide having a base sequence set forth in SEQ ID NO: 171,
wherein the recombinant polypeptide has a 2,3-dehydroadipyl-CoA reductase activity, and
the DNA has 924 to 1,386 bp;
(B-2) a recombinant polypeptide consisting of an amino acid sequence in which 1 to 10 amino acids are deleted, substituted, inserted, and/or added in an amino acid sequence of a polypeptide encoded by a DNA that is a PCR amplification product obtained using a chromosomal DNA of the genus Burkholderia (Burkholderia sp.) LEBP-3 strain as a template, and using, as a primer,
(b1) a nucleotide consisting of a base sequence set forth in SEQ ID NO: 170 and a nucleotide consisting of a base sequence set forth in SEQ ID NO: 171, or
(b2) a nucleotide having a base sequence set forth in SEQ ID NO: 170 and a nucleotide having a base sequence set forth in SEQ ID NO: 171,
wherein the recombinant polypeptide has a 2,3-dehydroadipyl-CoA reductase activity, and
the DNA has 924 to 1,386 bp; or
(B-3) a recombinant polypeptide encoded by a DNA consisting of a degenerate isomer of a PCR amplification product obtained using a chromosomal DNA of the genus Burkholderia (Burkholderia sp.) LEBP-3 strain as a template, and using, as a primer,
(b1) a nucleotide consisting of a base sequence set forth in SEQ ID NO: 170 and a nucleotide consisting of a base sequence set forth in SEQ ID NO: 171, or
(b2) a nucleotide having a base sequence set forth in SEQ ID NO: 170 and a nucleotide having a base sequence set forth in SEQ ID NO: 171,
wherein the recombinant polypeptide has a 2,3-dehydroadipyl-CoA reductase activity, and
the DNA has 924 to 1,386 bp.
